(19) Europäisches Patentamt
European Patent Office
Office européen des brevets

(11) EP 4 613 273 A1

(12) EUROPEAN PATENT APPLICATION

(43) Date of publication:
**10.09.2025 Bulletin 2025/37**

(21) Application number: **24305339.4**

(22) Date of filing: **06.03.2024**

(51) International Patent Classification (IPC):
***A61K 31/496*** *(2006.01)* ***A61P 1/02*** *(2006.01)*
***A61P 19/00*** *(2006.01)* ***A61P 19/02*** *(2006.01)*
***A61P 19/10*** *(2006.01)*

(52) Cooperative Patent Classification (CPC):
**A61K 31/496; A61P 1/02; A61P 19/00; A61P 19/02; A61P 19/10**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(71) Applicants:
- **Vetbiolix**
**59120 Loos (FR)**
- **Medivir Aktiebolag**
**141 22 Huddinge (SE)**

(72) Inventor: **The designation of the inventor has not yet been filed**

(74) Representative: **Cabinet Beau de Loménie**
**103, rue de Grenelle**
**75340 Paris Cedex 07 (FR)**

# (54) TREATMENT OF BONE LOSS

(57) A compound selected from N-[(S)-1-((3aS,6S,6aS)-6-fluoro-3-oxo-hexahydro-furo[3,2-b]pyrrole-4-carbonyl)-3-methyl-butyl]-4-[2-(4-methyl-piperazin-1-yl)-thiazol-4-yl]-benzamide, N-[(S)-1-((3aS,6S,6aS)-6-Fluoro-3,3-dihydroxy-hexahydro-furo[3,2-b]pyrrole-4-carbonyl)-3-methyl-butyl]-4-[2-(4-methyl-piperazin-1-yl)-thiazol-4-yl]-benzamide, mixtures thereof, and pharmaceutically acceptable salts thereof has utility in the treatment of degenerative dental pathologies in companion animals, especially periodontic disease and/or feline odontoclastic lesion (FORL) in domestic cats.

EP 4 613 273 A1

## Description

### FIELD OF THE INVENTION

The present invention relates to a novel veterinary use of a known cysteine protease inhibitor. In particular it relates to the use of a compound selected from N-[(S)-1-((3aS,6S,6aS)-6-fluoro-3-oxo-hexahydro-furo[3,2-b]pyrrole-4-carbonyl)-3-methyl-butyl]-4-[2-(4-methyl-piperazin-1-yl)-thiazol-4-yl]-benzamide, N-[(S)-1-((3aS,6S,6aS)-6-Fluoro-3,3-dihydroxy-hexahydro-furo[3,2-b]pyrrole-4-carbonyl)-3-methyl-butyl]-4-[2-(4-methyl-piperazin-1-yl)-thiazol-4-yl]-benzamide, mixtures thereof, and pharmaceutically acceptable salts thereof, in the treatment of a degenerative disorder mediated by cathepsin K in an animal, including a disorder associated with bone loss and/or a disorder associated with excessive bone resorption such as periodontal disease, tooth resorption and/or osteoarthritis.

A typical manifestation of a pathology treatable in accordance with the invention is periodontal disease and associated periodontitis in felines, such as cats and canines, such as dogs. Another typical manifestation of a pathology treatable in accordance with the invention is feline tooth resorption, feline odontoclastic resorptive lesions ("FORL") in felines such as cats, or canine tooth resorption in canines such as dogs.

### BACKGROUND

Bone is a dynamic tissue that is continually remodelled throughout life depending on factors such as nutrition and the load the bone must carry. Normal bone formation depends on the delicate balance between new bone addition and old bone resorption. Bone formation is based on the deposition of bone matrix by osteoblasts and bone resorption and more specifically mineralized tissue, chiefly calcium carbonate and calcium phosphate resorption in vertebrates is achieved by osteoclasts.

Osteoclasts are multinucleated cells of up to 400pm related to macrophage and other cells that develop from monocyte cells, which are actively motile cells that migrate along the surface of bone. Like macrophage, osteoclasts are derived from haematopoietic progenitor cells. The bone resorption is initiated when osteoclasts attach to the surface of mineralized bone, forms a tight "sealing zone" and secrete necessary acids and proteases that initiate the resorption of mineralized tissue from the bone. After a period of several hours to days, the osteoclasts detach from the bone, leaving a pit on the bone surface. Under normal conditions, the pit is a target for osteoblasts, which deposit a material that ultimately becomes new bone.

Bone loss can appear when the bone resorptive process is dominant over the bone formative process. Diseases associated with bone loss are usually accompanied by increased osteoclast activation. Such diseases associated with bone loss and/or diseases associated with bone resorption comprise, for example, (i) disorders associated with alveolar bone loss and/or a disorder associated with alveolar bone resorption, for example periodontal disease, (ii) tooth resorption, (iii) arthritis, such as osteoarthritis or rheumatoid arthritis, and (iv) osteoporosis.

Periodontal disease is one of the most common oral diseases in dogs (Lund et al. 1999; Robinson et al. 2016). While visual oral assessment of conscious dogs, report an average prevalence of 9.3 to 18.2% within the population (O'Neil et al. 2014; Wallis and Holcombe 2020), detailed examinations of anaesthetised dogs present much higher number, in some publications even up to 90% (Butković et al. 2011; Hamp et al. 1984; Hoffman and Gaengler 1996). The incidence has been observed to correlate with age as well as small and brachycephalic breeds (Pavlica et al. 2008; Marshall et al. 2014).

Periodontal disease can be defined as plaque-induced disease of any part of the periodontium - tissue that holds the tooth in the mouth - and presents five degrees of severity (www.avdc.org):

- Normal (PD0) - clinically normal. Gingival inflammation or periodontitis is not clinically evident.
- Stage 1 (PD1) - gingivitis. There is no periodontal attachment loss. The height and architecture of the alveolar margin are normal.
- Stage 2 (PD2) - early periodontitis. The loss of periodontal attachment is less than 25% as measured either by probing of the clinical attachment level, or radiographic determination of the distance of the alveolar margin from the cementoenamel junction relative to the length of the root, or there is a stage 1 furcation involvement in multirooted teeth. There are early radiologic signs of periodontitis.
- Stage 3 (PD3) - moderate periodontitis. The loss of periodontal attachment is between 25 and 50% as measured either by probing of the clinical attachment level, radiographic determination of the distance of the alveolar margin from the cementoenamel junction relative to the length of the root, or there is a stage 2 furcation involvement in multirooted teeth.
- Stage 4 (PD4) - advanced periodontitis. The loss of periodontal attachment is more than 50% as measured either by probing of the clinical attachment level, or radiographic determination of the distance of the alveolar margin from the cementoenamel relative to the length of the root, or there is a stage 3 furcation involvement in multirooted teeth.

Dog's teeth are constantly covered by saliva which evaporating becomes deposited as a glycoprotein layer on the crowns of the teeth. If daily oral care is not applied, the biofilm accumulates getting thicker and more complex. After 2-3 days the calcium carbonate and calcium phosphate salts crystallizing, and mineralizing creates calculus impossible to be removed (Legeros and Shannin 1979). Plaque, the dental biofilm, is a mixture of around 500 bacterial species including periodontopathogens. The disease is thought to result from complex interaction between bacteria, the immune system response, and the structure of periodontium. The initial effect is inflammation of the gingival tissues with high number of neutrophils attracted to the site of pathogenic plaque. With time, many of them become overly full and burst releasing bacterial toxins, destructive enzymes, and cytokines. This process causes breakdown of the connective tissue integrity and propagates the inflammatory response resulting in the progressive destruction of the periodontal tissues and eventually attachment loss (Wiggs RB & Lobprise HB 1997). This can be observed as gingival recession, periodontal pocket formation, or both.

As the inflammation descends deeper into the tissue, inflammation-induced resorption leads to alveolar bone loss which is irreversible without advanced regenerative surgery (Shoukry M et al 2007; DeBowes LJ 2010). Alveolar bone loss from periodontal disease divides into focal or generalized loss and can be observed as horizontal (suprabony), affecting one of more roots of the teeth, or vertical (infrabony).

Untreated disease can cause local changes including oronasal fistulas, class II perio-endo lesions, pathologic fractures, ocular problems, osteomyelitis, and possibly an increased incidence of oral cancer (DeBowes LJ 2010, Niemiec BA 2013a). With the increasing number of bacteria and inflammatory mediators renal, hepatic, pulmonary, and cardiac diseases can arise together with osteoporosis, arthritis, adverse pregnancy effects, and diabetes (Niemiec BA 2013b).

Clinically, normal gingival tissues are coral pink and have a thin edge, with a smooth, regular texture and no demonstrable plaque or calculus. Normal sulcal depth in a dog is 0 to 3 mm (Wiggs RB & Lobprise HB 1997, DeBowes LJ 2010, Bellows J 2004). The first outward clinical sign of gingivitis is erythema of the gingiva, typically associated with calculus, which is followed by oedema and halitosis (Fiorellini JP et al 2006; DeBowes LJ 2010). As gingivitis progresses to periodontitis, the oral inflammatory changes intensify. The hallmark clinical feature of established periodontitis is attachment loss when periodontal attachment to the tooth recedes apically. In some cases, the loss results in gingival recession while the sulcal depth remains the same. In other cases, the gingiva remains at the same height while the area of attachment moves apically, thus creating a periodontal pocket (Niemiec BA 2013a). Attachment loss progresses, until tooth exfoliation in most cases. After tooth exfoliation occurs, the area generally returns to an uninfected state, but the bone loss is permanent.

The prevention of dental plaque formation and its removal are the key points to avoid periodontal disease development. It can be achieved by a combination of dental hygiene care procedures, special diet, chew toys availability and regular professional periodontal treatments (Soukup, 2010; Albuquerque et al., 2012). Once the disease occurs, the first line of treatment is always non- surgical, aiming at the removal of factors that promote disease progression. Scaling and root planning can be further accompanied by administration of antibiotics and nonsteroidal anti-inflammatory drugs (NAIDS) to promote the reduction of gingival inflammation and bleeding, pocket depths, subgingival bacteria and clinical attachment loss (CAL) (Soukup, 2010). Surgical treatment is necessary in severe cases, mostly in PD3 and PD4, in which moderate alveolar bone loss and furcation levels 2 and 3 are observed (Niemiec, 2008). With the main objective at periodontium regeneration (Soukup, 2010) numerous surgical techniques such as gingivectomy, gingivoplasty, open flap debridement, osseous grafting, guided tissue regeneration, use of bioactive products such as bone morphogenic protein (Anthony, 2000; Soukup, 2010; Stepaniuk and Gingerich, 2015; Lee et al., 2016a; Greenwell 2001) can be applied. Finally, periodontal disease treatment may require tooth extraction, namely in severe cases or when periodontal regeneration techniques and associated dental hygiene care are not achievable (Niemiec, 2008; Albuquerque et al., 2012).

Feline odontoclastic resorptive lesion (FORL) has become one of the most common dental diseases in cats. Dental resorptive lesions are also known by a variety of other names including odontoclastic resorptive lesions, feline neck lesions, cervical line lesions, chronic subgingival tooth erosions, feline external resorptive lesions, and/or subgingival resorptive lesions. This disease is characterized by cavitating lesions produced by odontoclastic resorption originating sub-gingivally and progressively eroding through the enamel/cementum and dentin layers into the pulpal tissues of the tooth. The odontoclasts progressively attack apparently healthy tooth substance, producing large painful tooth lesions, ultimately resulting in tooth breakage with retention of root fragments in the gum.

Observation of dental resorptive lesions during routine examination of cats can be difficult because relatively little erosion of the cementum and enamel may be evident at the buccal surface. However, the lesion is apparent when examined radiographically. Although the lesion may not be obvious, it is commonly accompanied by signs of pain. Other clinical signs include anorexia, concurrent gingivitis, and excessive salivation.

The primary cause of dental resorptive lesions is not definitively known. Possible causes include oral inflammation, plaque, periodontal disease, systemic disease conditions, dietary factors, breed predisposition, and defects and diseases in the tissues of the tooth or periodontium. Of the studies done on feline dental resorptive lesions, the prevalence of the lesions consistently increases with age.

Currently, there is no effective treatment for dental resorptive lesions in animals. Often, the affected teeth break

off, thus creating the potential for root sequestrum formation and subsequent infection. Typically, affected teeth must be extracted because attempts to save the tooth by drilling out the lesion and filling it with restorative materials are ineffective. Approximately 90% of the repair attempts fall out within two years because of ongoing resorption. In addition, new lesions often develop in other teeth within the mouth of an affected individual.

Unlike human cavities, the cause of dental resorptive lesions in animals, such as tooth resorption, FORL or periodontal disease, is unknown and there are currently no effective treatments for these diseases. Specifically, cells known as odontoclasts are found in the defect causing tooth resorption. What exactly triggers this reaction has not been precisely determined, however, recent research has indicated that acidosis is a major regulator of osteoclastic formation and functional activity in the cat. It is suggested that local pH changes may play a significant role in the pathogenesis of FORL.

While normal tissue pH is around 7.5-9.0 (depending on the mammal), inflammation in the tissue can reduce the local pH to 5.5 or lower. However, it is important to note that the oral cavity of companion animals such as dogs and cats presents a very different environment than that of humans. The pH of human saliva is around 7.0 (neutral), whereas the saliva of cats and dogs is much more alkaline with a pH around 9.0.

In human, treatments used nowadays to limit the bone loss due to the exacerbated activity of osteoclasts are mainly molecules from nitrogen bisphosphonate family. By preventing bone resorption by osteoclasts, bisphosphonates are medications that prevent the skeleton embrittlement and the establishment of osteoporosis. Bisphosphonates help to protect the bones against the effects of certain cancers and to treat some bone disorders such as postmenopausal osteoporosis or rheumatoid arthritis. For the osteoporosis treatment, bisphosphonates of reference are the zoledronate (Aclasta, Zometa), the alendronate (Fosamax), the risedronate (Actonel) or the etidronate (Didrocal, Didronel). Bisphosphonates cause osteoclast death by apoptosis. The Denosumab, a monoclonal antibody directed against the cytokine RANKL, has recently been placed on the market (Prolia, Xgeva). It prevents the differentiation of osteoclasts.

Still in human, drugs targeting cysteine proteases have been disclosed for treating bone loss and/or bone resorption. The papain superfamily of cysteine proteases is widely distributed in diverse species including mammals, invertebrates, protozoa, plants and bacteria. A number of mammalian cathepsin enzymes, including cathepsins B, F, H, K, L, O and S, have been ascribed to this superfamily, and inappropriate regulation of their activity has been implicated in a number of metabolic disorders including arthritis, muscular dystrophy, inflammation, glomerulonephritis and tumour invasion. Pathogenic cathepsin like enzymes include the bacterial gingipains, the malarial falcipains I, II, III et seq and cysteine proteases from Pneumocystis carinii, Trypanosoma cruzei and brucei, Crithidia fusiculata, Schistosoma spp.

In human, cathepsin K is the major enzyme involved in bone resorption (Duong 2012). It is predominantly secreted by activated osteoclasts to degrade type I collagen, osteonectin and other matrix proteins during bone resorption (Costa et al. 2011). Clinical disorders in which bone resorption is increased are very common and include Paget's disease of bone, osteoporosis, and the bone changes secondary to cancer, such as occur in myeloma and metastases from breast cancer. Clinical disorders of reduced bone resorption are less common and often have a genetic basis, i.e., due to cathepsin K deficiency in pycnodysostosis (Gelb et al. 1996). Inappropriate regulation of cathepsin K has been implicated in a number of disorders including osteoporosis, periodontal disease such as gingivitis and periodontitis, Paget's disease, hypercalcaemia of malignancy and metabolic bone disease. In view of its elevated levels in chondroclasts of osteoarthritic synovium, cathepsin K is implicated in diseases characterised by excessive cartilage or matrix degradation, such as osteoarthritis and rheumatoid arthritis.

In that matter, cathepsin K inhibitors have been proposed as novel treatment of metabolic bone-diseases with excessive bone resorption in human and have been a subject of extensive research during last two decades. In contrary to marketed antiresorptive agents (bisphosphonates and denosumab) cathepsin K inhibition results in increased number of osteoclasts, which, although impaired in their ability to resorb bone matrix, remain on the bone surface, capable of local signalling and maintaining osteoblast function and activity. Thus, although cathepsin K inhibition effectively limits osteoclast-mediated bone resorption, it also permits a relative preservation of bone formation (Drake et al.2017).

International patent application WO2005/066180 discloses a number of cysteine protease inhibitors for the treatment of human osteoporosis, including N-[(S)-1-((3aS,6S,6aS)-6-fluoro-3-oxo-hexahydro-furo[3,2-b]pyrrole-4-carbonyl)-3-methyl-butyl]-4-[2-(4-methyl-piperazin-1-yl)-thiazol-4-yl]-benzamide (corresponding to Example 8.19 therein).

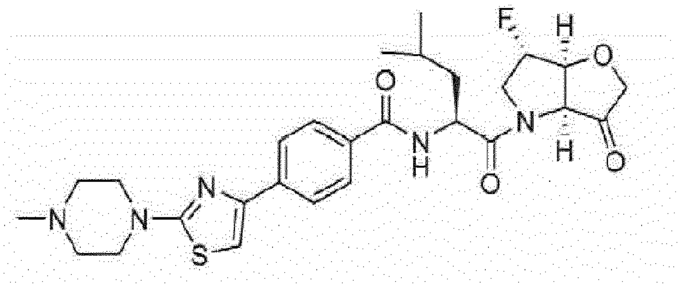

(Compound Ia)

N-[(S)-1-((3aS,6S,6aS)-6-fluoro-3-oxo-hexahydro-furo[3,2-b]pyrrole-4-carbonyl)-3-methylbutyl]-4-[2-(4-methyl-piperazin-1-yl)-thiazol-4-yl]-benzamide ("Compound Ia") is specifically disclosed in WO2005/066180 in the form of the free base.

It should be noted that the nomenclature of the compounds described herein may vary. Compound Ia free base is described herein as N-[(S)-1-((3aS,6S,6aS)-6-fluoro-3-oxo-hexahydro-furo[3,2-b]pyrrole-4-carbonyl)-3-methyl-butyl]-4-[2-(4-methyl-piperazin-1-yl)-thiazol-4-yl]-benzamide, but may also be referred to as N-((S)-1-((3aS,6R,6aR)-6-fluoro-3-oxotetrahydro-2H-furo[3,2-b]pyrrol-4(5H)-yl)-4-methyl-1-oxopentan-2-yl)-4-(2-(4-methylpiperazin-1-yl)thiazol-4-yl)benzamide. For the avoidance of doubt, both compound names refer to the same chemical structure of:

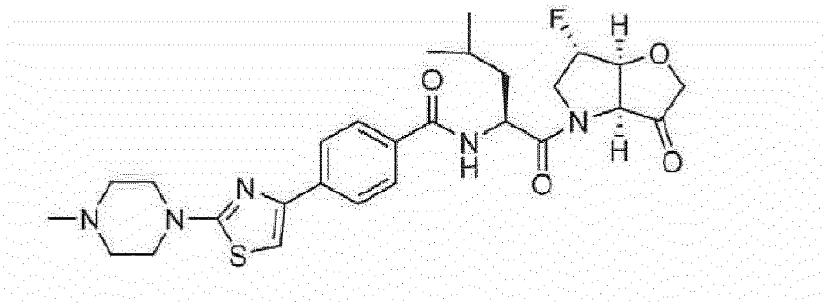

(Compound Ia)

The divergence in the compound naming arises due to there being two different conventions for naming the following bicyclic unit:

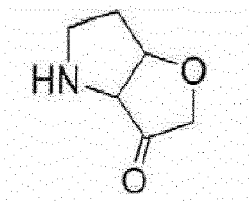

The unit may be referred to as "3-oxo-hexahydro-furo[3,2-b]pyrrole" or "3-oxotetrahydro-2H-furo[3,2-b]pyrrole".

Compound Ia is also registered with the CAS n°860343-27-7.

It should also be noted that Compound Ia described herein may exist in alternative forms, such as the keto form (Compound Ia) and the hydrated form (Compound Ib) which may coexist being in equilibrium in solution. The hydrated form, Compound Ib, is also disclosed in WO2005/066180.

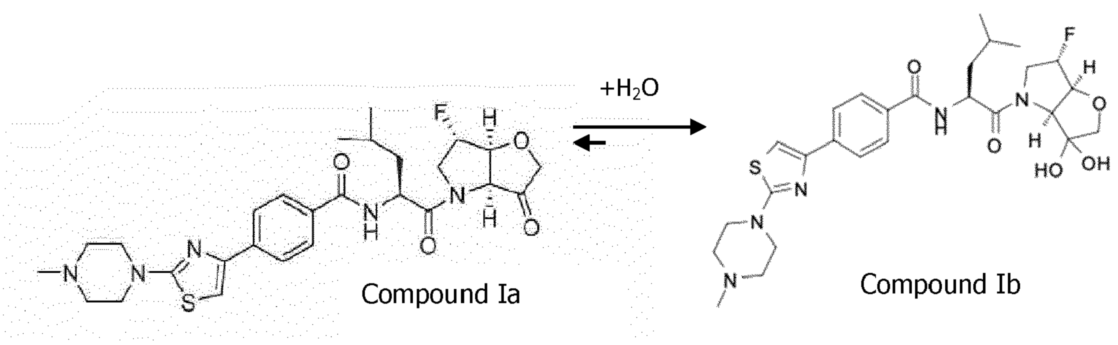


Compound Ia

Compound Ib

For the avoidance of doubt, Compound I refers indifferently to compound Ia, compound Ib or a mixture thereof.

International patent application WO2008/034781 discloses the besylate salt of Compound Ia, also proposed for the human osteoporosis indication.

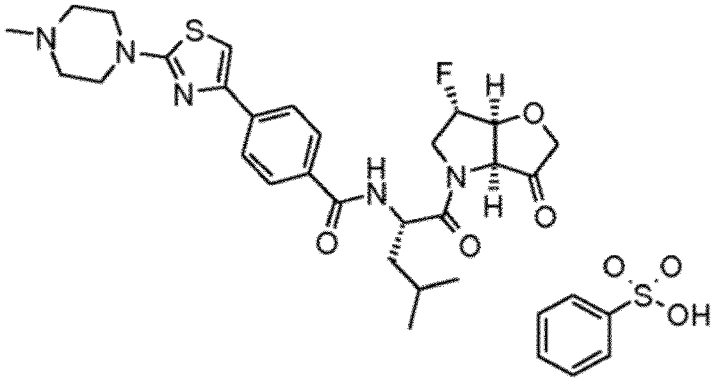

Compound I went into phase 1 clinical trials in humans in March 2007 under the acronym MIV-701, where it was postulated to inhibit cathepsin K in the lysozymes of osteoclasts. The results of the phase 1 clinical trial were presented at the VIth SCI-RSC Symposium on Proteinase Inhibitor Design, London UK 21-22 April 2008. The compound reduced the rate of bone resorption in a clinically relevant manner. A once daily dose of 300 mg reduced bone resorption biomarker CTX-1 production by greater than 50% in men and post-menopausal women. Drug absorption was dose-proportional.

However, as presented at the ACS Spring Meeting Anaheim, 21 March 2011, the further clinical development of MIV-701 was terminated following the surprising discovery instability of the compound in human whole blood, caused by an unknown blood-borne reductase (see the observed reduction reaction below). This instability was not present in plasma.

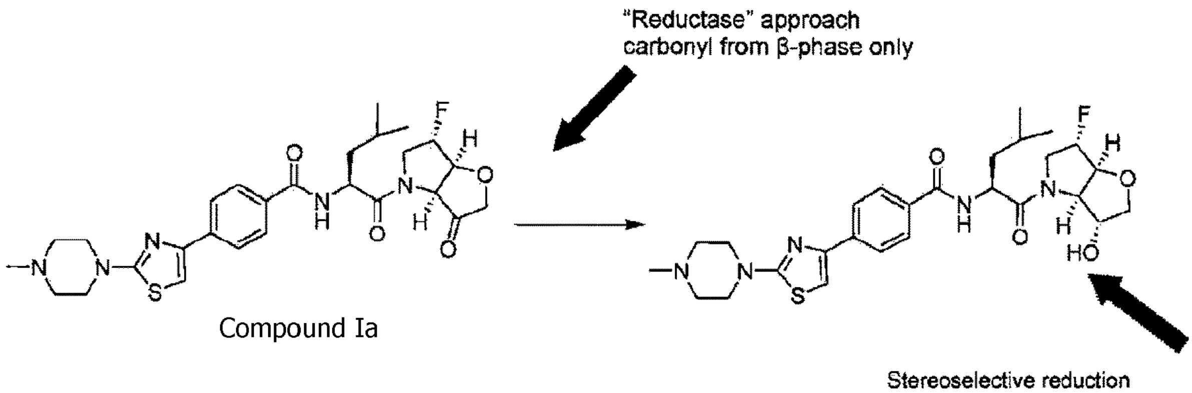


Compound Ia

While many pharmaceutical companies are working on the development of selective inhibitors for cathepsin K in human, there is no FDA approved drug till now. Odanacatib (ODN) developed by Merck & Co. is the only candidate which demonstrated high therapeutic efficacy in patients with postmenopausal osteoporosis in Phase III clinical trials. Unfortunately, the development of ODN was finally terminated due to the cardio-cerebrovascular adverse effects. Therefore, it arouses concerns on the undesirable cathepsin K inhibition in non-bone sites in human (Dai et al. 2020).

## SUMMARY OF THE INVENTION

The Applicants have surprisingly found that notwithstanding the clinical liabilities of the compound in humans, Compound I and pharmaceutically acceptable salts thereof, is efficacious in the treatment of disorders mediated by cathepsin K in animals, such as disorder associated with bone loss and/or a disorder associated with bone resorption, such as a periodontal disease, tooth resorption, arthritis and osteoporosis. In particular, Compound I and pharmaceutically acceptable salts thereof, is particularly efficacious in the treatment of degenerative dental pathologies, including periodontal disease and tooth resorption. Contrary to what was observed in human, the Applicants have surprisingly shown that Compound I and pharmaceutically acceptable salts thereof are particularly stable in canine and feline whole blood.

Therefore, the present invention relates to a compound selected from N-[(S)-1-((3aS,6S,6aS)-6-fluoro-3-oxo-hexahydro-furo[3,2-b]pyrrole-4-carbonyl)-3-methyl-butyl]-4-[2-(4-methyl-piperazin-1-yl)-thiazol-4-yl]-benzamide,

N-[(S)-1-((3aS,6S,6aS)-6-Fluoro-3,3-dihydroxy-hexahydro-furo[3,2-b]pyrrole-4-carbonyl)-3-methyl-butyl]-4-[2-(4-methyl-piperazin-1-yl)-thiazol-4-yl]-benzamide, mixtures thereof, and pharmaceutically acceptable salts thereof, for use in the treatment of a disorder mediated by cathepsin K in an animal.

## DETAILLED DESCRIPTION OF THE INVENTION

The present invention relates to a compound selected from N-[(S)-1-((3aS,6S,6aS)-6-fluoro-3-oxo-hexahydro-furo[3,2-b]pyrrole-4-carbonyl)-3-methyl-butyl]-4-[2-(4-methyl-piperazin-1-yl)-thiazol-4-yl]-benzamide (i.e. Compound Ia), N-[(S)-1-((3aS,6S,6aS)-6-Fluoro-3,3-dihydroxy-hexahydro-furo[3,2-b]pyrrole-4-carbonyl)-3-methyl-butyl]-4-[2-(4-methyl-piperazin-1-yl)-thiazol-4-yl]-benzamide (i.e. Compound Ib), mixtures thereof, and pharmaceutically acceptable salts thereof, for use in the treatment of a disorder mediated by cathepsin K in an animal.

It will be appreciated that Compound Ia (keto form) and Compound Ib (hydrated form) are two alternative forms of Compound I, which may coexist being in equilibrium in solution, and the invention extends to all such alternative forms, and to mixture of these two forms.

For the avoidance of doubt, Compound I refers indifferently to compound Ia, compound Ib or a mixture thereof. The term "animal" refers to any animal, except human. The animal may be a mammal. In a preferred embodiment, the animal is selected from a canine, a feline, a bovine, an ovine, an equine or a rodent, preferably selected from a canine or a feline. Preferably, the animal is a canine or a feline, such as a dog or a cat.

In some embodiments, the disorder mediated by cathepsin K is a disorder associated with bone loss and/or a disorder associated with bone resorption. The term "disorder associated with bone loss" refers to a disorder associated with an imbalance in the ratio of bone formation to bone resorption resulting in less bone than desirable in a subject. The term "disorder associated with bone resorption" refers to a disorder associated with the resorption of bone tissue, that is, the process by which osteoclasts break down the tissue in bones.

In some embodiments, the disorder mediated by cathepsin K is a disorder associated with alveolar bone loss and/or a disorder associated with alveolar bone resorption. The term "alveolar bone" refers to the part of the mandibular and maxillary bone which surrounds the teeth and forms the tooth sockets. Therefore, the term "disorder associated with alveolar bone loss" refers to a disorder associated with an imbalance in the ratio of alveolar bone formation to alveolar bone resorption resulting in less alveolar bone than desirable in a subject. Therefore, the term "disorder associated with alveolar bone resorption" refers to a disorder associated with the resorption of alveolar bone tissue, that is, the process by which osteoclasts break down the tissue in alveolar bone. Currently, there is no effective treatment for disorders associated with alveolar bone loss/resorption. Often, the affected teeth break off, thus creating the potential for root sequestrum formation and subsequent infection. Typically, affected teeth must be extracted because attempts to save the tooth by drilling out the lesion and filling it with restorative materials are ineffective. Approximately 90% of the repair attempts fall out within two years because of ongoing resorption. In addition, new lesions often develop in other teeth within the mouth of an affected individual.

In some embodiments, the disorder mediated by cathepsin K is a periodontal disease, such as gingivitis and/or periodontitis. The term "periodontal disease" refers to an infection and inflammation of the periodontium (the tissues that surround and support the teeth) due to plaque bacteria and the host's response to the bacterial insult. Gingivitis is common in dogs and cats and refers to inflammation of the gingiva in response to plaque antigen. Periodontitis is a more severe disease that involves inflammation of the periodontal ligament and alveolar bone, eventually causing loss of attachment (periodontal pocketing, gingival recession, bone resorption).

In some embodiments, the disorder is a disorder associated with alveolar bone loss and periodontal disease.

In some embodiments, the disorder mediated by cathepsin K is tooth resorption, such as dentin resorption and/or root resorption. The term "tooth resorption" refers to the progressive loss of dentin and cementum by the action of odontoclasts. It can occur on the external or internal tooth surface (external or internal resorption). Odontoclast activity can be stimulated by inflammation, pressure from adjacent structures, orthodontic tooth movement, as a result of normal processes such as exfoliation of deciduous teeth, or in the absence of these processes (idiopathic).

In some embodiments, the tooth resorption may be a Feline Odontoclastic Resorption Lesions (FORL) or a canine tooth resorption. FORL is a syndrome in cats characterized by resorption of the tooth by odontoclasts. Although odontoclasts share an embryogenic origin with the osteoclasts responsible for human osteoporosis, they are recognized as a distinct cell population and pathology. Indeed, odontoclastic resorption lesions are extremely rare in humans, even those suffering from advanced osteoporosis. FORL is a huge clinical problem in cats affecting one-third of all domestic cats older than 1 year and at 10 years 75% of all cats are affected. Certain breeds seem to have a tendency towards the condition, including Abyssinians, Asian shorthairs, Persians, and Siamese. Most cats which present with lesions consistent with FORL also have periodontal disease.

In some embodiments, the disorder mediated by cathepsin K is arthritis, such as osteoarthritis or rheumatoid arthritis. The term "arthritis" refers to an inflammation of a joint. Osteoarthritis is the most common form of arthritis. It occurs when the protective cartilage that cushions the ends of the bones wears down over time. Rheumatoid arthritis is an

autoimmune and inflammatory disease causing inflammation in the affected parts of the body. Rheumatoid arthritis mainly attacks the joints. In a joint with rheumatoid arthritis, the lining of the joint becomes inflamed, causing damage to joint tissue. This tissue damage can cause long-lasting or chronic pain, unsteadiness (lack of balance), and deformity (misshapenness).

In some embodiments, the disorder mediated by cathepsin K is osteoporosis. The term "osteoporosis" refers to a systemic skeletal disorder characterized by low bone mass, micro-architectural deterioration of bone tissue leading to bone fragility, and consequent increase in fracture risk.

The term "pharmaceutically acceptable salt" refers to salts which are, within the scope of sound medical judgment, suitable for use in contact with the tissues of human beings and animals without excessive toxicity, irritation, allergic response, or other problem or complication, and commensurate with a reasonable benefit/risk ratio. The pharmaceutically acceptable salt may be derivatives of Compound I wherein the parent compound is modified by making acid or base salts thereof. Examples of pharmaceutically acceptable salts include, but are not limited to, mineral or organic acid salts of basic residues such as amines; alkali or organic salts of acidic residues such as carboxylic acids; and the like. For example, such salts include acetates, ascorbates, benzenesulfonates, benzoates, besylates, bicarbonates, bitartrates, bromides/hydrobromides, Ca-edetates/edetates, camsylates, carbonates, chlorides/hydrochlorides, citrates, edisylates, ethane disulfonates, estolates esylates, fumarates, gluceptates, gluconates, glutamates, glycolates, glycollylarsnilates, hexylresorcinates, hydrabamines, hydroxymaleates, hydroxynaphthoates, iodides, isothionates, lactates, lactobionates, malates, maleates, mandelates, methanesulfonates, mesylates, methylbromides, methyl nitrates, methylsulfates, mucates, napsylates, nitrates, oxalates, pamoates, pantothenates, phenylacetates, phosphates/diphosphates, polygalacturonates, propionates, salicylates, stearates subacetates, succinates, sulfamides, sulfates, tannates, tartrates, teoclates, toiuenesulfonates, triethiodides, ammonium, benzathines, chloroprocaines, cholines, diethanolamines, ethylenediamines, meglumines and procaines. Further pharmaceutically acceptable salts can be formed with 30 cations from metals like aluminium, calcium, lithium, magnesium, potassium, sodium, zinc and the like. (also see Pharmaceutical salts, Birge, S.M. et al., J. Pharm. Sci., (1977), 66, 1-19).

In a preferred embodiment, the pharmaceutically acceptable salt of Compound I is the besylate salt of Compound I.

The term "treat" or "treatment" encompasses any beneficial effect or desirable effect on a pathology or a pathological state and may even include a minimal reduction in one or more measurable markers of the pathology or of the pathological condition. The treatment may, for example, involve either the reduction or improvement in symptoms of the pathology or the pathological state, or slowing of the progression of the disease or the pathological state. The term "treatment" does not necessarily mean the complete eradication or healing of the disease, nor of the associated symptoms. Those skilled in the art will recognise that the term "treatment" may also be extended to cover prophylaxis, that is prevention or retardation of the development of a pathology or a pathological state.

Compound I may be prepared by solution or solid phase synthetic procedures which are known to those skilled in the art, for example, according to the procedure described in WO2005/066180 (summarised in Scheme 1 below).

## Scheme 1

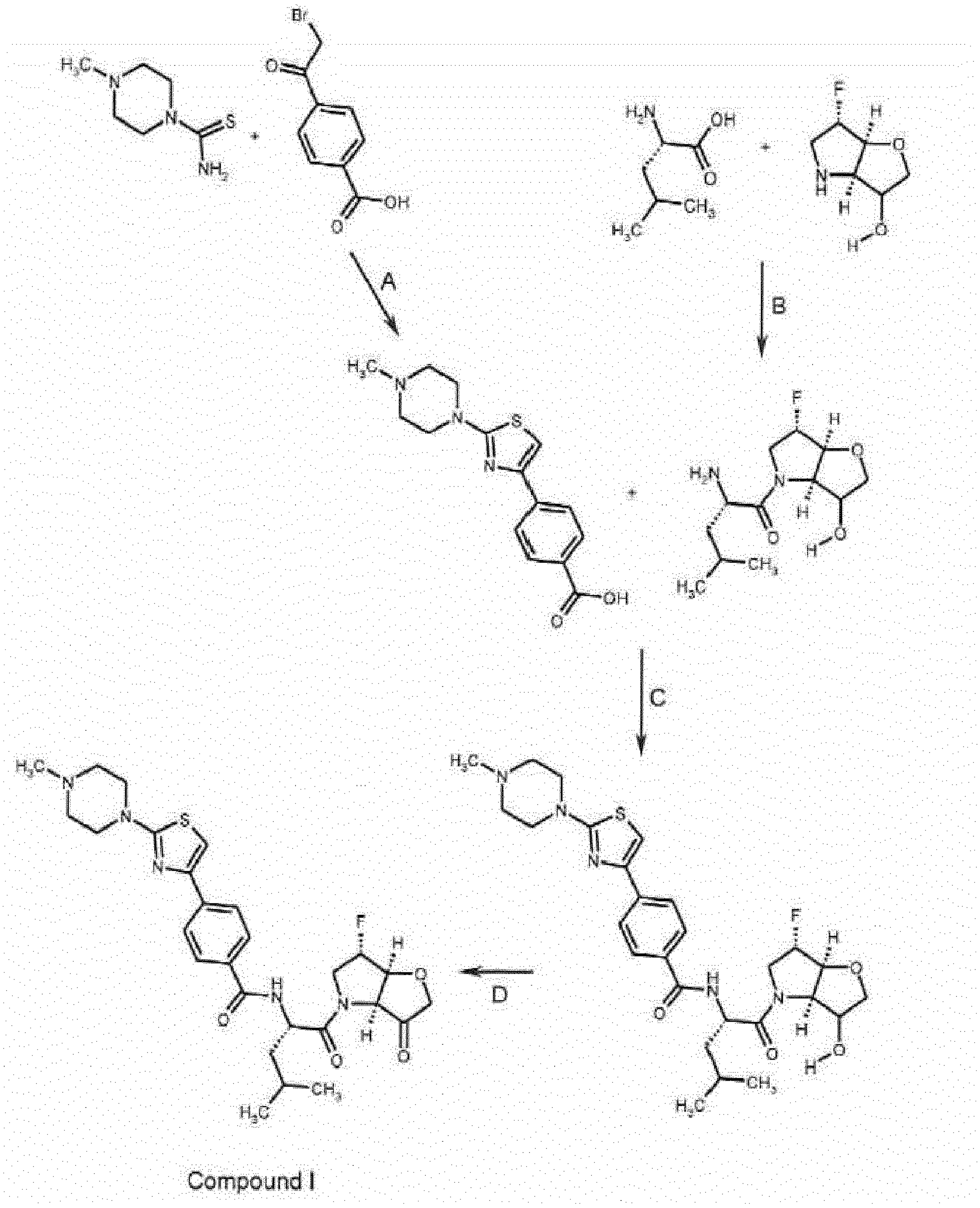

Compound I

For the avoidance of doubt, besylate salt of Compound I refers indifferently to the besylate salt of Compound Ia, the besylate form of Compound Ib or a mixture thereof.

Compound I benzenesulphonate (i.e. besylate salt of Compound I) may be prepared by the reaction of Compound I free base with benzenesulphonic acid. Typically, Compound I free base is dissolved in a suitable solvent (for example dichloromethane) before mixing with benzenesulphonic acid in a suitable solvent (for example tetrahydrofuran). In such circumstances, the Compound I benzenesulphonate may form a precipitate and, after allowing sufficient time for reaction to occur, may be separated by filtration.

In some embodiments, the ratio Compound Ia/Compound Ib (or the ratio pharmaceutically acceptable salt of Compound Ia/pharmaceutically acceptable salt of Compound Ib) in the mixture thereof is from 1/99 to 50/50, such as from 5/95 to 40/60, such as from 10/90 to 20/80, for example the ratio is 5/95, 10/90, 15/85 or 20/80.

Additional solvents in which Compound I free-base may be dissolved include acetone, and also mixtures of acetone and with methyl-tert-butyl ether (MTBE), methyl-ethyl-ketone (MEK) or methyl-isobutyl-ketone (MIBK).

Compound I or the pharmaceutically acceptable salt thereof may be administered to the animal, such as a cat or a

dog, in isolation, or typically be presented as part of a composition, such as a pharmaceutical composition. Such a composition will comprise Compound I or a pharmaceutically acceptable salt thereof together with one or more pharmaceutically acceptable excipients. Said pharmaceutically acceptable excipients will be suitable for administration and will be compatible with the other ingredients of the composition.

One or more pharmaceutically acceptable excipients must be adapted to the animal to be treated. For example, dogs and cats cannot tolerate any dental product with mint flavouring. Another example, Xylitol is a common ingredient found in human dental products including toothpastes and mouthwashes due to its anti-cavity properties; however, xylitol has been shown to be toxic to dogs producing liver damage and anaemia. Moreover, animals cannot expectorate dental product and therefore ingest all of the residual material. Although, little specific research has been performed as to the effects of fluoride on dog and cats, more care should be taken in the use of fluoride containing products in companion animals until its safe use has been established.

In some embodiments, the pharmaceutical composition may also comprise other therapeutic ingredients. Examples of other therapeutic ingredients may be antibiotics, non-steroidal-anti-Inflammatory drugs (ibuprofen, diclofenac, narpoxen), corticosteroids, methotrexate, IL-1 receptor antagonists (anakinra), TNF inhibitors (etanercept, certolizumab), JAK inhibitors (rituximab), hormone replacement therapy (estrogen, progesterone), bisphosphonates (alendronate, etidronate, ibandronate, pamidronate, risedronate, and zoledronic acid), anti-RANKL immunotherapy (denosumab), selective estrogen receptor modulators (raloxifene, bazedoxifene, lasofoxifene, tamoxifene), calcitonin, strontium ranelate, para-thyroid hormone (teriparatide, abaloparatide) sclerotine inhibitors (romosozumab, blosozummab).

In some embodiment, Compound I may be administered by any suitable route, such as oral, rectal, nasal, topical (including buccal and sublingual), vaginal or parenteral (including subcutaneous, intramuscular, intravenous and intradermal) administration.

In some embodiments, the composition includes those suitable for oral, rectal, nasal, topical (including buccal and sublingual), vaginal or parenteral (including subcutaneous, intramuscular, intravenous and intradermal) administration. In a preferred embodiment, Compound I or pharmaceutically acceptable salt is administered orally or systemically.

The pharmaceutical composition may be suitable for the administration route, for example the pharmaceutical composition is suitable for oral administration. The compositions may conveniently be presented in unit dosage form (e.g. tablets and sustained release capsules) and may be prepared by any method known in the art of pharmacy.

Compositions for oral administration in the present invention may be presented as discrete units such as capsules, cachets or tablets each containing a predetermined amount of the active agent; as a powder or granules; as a solution or a suspension of the active agent in an aqueous liquid or a non-aqueous liquid; or as an oil-in-water liquid emulsion or a water in oil liquid emulsion and as a bolus etc.

Alternatively, the composition may be nutraceutically formulated, together with food, supplements, chew sticks or other veterinary products including toothpastes and dental prophylaxis pastes. A large number of taste masking and flavour enhancing additives (palatants) are commercially available for feline and canine products, from companies such as AFB International, Firmenich and SPF.

With regard to compositions for oral administration (e.g. tablets and capsules), the term excipient includes: binding agents, for example syrup, acacia, gelatine, sorbitol, tragacanth, polyvinylpyrrolidone (Povidone), methylcellulose, ethylcellulose, sodium carboxymethylcellulose, hydroxypropylmethylcellulose, sucrose and starch; fillers and carriers, for example corn starch, gelatine, lactose, sucrose, microcrystalline cellulose, kaolin, mannitol, dicalcium phosphate, sodium chloride and alginic acid; and lubricants such as magnesium stearate, sodium stearate and other metallic stearates, glycerol stearate stearic acid, silicone fluid, talc waxes, oils and colloidal silica. Palatants, as mentioned above for nutraceuticals can also be used. It may be desirable to add a colouring agent to make the dosage form readily identifiable. Tablets may also be coated by methods well known in the art.

A tablet may be made by compression or moulding, optionally with one or more accessory ingredients. Compressed tablets may be prepared by compressing in a suitable machine the Compound I or pharmaceutically acceptable salt thereof in a free-flowing form such as a powder or granules, optionally mixed with a binder, lubricant, inert diluent, preservative, surface-active or dispersing agent. Moulded tablets may be made by moulding in a suitable machine a mixture of the powdered compound moistened with an inert liquid diluent. The tablets may be optionally coated or scored and may be formulated so as to provide slow or controlled release of the active agent.

Other formulations suitable for oral administration include lozenges comprising the Compound I in a palatant flavoured base, usually a veterinarily palatable carbohydrate and acacia or tragacanth; pastilles comprising the Compound I benzenesulphonate in an inert base such as gelatine and glycerin, or sucrose and acacia; and mouthwashes comprising the Compound I in a suitable liquid carrier.

In some embodiments, the Compound I or pharmaceutically acceptable salt is administered in a unit dosage form, preferably as capsule or tablets.

Appropriate dosage levels for the Compound I or pharmaceutically acceptable salt thereof will depend, for example, upon the disorder to be treated, the stage of advancement of the disorder, the mode of administration and the age

and the weight of the animal receiving treatment. Suitable dosages may be determined by conventional animal trials. As an example, the compound or pharmaceutically acceptable salt may be administered, preferably orally, in a daily amount corresponding to 1-300 mg/kg, generally from 10 mg/kg to 250 mg/kg, typically from 25 mg/kg to 200 mg/kg, such as from 25 mg/kg to 150 mg/kg, for example from 50- mg/kg to 100 mg/kg.

In some specific embodiments, the animal is a dog and the compound or pharmaceutically acceptable salt is administered, preferably orally, in a daily amount corresponding to 20-75 mg/kg, for example from 25 mg/kg to 50 mg/kg, such as 25 mg/kg or 50 mg/kg. In some embodiments, the CTX-1 (Carboxy-terminal crosslinked telopeptide of type 1 collagen) plasma concentration in the animal is decreased by at least 10% compared to the CTX-1 plasma concentration before treatment, such as at least 15%, at least 20%, at least 25%, at least 30%, at least 30%, at least 35%, at least 40%, at least 45%, at least 50%, at least 55%, at least 60%, at least 65%, at least 70%, at least 75%, at least 80%, at least 85%.

## BRIEF DESCRIPTION OF THE FIGURES

Figure 1: Time course of MIV-701 plasma concentration evolution after single (D0) and repeated oral administration of MIV-701 besylate salt at 1mg/kg, 10 mg/kg and 100 mg/kg in healthy beagle dogs.

Figure 2: Relation between oral dose of MIV-701 besylate salt and MIV-701 daily plasma exposure after single (D0) or repeated (D13) administrations in healthy beagle dogs.

Figure 3: 24-hour evolution of CTX-1 plasma concentration after single (D0) oral administration of MIV-701 besylate salt (ng/mL) at 1 mg/kg, 10 mg/kg and 100 mg/kg in healthy beagle dogs.

Figure 4: 24-hour evolution of CTX-1 plasma concentration after single (D0) oral administration of MIV-701 besylate salt (% of baseline) at 1 mg/kg, 10 mg/kg and 100 mg/kg in healthy beagle dogs.

Figure 5: Evolution of pre-dose CTX-1 plasma concentration during 14-day treatment period with once-a-day oral administration of MIV-701 besylate salt at 1mg/kg/day and 10 mg/kg/day in healthy beagle dogs.

Figure 6: Time course of MIV-701 plasma concentration evolution after single (D0) and repeated (D13) oral administration of MIV-701 besylate salt at 2mg/kg, 10 mg/kg and 50 mg/kg in healthy cats.

Figure 7: Relation between oral dose of MIV-701 besylate salt and MIV-701 daily plasma exposure 1 day (D0) or 14 days (D14) after administration of MIV-701 besylate salt in healthy cats.

Figure 8: 24-hour evolution of CTX-1 plasma concentration after single (D0) oral administration of MIV-701 besylate salt (% of baseline) at 1 mg/kg, 10 mg/kg and 50 mg/kg in healthy cats (mean; n=4)

Figure 9: Evolution of pre-dose CTX-1 plasma concentration during 14-day treatment period with once-a-day oral administration of MIV-701 besylate salt at 2 mg/kg/day and 10 mg/kg/day in healthy cats.

Figure 10: Study schedule of the clinical trial in dogs with alveolar bone loss induced by spontaneously developed periodontal disease.

Figure 11: Occlusal view of a dog dentition marked (red marks) with specific examination sites (from Kortegaard et al. 2014).

Figure 12: Sagittal cone beam tomograms showing the measurements of the distances A) from the CEJ to the AC in the mesial surface of tooth, B) from the CEJ to the bottom of the defect, and C) the width of the defect in the distal surface of tooth (from De Faria Vasconcelos et al. 2012).

Figure 13: Periapical radiographs illustrating distances A) from CEJ to AC in the mesial surface of tooth, B) from CEJ to the bottom of the defect, and C) the width of the defect on the mesial surface of tooth (from De Faria Vasconcelos et al. 2012).

Figure 14: CTX-1 plasma levels at Day-0 (pre-treatment), Day-30 and Day-60 in all dogs included in the study (25 mg/kg/day-group and 50 mg/kg/day-group with median $\pm$ 95% CI.

Statistical difference between groups was assessed according to Man-Whitney test with correction for multiple comparisons using the Holm-Sidak method. Ns=non-significant (p>0.05).

Figure 15: CTX-1 plasma levels at Day-0 (pre-treatment), Day-30 and Day-60 in all dogs included in the study (25 mg/kg/day and 50mg/kg/day groups were combined at Day-30 and Day-60 respectively while all dogs were on 50mg/kg/day at Day-90) with median ± 95%CI.

Statistical difference between groups was assessed according to Wilcoxon matched-pairs signed rank test (N=10).

Figure 16: Width of alveolar pocket measured before (Day-0; pre-treatment) and after 90-day oral administration of MIV-701 besylate salt in CTX-1-responders (N=9) using CBCT-scan (left) and X-ray radiography (right). Results are expressed as Median tCI 95%. Significance was assessed using Wilcoxon matched-pairs signed rank test. Mean = mean of mesial + distal measures for each tooth; N=27 (9 dogs; 3 teeth per dog).

Figure 17: Depth of alveolar pocket measured before (Day-0; pre-treatment) and after 90-day oral administration of MIV-701 besylate salt in CTX-1-responders using CBCT-scan (left) and X-ray radiography (right). Results are expressed as Median tCI 95%. Significance was assessed using Wilcoxon matched-pairs signed rank test. Mean = mean of mesial + distal measures for each tooth; N=27 (9 dogs; 3 teeth per dog).

Figure 18: Height of alveolar pocket measured before (Day-0; pre-treatment) and after 90-day oral administration of MIV-701 besylate salt in CTX-1-responders using CBCT-scan (left) and X-ray radiography (right). Results are expressed as Median tCI 95%. Statistical significance was assessed using Wilcoxon matched-pairs signed rank test. Mean = mean of mesial + distal measures for each tooth; N=27 (9 dogs; 3 teeth per dog).

Figure 19: Clinical Attachment Loss (CAL), Periodontal Probing Depth (PPD) and Gingival Bleeding Index (GBI) measured before (Day-0; pre-treatment) and after 90-day oral administration of MIV-701 besylate salt in CTX-1-responders. Results are expressed as Median tCI 95%. Statistical significance was assessed using Wilcoxon matched-pairs signed rank test. Mean = mean of mesial + distal measures for each tooth; N=27 (9 dogs; 3 teeth per dog).

## EXAMPLES

**Example 1: Synthesis of N-[(S)-1-((3aS,6S,6aS)-6-Fluoro-3-oxo-hexahydrofuro[3,2-b]pyrrole-4-carbonyl)-3-methyl-butyl]-4-[2-(4-methyl-piperazin-1-yl)-thiazol-4-yl]- 10 benzamide free base.**

Compound I free base is prepared using the method summarised in Scheme 2 below (steps 15 to 20).

## Scheme 2

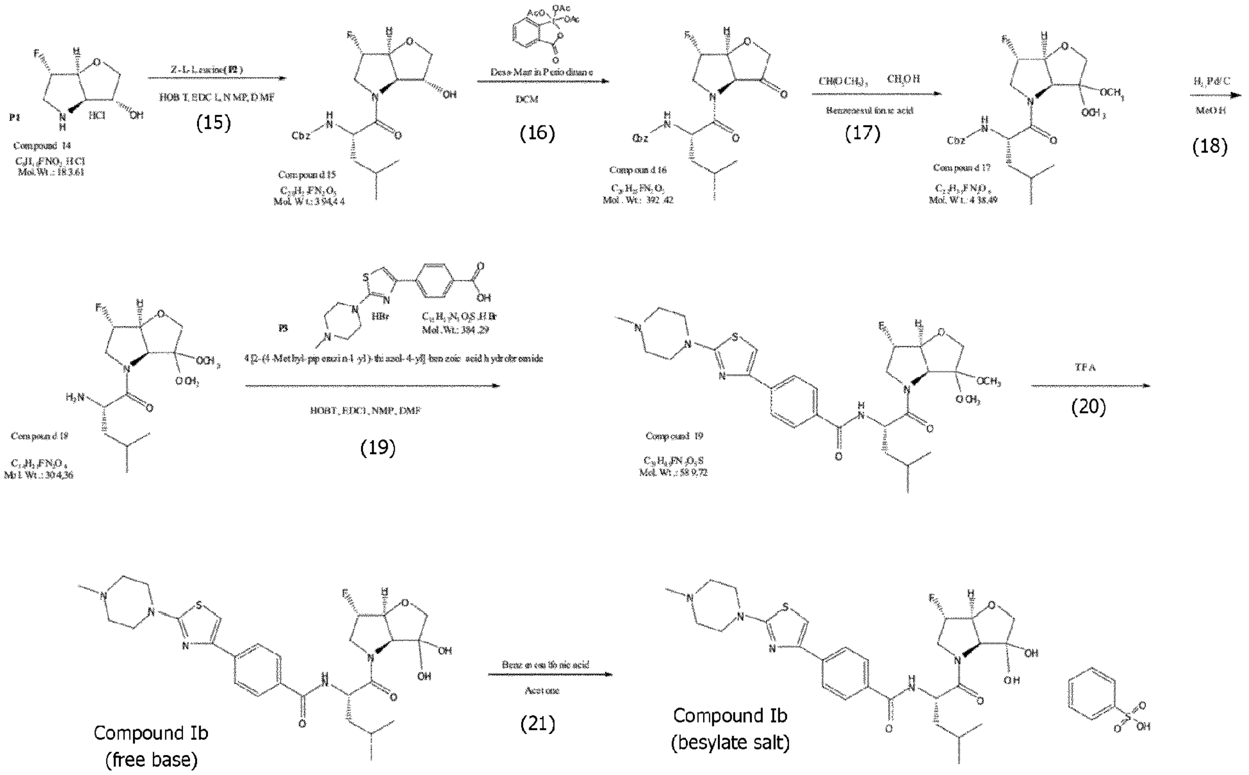


Reaction steps:

15) N-carbobenzyloxy-l-leucine, 1-hydroxybenzotriazole hydrate, N-(3-dimethylaminopropyl)-N'-ethylcarbodiimide, 4-methyl morpholine, N.N-dimethylformamide

16) Dess-martin periodinane, dichloromethane

17) Trimethylorthoformate, benzenesulphonic acid, methanol

18) Palladium on carbon, hydrogen, methanol

19) 4-[2-(4-Methyl-piperazin-1 -yl)-thiazol-4-yl]-benzoic acid hydrobromide, 1-hydroxybenzotriazole hydrate, N-(3-dimethylaminopropyl)-N'-ethylcarbodiimide, 4-methyl morpholine, N.N-dimethylformamide

20) Trifluoroacetic acid

21) Benzene sulphonic acid, acetone, 2% water.

A representative synthesis of compound 1 as the free base (i.e. steps 15-20 of Scheme 2) is described immediately below. The salt form can be subsequently prepared as described in Example 2 below.

Initial synthetic steps to synthesise the bicyclic moiety (3R,3aR,6S,6aS)-6-Fluoro-hexahydrofuro[3,2-b]pyrrol-3-ol hydrochloride were derived from the synthesis described in WO 2005/066180 (Compound 64, page 42).

**Synthesis of [1S-1-((3R,3aR,6S,6aS)-6-Fluoro-3-hydroxy-hexahydro-furo[3,2-b]pyrrole-4- carbonyl)-3-methyl-butyl]-carbamic acid benzyl ester**

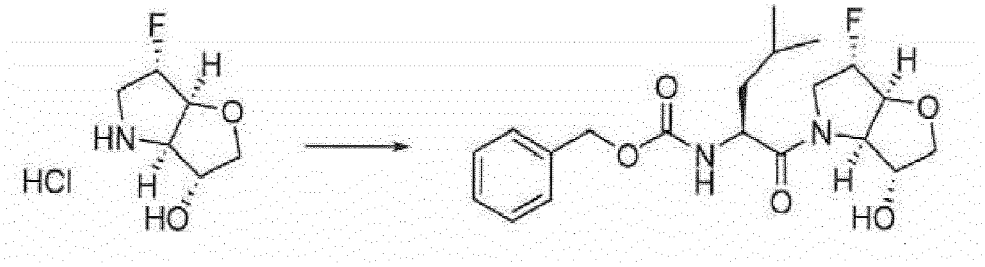

A 160 L glass-lined steel reactor set-up for cooling to -10 °C and heating to 70 °C is charged with N.N-dimethylformamide (7.9 kg) and (3R,3aR,6S,6aS)-6-fluoro-hexahydro-furo[3,2-b]pyrrol- 3-ol hydrochloride (1.9 kg, 10.3 mol). To the suspension is added 1-hydroxybenzotriazole (12 % H2O) (1.6 kg, 10.6 mol), Z-L-Leucine (94 % pure, 2.9 kg, 10.3 mol) followed by N-(3-dimethylaminopropyl)-N'-ethyl-carbodiimide hydrochloride (3.6 kg, 18.8 mol). The reaction mixture is kept under nitrogen, and the temperature is adjusted to 20-25 °C. Over a period of 1 - 2 hours, 4-methylmorpholine (1.6 kg, 15.8 mol) is added. The resulting solution is allowed to react for 4 to 24 hours. The course of the reaction is followed by TLC until the reaction is deemed complete (typically 10 to 24 hours). The completed reaction mixture is quenched using tap water (79 kg). The product is extracted into dichloromethane (4x13 kg). The organic phases are washed with 1M hydrochloric acid (2x10 kg) and 6 % sodium hydrogencarbonate (10.6 kg). The organic extracts are combined, and the solvent is removed by distillation at reduced pressure, finally at 50 - 60°C and at a pressure ≤150 mbar. The residue is dissolved in tert-butyl methyl ether (10 kg) and washed with tap water (2x13 kg) and saturated with 25 % sodium chloride (5.2 kg). The aqueous phases are extracted with tert-butyl methyl ether (10 kg). The organic phases are combined, and the solvent is removed by distillation to dryness at reduced pressure, finally at 50 - 60°C and at a pressure s 150 mbar. Toluene (5 kg) is added, and distillation is continued as above. The distillation residue is re-dissolved in toluene (8 kg) and purified by column chromatography on a column prepared from silica gel 60, 40-63 μ (24 kg) and toluene (53-74 kg). First the column is eluted with a mixture of toluene (251 kg) and ethyl acetate (29 kg), then with a mixture of toluene (168 kg) and ethyl acetate (116 kg). Fractions of approximately 10 L, are taken and analysed by TLC. Sufficiently pure fractions (purity ≥ 90 %) are pooled, and the solvent is removed by distillation at reduced pressure, finally at 50 - 60°C and p ≤ 50 mbar. The residue is re-dissolved in dichloromethane (25 kg). A sample of this solution is used for estimating the yield 3.8 kg (94 %).

**Synthesis [(S)-1-((3aS,6S,6aS)-6-fluoro-3-oxo-hexahydro-furo[3,2-b]pyrrole-4-carbonyl)-3-methyl-butyl]-carbamic acid benzyl ester**

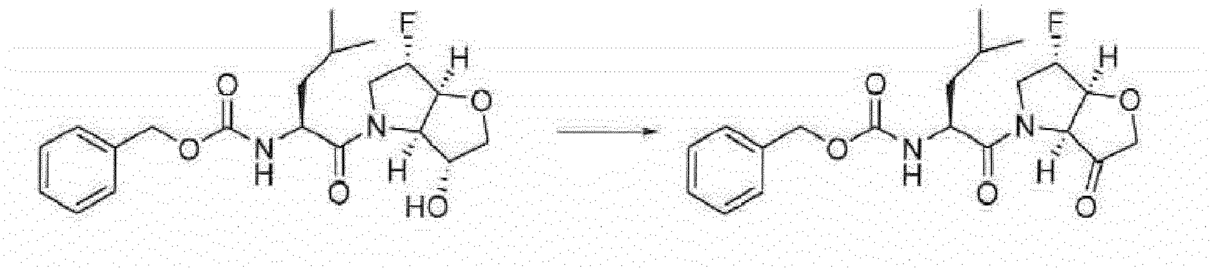

A 63 L glass-lined steel reactor set up for cooling to -10°C and heating to 70°C is charged with the dichloromethane solution of [1S-1-((3R,3aR,6S,6aS)-6-fluoro-3-hydroxy-hexahydrofuro[3,2b]pyrrole-4-carbonyl)-3-methyl-butyl]-carbamic acid benzyl ester (neat 3.8 kg, 9.6 mol). Dess-Martin periodinane (4.6 kg, 10.8 mol) is added. The suspension is kept under nitrogen and the temperature is adjusted to 20 - 25°C. The reaction is stirred for 10 to 24 hours. The course of the reaction is followed by LC-MS. The reaction is stirred for another 4-24 hours. The reaction mixture is quenched with a suspension of sodium hydrogen carbonate (2.4 kg, 28.6 mol) and tap water (26 kg). The reaction mixture is stirred for 2 to 4 hours, and celite 545 (2.1 kg) is added. The suspension is filtered on a pad of celite 545 back into the reactor. The lower, organic phase is separated and secured. The filter cake is washed with dichloromethane (26 - 40 kg). The filtrate is used to wash the upper, aqueous phase. The lower, organic phase is separated and secured. The organic phases are washed with 6 % sodium hydrogen carbonate (26 kg), and then washed with 25 % sodium chloride (5.2 kg). The organic phases are combined and dried with magnesium sulfate (3-5 kg). The suspension is filtered. The filter cake is washed with dichloromethane (10-15 kg). The filtrate and wash are combined and evaporated by distillation at reduced pressure until a final volume of 10 - 15 L, using a jacket temperature of 50 - 60°C. Methanol (15-20 kg) is added to the residue and

concentrated to a final volume of 15 - 20 L by distillation at reduced pressure, using a jacket temperature of 50 - 60°C. A sample of this solution is used for estimating the yield 4.3 kg (113 %).

**[(S)-1-((3aS,6S,6aS)-6-Fluoro-3-3-dimethoxy-hexahydro-furo[3,2-b]pyrrole-4-carbonyl)-3-methyl-butylj-carbamic acid benzyl ester**

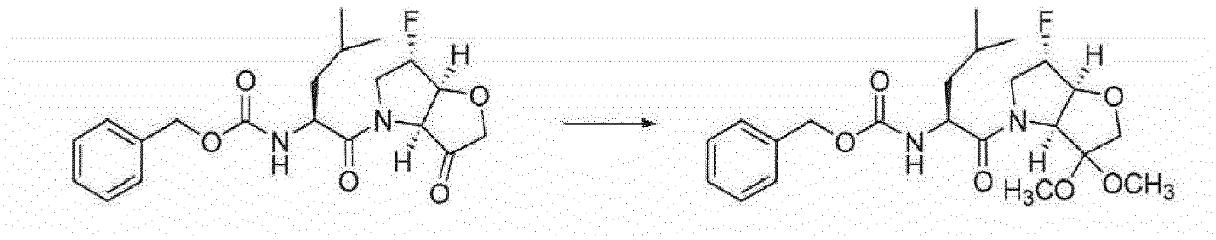

A 63 L glass-lined steel reactor set up for cooling to -10°C and heating to 70°C is charged with the methanol solution of [1S-1-((3aS,6S,6aS)-6-fluoro-3-oxo-hexahydro-furo[3,2-b]pyrrole-4-(carbonyl)-3-methyl-butyl]-carbamic acid benzyl ester (neat 4.3 kg, 11.0 mol). Trimethyl orthoformate (6 kg, 56.5 mol) is added. Benzenesulfonic acid (0.2 kg, 1.3 mol) is added. The mixture is kept under nitrogen, and the temperature is adjusted to 40 - 60°C. The reaction is continued for 4 to 8 hours (acceptance criteria; absent or barely visible, based on TLC), allowing the formed methyl formate to distil off.

The solvent is removed by distillation at reduced pressure, finally at pressure ≤ 50 mBar and at a jacket temperature of 50 - 60°C. The residue is dissolved in toluene (8 kg). The resulting solution is quenched on 6 % sodium hydrogencarbonate (11 kg). The phases are separated, and the aqueous phase is extracted with additional toluene (4 kg). The organic phases are washed with 25% sodium chloride solution (5.2 kg). The organic phases are combined, and the solvent is removed by distillation at reduced pressure, finally at p ≤ 50 mBar and at a jacket temperature of 50 - 60°C. The evaporation residue is re-dissolved in toluene (4.5 kg). The crude product solution is purified on a column prepared from silica gel 60 (21 kg) and toluene (45 kg). The toluene solution of the crude product is applied to the column. The column is eluted with toluene (95 kg), 2.5 % (v/v) acetone in toluene (230 kg) and then with 5 % (v/v) acetone in toluene (138 kg). Fractions of approximately 10 L are taken. Fractions which look pure by TLC 10 (purity > 90 % by TLC) are pooled, and the solvent is removed by distillation at reduced pressure, finally at p ≤ 50 mBar, and at a jacket temperature of 50 - 60°C. The residue is re- dissolved in methanol (10 kg). A sample of this solution is used for estimating the yield - 4.3 kg (90 %).

**(S)-2-Amino-1-((3aS,6S,6aS)-6-fluoro-3,3-dimethoxy-hexahydro-furo[3,2-b]pyrrol-4-yl)-4-methyl-pentan-1-one**

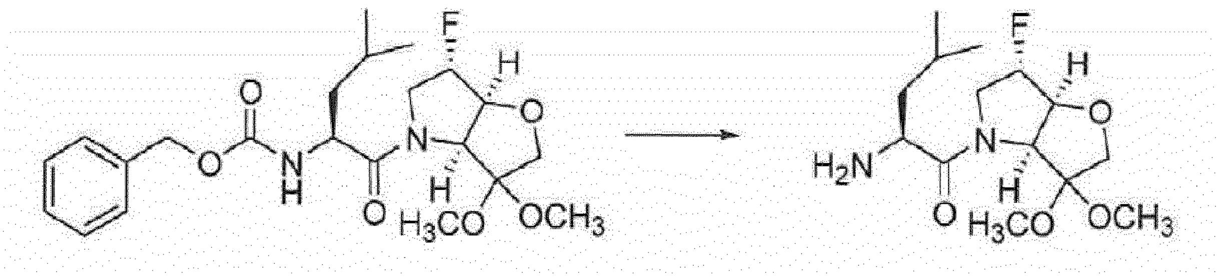

A 63 L glass-lined steel reactor set up for cooling to -10°C and heating to 70°C is filled with nitrogen and charged with 10 % palladium on charcoal (0.17 -0.18 kg). The methanolic solution of [1S-1-((3aS,6S,6aS)-6-fluoro-3,3-dimethoxy-hexahydro-furo-[3,2-b]pyrrole-4-carbonyl)-3-methyl-butylj-carbamic acid benzyl ester (neat 3.5 kg, 8.0 mol) is added. The solution is kept under hydrogen, and the temperature is adjusted to 20 - 30°C. The hydrogenation is continued in a stream of hydrogen for 4 to 8 hours. The course of the reaction is followed by TLC and when deemed to have reached completion, the reaction mixture is filtered through celite (2 - 3 kg). The filter cake is washed with methanol (5-10 kg). The filtrates are combined, and the 25 solvent is removed by distillation at reduced pressure, finally at a pressure ≤ 50 mBar, and at a jacket temperature of 50 - 60°C. The evaporation residue is dissolved in toluene (3-6 kg) and sampled for yield and purity determination. The solvent is removed at reduced pressure, finally at a pressure ≤ 50 mBar and at a jacket temperature of

50 - 60°C. The evaporation residue is dissolved in N.N-dimethylformamide (25 - 26 kg). Yield of desired product is 2.2 kg (91 %).

**N-[(S)-1-((3aS,6S,6aS)-6-Fluoro-3,3-dimethoxy-hexahydro-furo[3,2-b]pyrrole-4-carbonyl)-3-methyl-butyl]-4-[2-(4-methyl-piperazin-1-yl)-thiazol-4-yl]-benzamide**

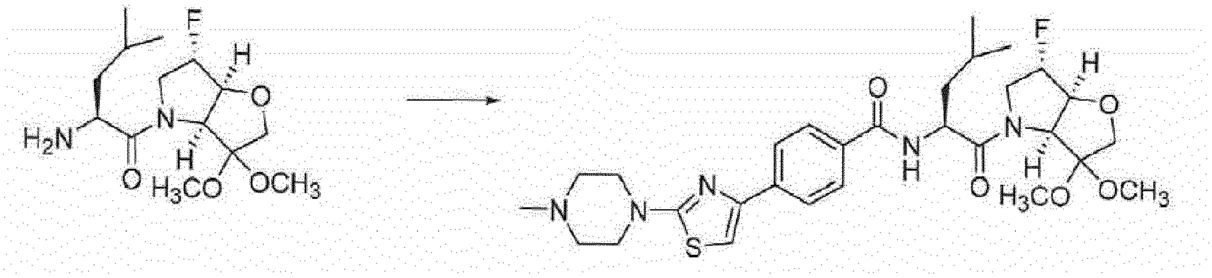

A 160 L glass-lined steel reactor set up for cooling to -10°C and heating to 70 °C is filled with nitrogen and charged with the N.N-dimethylformamide solution of 2-amino-[1S-1-((3aS,6S,6aS)-fluoro-3,3-dimethoxy-hexahydro-furo-[3,2-b]pyrrole-4-yl)-4-methyl]-pentan-1-one (~ 50 kg, neat ~ 3.5 kg, 11.5 mol). 1-Hydroxybenzotriazole, 12 % water (2.05 kg, 13.5 mol), 4-[2-(4-methyl-1- piperazinyl)-4-thiazolyl]benzoic acid, hydrobromide (4.45 kg, 11.6 mol) and N-(3- dimethylaminopropyl)-N'-ethyl-carbodiimide, hydrochloride (2.6 kg, 13.6 mol) are added. The mixture is stirred at 15 - 20°C under nitrogen. 1-Methylmorpholine (1.20 kg, 11.9 mol) is added over a period of 12 hours. The mixture is then stirred at 15 - 20°C for a minimum of 4 hours. A sample of the reaction mixture is taken for IPC. Once the reaction is deemed to have reached completion, the reaction mixture is quenched on a mixture of tap water (120 kg), sodium hydrogencarbonate (7.4 kg) and tert-butyl methyl ether (43 kg). The organic phase is separated. The aqueous phase is further extracted with tert-butyl methyl ether (3 × 21 kg). The organic phases are initially washed with tap water (81 kg), then with a mixture of tap water (41 kg) and sodium hydrogencarbonate (0.4 kg) and finally with 25 % sodium chloride (20 kg). The organic phases are combined, and the solvent is removed by distillation at reduced pressure, finally at 50 - 60°C and p ≤ 100 mbar. The residue is dissolved in dichloromethane (55 kg) and then stirred with a mixture of tap water (50 kg) and 25 % ammonium hydroxide (0.20 kg) for 5 -10 minutes. The organic phase is separated. The aqueous phase is further extracted with dichloromethane (2 × 20 kg). The organic phases are washed once more with a mixture of tap water (50 kg) and 25 % ammonium hydroxide (0.20 kg). The organic phases are checked by HPLC to ensure that a maximum of 2 % of (4-[2-(4-methy]-1-piperazinyl)-4-thiazolyl]benzoic acid, hydrobromide remains in the solution. The organic phases are combined and dried with magnesium sulfate (5 kg). The suspension is filtered, and the filter cake is washed with dichloromethane (20 kg). Toluene (15 kg) is added to the filtrate and the wash. The solvent is removed by distillation at reduced pressure, finally at 50 - 60°C and p ≤ 100 mbar. The residue is dissolved in a mixture of toluene (5.55 kg) and acetone (1.30 kg) and purified on a column prepared from silica gel 60 (63 kg) and a mixture of toluene (104 kg) and acetone (24 kg). The column is eluted with a mixture of toluene (945 kg) and acetone (216 kg), followed by toluene (627 kg) and acetone (380 kg), and finally acetone (400 kg). The pure fractions (purity ≥ 90 %) are selected by HPLC, and the solvent is then removed by distillation at reduced pressure, finally at 50 - 60°C and p ≤ 100 mbar. The residue is dissolved in dichloromethane (7 kg). Yield: 5.2 kg (76 %) (based on an evaporation residue).

**N-[(S)-1-((3aS,6S,6aS)-6-Fluoro-3,3-dihydroxy-furo[3,2-b]pyrrole-4-carbonyl)-3-methyl-butyl]-4-[2-(4-methyl-piperazin-1-yl)-thiazol-4-yl]-benzamide (Compound Ib)**

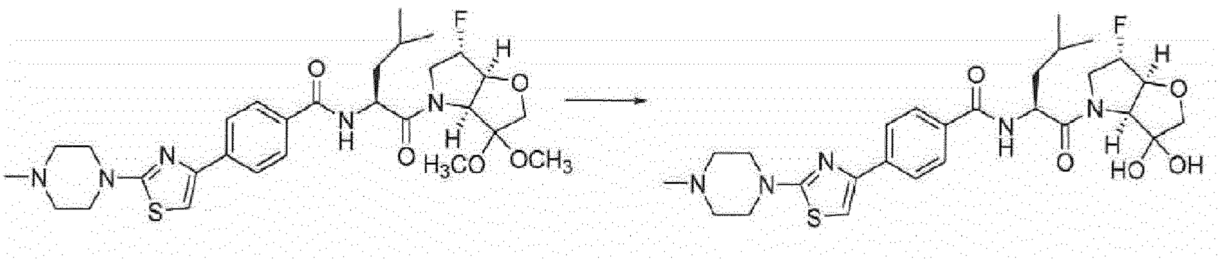

A 63 L glass-lined steel reactor set up for cooling to - 10 °C and heating to 30 °C is filled with nitrogen, and charged with trifluoroacetic acid (37.5 kg), and cooled to 9 -10°C. Keeping the reaction temperature below 15°C, the solution of N-[1S-1-((3aS,6S,6aS)-6-fluoro-3,3-dimethoxy-hexahydro-furo[3,2-b]pyrrole-4-carbonyl)-3-methyl-butyl]-4-[2-(4-methyl-piperazin-1-yl)-thiazol-4-yl]benzamide (2.6 kg, 4.4 mol) in dichloromethane is added. The temperature of the reaction mixture is adjusted to 20 - 22°C, and the reaction is continued until deemed to have reached completion by HPLC (typically 5-6 hours). Heptane (35 kg) are added. The solvent is evaporated by distillation at reduced pressure, using a jacket temperature of 20 - 22°C until a residue of approximately 8 L remains. Heptane (25 kg) are added. The solvent is again evaporated by distillation at reduced pressure, using a jacket temperature of 20 - 22°C until a residue of approximately 3 L remains. The residue is dissolved in dichloromethane (66 kg). The dichloromethane solution is quenched on a suspension of sodium hydrogencarbonate (3.6 kg) and tap water (60 kg). The organic phase is separated. The aqueous phase is extracted with dichloromethane, and the organic phase is secured. The organic phases are washed with 6 % aqueous sodium hydrogencarbonate (2 × 43.5 kg). The organic phases are washed with 25 % sodium chloride (62 kg). The combined organic phase is applied onto a column prepared from silica gel 60 (11 kg) and dichloromethane. The column is then washed with dichloromethane (20 kg). The product is then washed out with 5 % aqueous acetone (w/w) (123 kg). The fractions containing the desired compound are pooled and evaporated, finally using a rotary evaporator, to dryness at reduced pressure and at a jacket temperature of 15 - 25°C. Yield: 2.3 kg (96 %) (based on an evaporation residue). The product is depicted above as the hydrate.

**<u>Example 2:</u> An optimised procedure for the preparation of N-[(S)-1-((3aS,6S,6aS)-6-Fluoro-S-oxo-hexahydro-furo[3,2-b]pyrrole-4-carbony)-3-methyl-butyl]-4-[2-(4-methyl-piperazin-1-yl)-thiazol-4-yl]-benzamide benzenesulphonate (besylate salt of Compound Ib).**

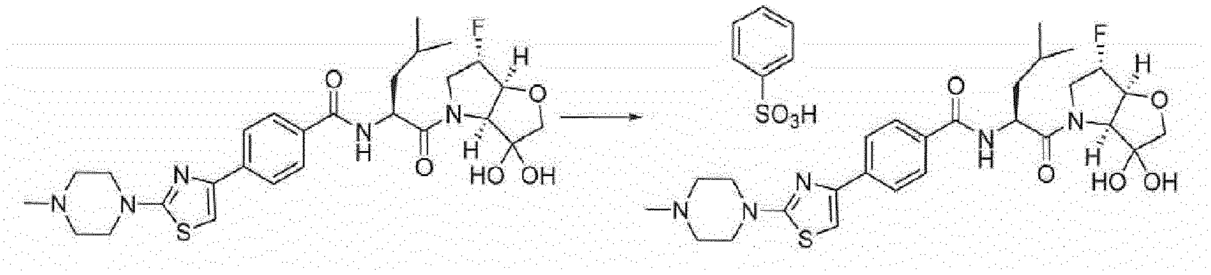

A 63 L glass-lined steel reactor set up for cooling to -10°C and heating to 30°C is filled with nitrogen, and charged with N-[(S)-1-((3aS,6S,6aS)-6-fluoro-3-oxo-hexahydro-furo[3,2-b]pyrrole-4-carbonyl)-3-methyl-butyl]-4-[2-(4-methyl-piperazin-1-yl)-thiazol-4-yl]-benzamide (5.0 kg, 8.9 mol) and acetone (16 kg).

Purified water (0.5 kg) is added. A pH electrode is installed for measuring pH in the range of 1 to 7. The temperature of the suspension is adjusted to 15-20°C. Keeping the reaction temperature below 20°C, benzenesulphonic acid (approximately 1.5 kg, 9.5 mol) is added in order to obtain a final pH of 3.5 to 4.5. The resulting solution is seeded. The crystallizing suspension is cooled to 5 - 8°C. Stirring at this temperature is continued for 20-72 hours. The suspension is filtered, and the filter cake is washed with acetone (6-10 kg). The wet filter cake is dried in a vacuum dryer, finally at 20 - 25°C and pressure ≤ 5 mBar for 3-5 days, or until the level of acetone is below an acceptable level. Yield 5.5 kg (86 %).

**<u>Example 3</u> : Safety and pharmacokinetic of MIV-701 (Compound Ib) and proof of target engagement in healthy beagle dogs after single and repeated administrations of MIV-701-besylate salt at three doses.**

1. <u>Objectives</u>

This study in healthy beagle dogs had four main objectives:

- To assess the safety of repeated oral administration of MIV-701 for 14 days at three doses (1, 10 and 100 mg/kg; once a day).
- To assess plasma pharmacokinetic of MIV-701 after single (D0) and repeated oral administration (D13) at three oral doses of MIV-701 besylate salt.
- To assess MIV-701/MIV0604 plasma exposure ratio after single (D0) and repeated administration (D13) of MIV-701 besylate salt at three doses.

**MIV-701 (= MV061606)**

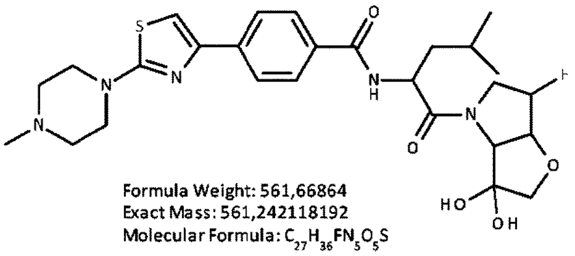

Formula Weight: 561,66864
Exact Mass: 561,242118192
Molecular Formula: $C_{27}H_{36}FN_5O_5S$

**MV06404**

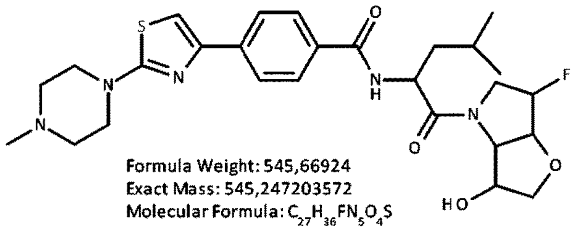

Formula Weight: 545,66924
Exact Mass: 545,247203572
Molecular Formula: $C_{27}H_{36}FN_5O_4S$

- To demonstrate effectiveness of single (D0) and repeated (D13) oral administrations of MIV-701 besylate salt on plasma concentration of CTX-1, a circulating marker of bone resorption.

2. Experimental Protocol

Compound Ib besylate salt (referred to as MIV-701 besylate salt) was administered once daily for 14 days to 3 healthy beagle dogs at three different doses: 1 mg/kg, 10 mg/kg and 100 mg/kg.
The doses were consecutively administered for 14 days with a >5-day washout period between two dose levels.
Each treatment period can be resumed as follows:

- J0 (T+24h): first Compound Ib besylate salt administration, withdrawal of blood samples for pharmacokinetic and plasma CTX-1 assessment (pre-dose and 0.5h, 1h, 2h, 4h, 8h and 24h after drug administration), pre-dose blood samples for hematology and clinical biochemistry (baseline values for safety assessment).
- J1 to J12: pre-dose blood samples
- J13: last Compound Ib besylate salt administration, withdrawal of plasma samples for pharmacokinetic and plasma CTX-1 assessment (pre-dose and 0.5h, 1h, 2h, 4h, 8h and 24h after drug administration), pre-dose sampling for hematology and clinical biochemistry (baseline values for safety assessment).
- J15-J≥19: washout period.

3. Methods

*3.1._Bioanalytical method and pharmacokinetic analyses*

*Plasma Sample preparation*

After centrifugation of blood samples plasma aliquots were stored at -20°C. After thawing on ice, 30 µL of plasma were transferred into 1.5 ml microtube containing 270 µL acetonitrile. Microtubes were then vigorously stirred and then centrifuged (12000 rpm, 10 min, 4°C) and the supernatant was transferred in Matrix tube for LC-MS/MS analysis. Calibration curve (9 points, 3 to 30 000 nM) was obtained by spiking plasma samples from naive dogs with appropriate quantities of Compound Ib.

*3.2. LC-MS/MS*

Equipment: LC-MS/MS Acquity I Class-Wevo TQD (Waters®):
- Acquity I Class
◦ Column: Waters® Acquity BEH C8, 50*2.1mm, 1.7 µm (40°C)
◦ Mobile phase:

  ▪ A: Ammonium Formate 5mM, pH=3.8
  ▪ B: Ammonimuformate 5 mM in $CH_3CN$ 5% aqueous, pH=3.8

◦ Injection volume: 1 µL
◦ Speed: 600 µL/min
◦ Gradient:

| Time (min) | B(%) |
|---|---|
| 0.00 | 2% |
| 0.20 | 2% |
| 2.00 | 98% |
| 2.50 | 98% |
| 2.60 | 2% |
| 4.00 | 2% |

- Xevo TQD
◦ Source temperature: 150°C
◦ Desolvatation temperature: 600°C
◦ Cone gaz speed: 50L/h
◦ Desolvatation gaz speed: 1200L/h
- Analytes MS characteristics

| ID_Structure | MW (g/mol) | Mode | Capillary voltage (kV) | Parent Ion | Cone Voltage (V) | Product Ion | Collision Energy (eV) |
|---|---|---|---|---|---|---|---|
| MIV-701 (compound Ib) | 561.7 | ES + | 0.5 | 562.3 | 54 | 286.2 | 30 |
| MV06404 | 545.7 | ES + | 0.5 | 546.3 | 54 | 286.2 | 30 |

MIV-701 (= MV061606)

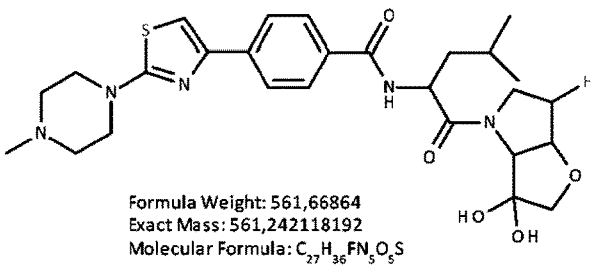

Formula Weight: 561,66864
Exact Mass: 561,242118192
Molecular Formula: $C_{27}H_{36}FN_5O_5S$

MV06404

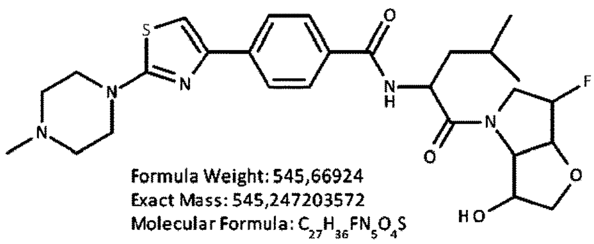

Formula Weight: 545,66924
Exact Mass: 545,247203572
Molecular Formula: $C_{27}H_{36}FN_5O_4S$

*3.3 Pharmacokinetic analyses*

Terminal elimination half-life (t1/2) was calculated according to the following formula:

$$T_{1/2}\ \text{elimination} = \ln(2)/k_{el}$$

$k_{el}$= slope of ln(C)=f(temps) calculated on the last points of the curves (elimination phase)/ MIV-701 exposure (Area Under the Curve; AUCt) was calculated according to trapezoidal method according to the following formula:

$$AUC_{t->inf} = [C]_t/k_{el}$$

4. Results

*Safety*

Overall, oral administrations of MIV-701 besylate salt for 14 days were well tolerated at all doses. No obvious signs of toxicity were observed under the experimental conditions of this in-life phase, regardless of the dose administered.

*Pharmacokinetic results are resumed in Figures 1-2 and Tables 1-2.*

*Table 1: Plasma pharmacokinetic parameters of MIV 701 after single (D0) and repeated (D13) oral administration of MIV-701 besylate salt at 1mg/kg, 10 mg/kg and 100 mg/kg in beagle dogs.*

| | 1 mg/kg | | | | 10 mg/kg | | | | 100 mg/kg | | |
|---|---|---|---|---|---|---|---|---|---|---|---|
| **Day 0** | Kel (2-8h) | -0.236 | $h^{-1}$ | | Kel (8-24h) | -0.167 | $h^{-1}$ | | Kel (8-24h) | -0.174 | $h^{-1}$ |
| | T1/2 elimi-nation | 2.9 | h | | T1/2 elimination | 4.1 | h | | T1/2 elimination | 4.0 | h |
| | Cmax | 20.0 | nM | | Cmax | 139.0 | nM | | Cmax | 1276 | nM |
| | Tmax | 0.5 | h | | Tmax | 1.0 | h | | Tmax | 8.0 | h |
| | AUC(0-tfinal) | 42.9 | h.nM | | AUC(0-tfinal) | 1 351 | h.nM | | AUC(0-tfinal) | 19 418 | h.nM |
| | AUC(0-infini) | 48,8 | h.nM | | AUC(0-infini) | 1384 | h.nM | | AUC(0-infini) | 19 869 | h.nM |
| **Day 13** | Kel (2-8h) | -0.080 | $h^{-1}$ | | Kel (4-24h) | -0.142 | $h^{-1}$ | | Kel (4-24h) | -0.144 | $h^{-1}$ |
| | T1/2 elimi-nation | 8.6 | h | | T1/2 elimination | 4,9 | h | | T1/2 elimination | 4.8 | h |
| | Cmax | 6.6 | nM | | Cmax | 130 | nM | | Cmax | 1 623 | nM |
| | Tmax | 2 | h | | Tmax | 4 | h | | Tmax | 1.0 | h |
| | AUC(0-tfinal) | 36 | h.nM | | AUC(0-tfinal) | 1843 | h.nM | | AUC(0-tfinal) | 20 716 | h.nM |
| | AUC(0-infini) | 83 | h.nM | | AUC(0-infini) | 1905 | h.nM | | AUC(0-infini) | 21 361 | h.nM |

At the three doses tested, Cmax and plasma exposure (AUCs) obtained at D0 and D13 were comparable and did not show plasma accumulation of MIV-701 after 14-day repeated oral administration of MIV-701 besylate salt.

At both D0 and D13, MIV-701 Cmax and AUC increase with the administered dose in a linear manner.

Table 2 compares peak area obtained in dog for MIV-701 and its metabolite MIV-06404 in plasma after single oral administration of MIV-701 besylate salt. This analysis showed that MIV-701 daily exposures were 3 to 7 folds higher than MIV-06404 daily exposure. This ratio contrasts with the results obtained in phase 1 trial in healthy human volunteers where plasma exposures of MIV-701 were 25 to 100 folds lower than those of MIV-06404. Because of this extensive drug metabolisation after oral administration in human, clinical development of MIV-701 for treating human bone diseases was discontinued. As compared to human, MIV-701 undergoes minor metabolization in dogs qualifying MIV-701 as a suitable drug candidate for treating canine bone diseases.

*Table 2: MIV-701/MIV-06404 exposure ratio after single oral administration of MIV-701 besylate salt at various doses in dog and human.*

| Species | Doq | | | Human* | | | |
|---|---|---|---|---|---|---|---|
| Dose | 1 mg/kg | 10 mg/kg | 100 mg/kg | 25 mg | 75 mg | 150 mg | 300 mg |
| MIV-701/MIV06404 ratio | 7.0 | 3.6 | 3.3 | 0.01 | 0.02 | 0.03 | 0.04 |
| **Results from the Phase 1 clinical trial in human healthy volunteers.* | | | | | | | |

*Effects on plasma CTX-1 plasma concentrations are resumed in Figure 3 and Figure 4*

As shown in Figure 3, single oral administration of MIV-701 besylate salt at 1 mg, 10 mg and 100 mg provoked dose-dependent effect on plasma CTX-1, a well-known marker of bone resorption. At D0 (Figure 3) and at the three doses tested, CTX-1 plasma concentration declined immediately after oral administration, the inhibition peak being obtained 8 hours after administration. For 10mg/kg and 100 mg/kg doses, CTX-1 plasma concentration 24 hours after administration did not go back to pre-dose concentration 24 hours after administration.

After once a day repeated oral administrations of MIV-701 besylate salt to healthy beagle dogs, a dose-dependent inhibition of pre-dose plasma CTX-1 plasma concentration was obtained at D1 (i.e. 24 hours after first dose at D0) and is sustained throughout the 14 days treatment period (Figure 5). At the dose of 10 mg/kg a 40 % inhibition vs

baseline was maintained from D1 throughout up to the end of the 14 days treatment period.

**Example 4: Safety, pharmacokinetic of MIV-701 and proof of target engagement in healthy cats after single and repeated administrations of MIV-701-besylate salt at three doses**

1. Objectives

This study in healthy cats had three main objectives:

- To assess the safety of repeated oral administration of MIV-701 for 14 days at three doses (2, 10 and 50 mg/kg; once a day) in healthy cats.
- To assess plasma pharmacokinetic parameters of MIV-701 after single (D0) and repeated oral administration (D13) at three oral doses of MIV-701 besylate salt.
- To assess MIV-701/MIV-0604 plasma exposure ratio after single (D0) and repeated administration (D13) of MIV-701 besylate salt at three doses.
- To demonstrate effectiveness of single (D0) and repeated (D13) oral administrations of MIV-701 besylate salt on plasma concentration of CTX-1, a circulating marker of bone resorption.

2. Experimental protocol

MIV-701 besylate salt was administered once daily for 14 days to 4 healthy cats at three increasing doses: 2 mg/kg, 10 mg/kg and 50 mg/kg.
The three doses were consecutively administered for 14 days with a >5-day washout period between two dose levels.
Each treatment period can be resumed as follows:

- J0 (T+24h): first administration, withdrawal of blood samples for pharmacokinetic and plasma CTX-1 assessment (pre-dose and 0.5h, 1h, 2h, 4h, 8h and 24h after first drug administration), pre-dose blood samples for hematology and clinical biochemistry (baseline values for safety assessment).
- J5 (24 hours after first administration) and J8: pre-dose blood samples.
- J13: last administration, withdrawal of plasma samples for pharmacokinetic and plasma CTX-1 assessment (pre-dose and 0.5h, 1h, 2h, 4h, 8h and 24h after last drug administration), pre-dose sampling for hematology and clinical biochemistry (baseline values for safety assessment).
- J15 to J≥19: washout period.

3. Analytical method

*Plasma Sample preparation*

After centrifugation of blood samples plasma aliquots were stored at -20°C._After thawing on ice, 30 μL of plasma were transferred into 1.5 ml microtube containing 270 μL acetonitrile. Microtubes were then vigorously stirred and then centrifuged (12000 rpm, 10 min, 4°C) and the supernatant was transferred in Matrix tube for LC-MS/MS analysis.
A calibration curve (9 points, 3 to 30 000 nM) was obtained by spiking plasma samples from naive cats with appropriate quantities of Compound I.

*LC-MS/MS :*

Equipment: LC-MS/MS Acquity I Class-Wevo TQD (Waters®):
- Acquity I Class
◦ Column: Waters® Acquity BEH C8, 50*2.1mm, 1.7 μm (40°C)
◦ Mobile phase:

  - A: Ammonium Formate 5mM, pH=3.8
  - B: Ammonimuformate 5 mM in $CH_3CN$ 5% aqueous, pH=3.8

◦ Injection volume: 1 μL
◦ Speed: 600 μL/min
◦ Gradient:

| Time (min) | B (%) |
|---|---|
| 0.00 | 2% |
| 0.20 | 2% |
| 2.00 | 98% |
| 2.50 | 98% |
| 2.60 | 2% |
| 4.00 | 2% |

- Xevo TQD

  ◦ Source temperature: 150°C
  ◦ Desolvatation temperature: 600°C
  ◦ Cone gaz speed: 50L/h
  ◦ Desolvatation gaz speed: 1200L/h

- Analytes MS characteristics

| ID_Structure | MW (g/mol) | Mode | Capillary voltage (kV) | Parent Ion | Cone Voltage (V) | Product Ion | Collision Energy (eV) |
|---|---|---|---|---|---|---|---|
| MIV-701 | 561.7 | ES + | 0.5 | 562.3 | 54 | 286.2 | 30 |
| MV06404 | 545.7 | ES + | 0.5 | 546.3 | 54 | 286.2 | 31 |

*Pharmacokinetic analyses*

Terminal elimination half-life (t1/2) was calculated according to the following formula:

$$T_{1/2}\,\text{elimination} = \ln(2)/k_{el}$$

$k_{el}$= slope of ln(C)=f(temps) calculated on the last points of the curves (elimination phase)/ MIV-701 exposure (Area Under the Curve; AUCt) was calculated according to trapezoidal method accroding to the following formula:

$$AUC_{t\text{->}inf} = [C]_t/k_{el}$$

4. Results

*Safety*

Under the conditions of this study, a repeated oral administration of MIV-701 besylate salt at 2 mg/kg/day and 10 mg/kg/day, during 14 days did not induce any adverse or irreversible effect. A repeated oral administration of MIV-701 besylate salt at 50 mg/kg/day during 4 days induced some digestive and behavioral degradations from D0 to D4 that leaded to treatment withdrawal. These signs were perfectly reversible after treatment cessation.

*Pharmacokinetic results are resumed in Figures 6-7 and Table 3.*

Figure 6 and Table 3 show that, at both D0 and D13, MIV-701 Cmax and AUC increase with the administered dose in a linear manner. At 2 mg/kg and 10 mg/kg, plasma exposure (AUC) obtained at D13 were respectively 1.2 and 1.9 folds higher than AUC at D0 suggesting moderate plasma accumulation of MIV-701 after repeated administration of MIV-701 besylate salt.

*Table 3: Plasma pharmacokinetic parameters of MIV 701 after single (D0) and repeated (D13) oral administration of MIV-701 besylate salt at 2mg/kg, 10 mg/kg and 50 mg/kg in cats.*

| | **2 mg/kg** | | | | **10 mg/kg** | | | | **50 mg/kg** | | |
|---|---|---|---|---|---|---|---|---|---|---|---|
| | | | | | | | | | | | |
| **Day 0** | Kel (4-24h) | -0.236 | $h^{-1}$ | | Kel (4-24h) | -0.152 | $h^{-1}$ | | Kel (4-24h) | -0.142 | $h^{-1}$ |
| | T1/2 elimination | 2.9 | h | | T1/2 elimination | 4.6 | h | | T1/2 elimination | 4.9 | h |
| | Cmax | 122 | nM | | Cmax | 249.0 | nM | | Cmax | 3 887 | nM |
| | Tmax | 2.0 | h | | Tmax | 2.0 | h | | Tmax | 2.0 | h |
| | AUC(0-tfinal) | 808 | h.nM | | AUC(0-tfinal) | 2 776 | h.nM | | AUC(0-tfinal) | 33 608 | h.nM |
| | AUC(0-infini) | 811 | h.nM | | AUC(0-infini) | 2 851 | h.nM | | AUC(0-infini) | 34 812 | h.nM |
| **Day 13** | Kel (4-24h) | -0.179 | $h^{-1}$ | | Kel (4-24h) | -0.122 | $h^{-1}$ | | | | |
| | T1/2 elimination | 3.9 | h | | T1/2 elimination | 5.7 | h | | | | |
| | Cmax | 141 | nM | | Cmax | 663 | nM | | | | |
| | Tmax | 2.0 | h | | Tmax | 0.5 | h | | | | |
| | AUC(0-tfinal) | 972 | h.nM | | AUC(0-tfinal) | 5 067 | h.nM | | | | |
| | AUC(0-infini) | 984 | h.nM | | AUC(0-infini) | 5 303 | h.nM | | | | |

Figure 7 and Table 4 show that, at 2mg/kg, 10 mg/kg and 50 mg/kg in cat, the MIV-701/MIV-06404 exposure ratio at D0 was measured at 6.9, 6.2 and 3.9 respectively. At 2mg/kg, 10 mg/kg the MIV-701/MIV-06404 exposure ratio at D13 was measured at 5.0 and 3.4 respectively. For comparison, in human this ratio after single administrations to healthy volunteers have been measured at 0.03 precluding further development of MIV-701 besylate salt for treating human diseases.

*Table 4: MIV-701/MIV-06404 exposure ratio after single oral administration of MIV-701 besylate salt at various doses in cat and human.*

| Species | Cat | | | Human* | | | |
|---|---|---|---|---|---|---|---|
| Dose | 2 mg/kg | 10 mq/kq | 50 mq/kq | 25 mg | 75 mg | 150 mg | 300 mg |
| MIV-701/MIV-06404 ratio | 6.9 | 6.2 | 3.9 | 0.01 | 0.02 | 0.03 | 0.04 |
| **Results from a Phase 1 clinical trial in human healthy volunteers.* | | | | | | | |

*Effects on plasma CTX-1 plasma concentrations are resumed in figure 8-9.*

As shown in Figure 8, single oral administration of MIV-701 besylate salt at 2 mg/kg, 10 mg/kg and 50 mg/kg provoked comparable reduction of plasma CTX-1, a well-known marker of bone resorption. At D0 (Figure 8) and at the three doses tested, CTX-1 plasma concentration declined immediately after oral administration, the inhibition peak being obtained 4 hours after administration. At all doses tested, CTX-1 plasma concentration 24 hours after administration did not go back to pre-dose concentration 24 hours after administration.

As shown in Figure 9, after once-a-day repeated oral administrations of MIV-701 besylate salt at 2 and 10 mg/kg to healthy cats, a rapid drop of pre-dose plasma CTX-1 plasma concentration was obtained at D1 (i.e. 24 hours after first dose at D0) and is sustained throughout the 14 days treatment period (Figure 9). At the dose of 10 mg/kg a 60% inhibition vs baseline was obtained at the end of the 14 days treatment period.

*Conclusion in healthy cats*

Up to the dose of 10 mg/kg/day, oral administration of MIV-701 for 14 days is well tolerated in cat.
In cat, Cmax and daily exposure of MIV-701 increase with the administered dose of MIV-701 besylate salt.
In contrast to human, MIV-701 is slightly metabolized in cat qualifying MIV-701 as a drug candidate for the treatment of feline diseases.
At safe doses of 2 and 10 mg/kg, oral administration of MIV-701-besylate for 14 days provokes a sustained inhibition of plasma CTX-1, a marker of bone resorption process.

**Example 5: Efficacy and tolerance assessment of oral treatment with MIV-701 besylate salt, in dogs with alveolar bone loss associated with spontaneously developed periodontal disease.**

The present study was performed on 10 Beagle dogs suffering from alveolar bone loss induced by spontaneously developed periodontal disease. All dogs were treated with MIV-701 besylate salt for minimum 30 day and maximum 90 days, depending on the tolerance at intermediate visits. The product is administrated to the dog by its owner at home, once a day with a meal.
There were two treatment groups:

- Group A (5 dogs) with a dose of 25 mg/kg.
- Group B (5 dogs) with a dose of 50 mg/kg.

Dogs were allocated into one of two groups at visit V2 (Day 0) based on the inclusion order.
The choice of doses was based on good tolerance of a daily dose of 100 mg/kg for 14 consecutive days in previous tolerance study in healthy Beagles (results not shown).

1. Study objective

This is a non-comparative, randomized and open-label study aiming at efficacy and tolerance assessment of MIV-701 besylate salt in client-owned dogs with alveolar bone loss induced by spontaneously developed periodontal disease.

*1.1. Primary Objective*

The primary objective of the study was to assess the effects of MIV-701 besylate treatment on plasmatic level of bone resorption marker - C-terminal telopeptides of type I collagen (CTX-1) in client-owned dogs.
Bone is a dynamic tissue that undergoes constant remodeling throughout its life span. Physiologically, bone turnover is balanced between bone formation and resorption at various rates leading to continuous remodeling of the bone. The resorptive step can be divided into two basic processes, mineral solubilization and focalized matrix degradation. Whereas mineral solubilization depends on the production and secretion of acid by the osteoclast, matrix degradation is mainly due to the activity of cathepsin K.
In human, approximately 90% of the organic matrix of bone is type 1 collagen. During bone resorption, osteoclasts secrete a mixture of acid and neutral proteases, with cathepsin K among others, that degrade the collagen fibrils into molecular fragments including C-terminal telopeptide type 1 (CTX-1). This telopeptide is then excreted into the body fluids, i.e., blood and urine, and is useful for showing acute changes in bone resorption. Its altered level has been associated with osteoporosis and postmenopausal osteopenia in women as well as with generalized periodontitis in the period of its exacerbation (Mazur 2006).
In human, studies have shown that CTX-1 is a specific and sensitive biomarker of bone resorption that can rapidly indicate the response to therapy (Baim S and Miller PD 2009). Up to 81% reduction in CTX-1 has been observed in double-blinded, randomized placebo-controlled phase I clinical trial on healthy post-menopausal woman treated daily with cathepsin K inhibitor (Stoch et al. 2009). Nevertheless, circadian variation and influence of food intake must be considered while collecting the blood sample for analysis. In human, it has been demonstrated that the bone resorption is at its maximum early in the morning (05:00 - 08:00 AM) to further decline to minimum in late afternoon. The magnitude between the lowest and the highest level can be further affected by the food intake and reach even 100% difference. Data regarding canine CTX-1 circadian rhythm are limited, however present similar trend (Nikahval et al. 2016; Vrbanac et al. 2020). Therefore, it is recommended to analyse the CTX-1 concentration in morning sample collected after overnight fasting (Chubb 2012).

*1.2. Secondary Objectives*

The secondary objectives were:

- to evaluate the extend of alveolar bone loss in client-owned dogs before and after treatment with MIV-701-besylate salt.
- To evaluate tolerance of MIV-701-besylate salt in client-owned dogs.

*1.3. Exploratory Objectives*

The secondary objectives were:

- To measure Clinical Attachment Loss (CAL) in client-owned dogs before and after treatment with MIV-701-besylate salt.
- To determine Periodontal Probing Depth in client-owned dogs before and after treatment with MIV-701-besylate salt.
- To assess bleeding on probing loss in client-owned dogs before and after treatment with MIV-701-besylate salt.

2. Investigational treatment

**Name:** MIV-701 besylate salt

**Description:** Cathepsin K inhibitor

**Active ingredient:** MIV-701

**Route of administration:** orally

**Dosage:** 25 mg/kg for group A and 50 mg/kg for group B

**Administration protocol and dosage:** MIV-701 besylate salt was administrated to dog once-a-day, at home with a standard meal. It was allowed to administrate the capsule(s) with a small amount of wet food/favorite snack. Opening of the capsule and spilling the product over the food was forbidden.

Treatment was administrated for 90 days. Every 30 days, based on the results of clinical exam, blood parameters and potential occurrence of adverse events suspected to be drug-related, the treatment prolongation for another 30 days, or discontinuation of treatment was decided by the Investigator.

At visit V4 (Day 60), dogs of group A that had well tolerated MIV-701 besylate salt entered group B for last 30 days of treatment.

3. Patients population

**Study population:** Dogs with alveolar bone loss induced by spontaneously developed periodontal disease.

Number of patients: 10 dogs (5 for Group A and 5 for Group B).

All animals are companion dogs, selected by the Investigator based on the results of clinical exam, blood analyses, periodontal parameters assessment and dental imaging.

Inclusion criteria:

- The Owner approved and signed the informed consent form prior to the participation of his/her dog in the study.
- Adult dog with finished, permanent teeth eruption.
- Mesocephalic or dolichocephalic dog.
- Dog weighting between 3 and 20 kilograms.
- Dog presenting minimum 3 mandibular teeth with periodontal disease stage 2 or 3, and alveolar bone loss.
- Dog routinely maintained on dry diet.
- Dog in which active dental homecare cannot be applied.

Non-inclusion criteria:

- Females known to be pregnant or lactating.
- Brachycephalic dog.

- Dog with gingival hyperplasia, neoplasia, autoimmune disease and immunosuppressive disease or concurrent systemic disease.
- Dog with a disease requiring chronic treatment with NSAiDs or steroids.
- Dog treated with bisphosphonates.
- Dog vaccinated within last 30 days unless 4 weeks washout period is applied prior to visit V2 (Day 0).
- Dog requiring vaccination against rabies during next 97 days.
- Dog that received non-steroidal anti-inflammatory drug within last 14 days unless 2 weeks washout period is applied prior to visit V2 (Day 0).
- Dog that received short-acting steroids within last 14 days unless 2 weeks washout period is applied prior to visit V2 (Day 0).
- Dog that received intermediate - acting steroids within last 30 days unless 4 weeks washout period is applied prior to visit V2 (Day 0).

Dog that received long-acting steroids within last 12 weeks.

Premature withdrawal criteria:

- Any adverse event interfering with study objectives or leading to a long-term treatment interruption.
- More than 4 consecutive doses not administered.
- More than two episodes of maximum 4 consecutive doses not administrated within 60 consecutive days.
- Owner’s consent withdrawal.
- Dog lost to follow-up.

Any other event incompatible with study participation at the Investigator’s judgement.

Concomitant treatments (authorized / forbidden):

- Compulsory treatment:

    ◦ Dental scaling with polishing including subgingival debridement and closed root planning.
    ◦ General anesthesia with tracheal intubation of Investigator’s choice for dental scaling, polishing, subgingival debridement, closed root planning, dental radiographs, cone-beam computed tomography (CBCT) or CT-scan, and periodontal parameters assessment.

- Authorized concomitant treatments:

    ◦ Local treatment (including surgery) of periodontal disease, applied to other than selected teeth for the study.
    ◦ NSAIDS used between visit V2 (Day 0) and visit V5 (Day 90t 3 days) for maximum 3 consecutive days.
    ◦ Every analgesic agent (NSAIDs, morphine and morphine-like analgesics) used for 5 consecutive days if administrated due to tooth extraction/surgery at visit V2 (Day 0).
    ◦ Antibiotic therapy.
    ◦ Prophylactic treatments used in routine such as flea’s prevention products and deworming products.

- Forbidden treatments:

    ◦ Any active or passive home care including tooth brushing, dental supporting diet, supplements and chews, oral rinses, water additives and gels for oral care.
    ◦ Any regenerative procedure and/or flap procedure, applied to the selected teeth for the study.
    ◦ Any NSAiDs applied before visit V2 (Day 0)
    ◦ NSAiDs applied longer than 3 consecutive days between visit V2 (Day 0) and visit V5 (Day 90t 3 days).
    ◦ NSAIDs applied longer than 5 consecutive days after tooth extraction/surgery at visit V2 (Day 0).
    ◦ Bisphosphonates.
    ◦ Glucocorticosteroids at any dose.

4. <u>Study schedule (Figure 10)</u>

**Visit V1 (between Day -7 and Day 0)** - **Screening**

- Information and Owner's consent form.
- Record of past and current medical history.
- Clinical examination.
- Blood collection for hematology, biochemistry and ionized calcium analyses, unless historical results from no more than 30 days before inclusion are available.

**Visit V2 (Day 0) - Inclusion (between 08:00-10:00, on fasting dog)**

- Blood collection for plasmatic CTX-1 level evaluation.
- Full mouth dental CBCT-scan
- Dental scaling and polishing including subgingival debridement and closed root planning.
- Full mouth dental exam with periodontal parameters assessment.
- Selection of 3 teeth for the study.
- Dental radiographs of 3 teeth selected for the study.
- Inclusion/Non-inclusion criteria checkup.
- Randomization into one of two groups.
- Dispensation of therapeutic units for 30 days (+5 supplementary doses).
- OWNER BOOK dispensation.

**Visit V3 (Day 30 + 3 days) - Control 1 (between 08:00-10:00, on fasting dog)**

- Clinical exam.
- Blood collection for plasmatic CTX-1 level evaluation.
- Blood collection for hematology, biochemistry and ionized calcium analyses.
- Adverse Event (AE)/Serious Adverse Event (SAE) and concomitant treatment check.
- OWNER BOOK verification.
- If applicable, therapeutic units dispensation for 30 days (+5 supplementary doses). If the dog continues the treatment, the Investigator scheduled and performed visit V4 at Day 60±3 days and visit V5 at Day 90±3 days.

If the dog did not continue the treatment, the next scheduled and performed visit was visit V5 completed at Day 60 ±3 days instead of Day 90.

**Visit V4 (Day 60t 3 days) - Control 2 (between 08:00-10:00, on fasting dog)**

- Clinical exam.
- Blood collection for plasmatic CTX-1 level evaluation.
- Blood collection for hematology, biochemistry and ionized calcium analyses.
- AE/SAE and concomitant treatment check.
- OWNER BOOK verification and collection.
- If applicable, transition of dogs from group A to group B for next 30 days of MIV-701 treatment.
- If applicable, MIV-701 dispensation for 30 days (+5 supplementary doses).

**Visit V5 (Day 90t 3 days) - End of study (between 08:00-10:00, on fasting dog)**

- Clinical exam.
- Blood collection for plasmatic CTX-1 level evaluation.
- Blood collection for hematology, biochemistry and ionized calcium analyses.
- Full mouth dental CBCT or CT-scan.
- Periodontal parameters assessment of 3 teeth selected for the study.
- Dental radiographs of 3 teeth selected for the study.

- AE/SAE and concomitant treatment check.
- OWNER BOOK verification and collection.
- Recovery of unused MIV-701 from the Owner.

5. Observations and procedures

*5.1. Blood sampling*

Blood sampling was performed at each visit. Each collected sample was recorded in BLOOD SAMPLE COLLECTION FORM.

During visit V2 (Day 0) and visit V5 (Day 90t 3 days) the blood sample was collected before dog's anesthesia.

Whole blood sample was collected from the jugular or cephalic vein and if needed, further processed to obtain canine serum or plasma. As far as possible, the blood samples planned for the same day was collected with the single puncture.

*5.2. Hematology analysis*

Whole blood sample for hematology analysis was collected at visit V1 (between Day -7 and Day 0), visit V3 (Day 30+3 days), Visit V4 (Day 60t 3 days) and Visit V5 (Day 90t 3 days).

The result from visit V1 (between Day -7 and Day 0) was used as a baseline value for the safety evaluation criteria.

Each time 1 ml of the peripheral whole blood was collected on a tube with anti-coagulant (EDTA) and gently inverted several times. A complete blood count: WBC with details of lymphocytes, monocytes and polynuclear cells (neutrophils, eosinophils and basophils); Red blood cells (RBC); Hematocrit (HCT); Hemoglobin (HGB); Reticulocytes (RETIC); Mean corpuscular volume (MCV); Mean Corpuscular Hemoglobin (MCH); Mean Corpuscular Hemoglobin Concentration (MCHC); platelets count (PLT) was performed.

Each investigation site was responsible for their blood analysis and provided its results in form of laboratory report scan to the study Monitor. Results were then recorded by the Monitor in Central Data Base.

*5.3. Biochemistry analysis*

Whole blood sample for biochemistry analysis was collected at visit V1 (between Day -7 and Day 0), visit V3 (Day 30+3 days), Visit V4 (Day 60t 3 days) and Visit V5 (Day 90t 3 days).

The result from visit V1 (between Day -7 and Day 0) was used as a baseline value for the safety evaluation criteria.

Each time 1.3 ml of the peripheral whole blood was collected on lithium heparin tube and gently inverted several times. Next, sample was centrifuged at 2500 G for 5 minutes at room temperature and heparin plasma will be collected. The following biochemical parameters: Albumin, Alanine transaminase (ALT), Alkaline phosphatase (ALP), creatinine, globulins, glucose, Total proteins, Urea, Albumin/globulin ratio and urea/creatinine ratio were analyzed.

Each investigation site was responsible for their blood analysis and provided its results in form of laboratory report scan to the study Monitor. Results were then recorded by the Monitor in Central Data Base.

*5. 4. CTX-1 analysis*

Whole blood sample for CTX-1 analysis was collected at visit V2 (Day 0), visit V3 (Day 30+3 days), Visit V4 (Day 60t 3 days) and Visit V5 (Day 90t 3 days).

Each time, the blood sample was collected after overnight fasting, between 08:00 and 10:00 in the morning. As far as possible the blood sample was taken at the same time each visit. The night before the visit, the Investigational Product was administrated to the dog at 21:00.

The result from visit V2 (Day 0) was used as a baseline value for the efficacy evaluation criteria.

Each time 2 ml of the peripheral whole blood was collected on an anti-coagulant tube (K2EDTA) and gently inverted several times. Next, sample was centrifuged at 1500 G for 10 minutes at room temperature. The obtained plasma was divided into two Eppendorf tubes labelled as "Aliquot 1" and "Aliquot 2". Minimum 0.5 ml was placed in "Aliquot 1" while the rest of plasma was placed in "Aliquot 2". All obtained aliquots were immediately stored at - 20°C on site.

Time of blood collection, number of prepared aliquots and storage start time were recorded in BLOOD SAMPLE COLLECTION FORM.

The CTX-1 analysis was performed at the end of the study by Central Laboratory. The shipment of aliquots from site to Central Laboratory was organized by the Monitor and divided into two, separated in time shipments. One with all "Aliquot 1" samples and second one with all "Aliquot 2" samples. The CTX-1 SAMPLE SHIPMENT FORM was joined to the shipment.

All results issued by the Central laboratory were transferred to the Monitor at the end of the study for data analysis.

*5.5. Full mouth exam at V2*

At visit V2 (Day 0) the Investigator performed oral examination on conscious and then anesthetized dog. The results of this exam were recorded in a dental chart of Investigator's choice and stored in paper Case Report Form of each patient.

*5.5.1. Examination of conscious patient at V2*

The exam started with inspection of the head evaluating the eyes, symmetry of the head, swelling, lymph nodes, nose and lips. Next, the occlusion and the functionality of the temporomandibular joint were checked. The investigator recorded any missing, fractured or discolored teeth. The examination of soft tissue of the oral cavity included oral mucosa, gingiva, palate, dorsal and ventral aspect of the tongue, tonsils, salivary glands and ducts, and focused on potential masses, swelling, ulcerations, bleeding and inflammation.
During conscious periodontal examination, the Investigator especially focused on gingival inflammation, calculus deposits and gingival recession.
Any occlusion abnormalities defined as either a skeletal malocclusion or malposition of single was recorded.

*5.5.2. Examination under general anesthesia at V2*

It was suggested that the examination performs in the order listed below. However, the Investigator was allowed to adapt it to his practice.

1. The dog was anesthetized with the Investigator's protocol of choice.
2. The Investigator examined oropharynx and tonsils in the fauces before endotracheal intubation and throat pack placing.
3. If possible, the photographs of each side of the patient and one from the rostral aspect were taken.
4. In order decrease the bacterial load 0.12% chlorhexidine oral rinse was applied.
5. The dog's dentition (primary, permanent or mixed) was determined.
6. The soft tissue of entire oral cavity and vestibular tissues including oral mucous membranes, lips and cheeks, palate, tongue and sublingual tissue for abnormalities and oral masses were evaluated.
7. The Investigator performed initial dental scaling and polishing including subgingival debridement and closed root planning.
8. If dental scaling revealed any pathology, additional intraoperative photographs were taken.
9. The Investigator performed dental examination with dental explorer of each tooth, beginning with the first incisor of each quadrant and progressing caudally tooth by tooth to cover the entire arch. The entire surface of each tooth was explored, especially the area just below the gingival margin to detect resorptive lesions. The Investigator recorded presence or absence of the teeth as well as any irregularity of tooth surface or change of color.
10. The Investigator performed periodontal examination.
11. Dental radiographs was done on minimum 3 selected teeth for the study as described in chapter 8.2.7
12. Based on clinical finding and dental CBCT-scan the Investigator performed staging of periodontal disease for each tooth.
13. Definitive dental scaling and polishing was performed.
14. If possible, postoperative photographs of each side of patient was taken.

*5. 6. Dental scaling and polishing at V2*

Dental scaling and polishing were performed at visit V2 (Day 0) after oral exam on the conscious patient, full mouth CBCT scan and first 6 steps of the oral exam under anesthesia. Each Investigator followed the 2019 AAHA Dental Care Guideline for Dogs and Cats.
It was suggested that the procedure is performed as described below, however it was allowed to adapt to each investigator practice.
First, the Investigator scaled the teeth supra- and subgingivally using a hand scaler (supragingivally), curette (subgingivally), or an appropriately powered ultrasonic scaled followed by a curette inserted subgingivally to remove additional plaque and calculus. Teeth cleaning included hand scaling of the accessible root surfaces. Next, the teeth crown was polished using a disposal low-speed hand piece prophy angle, individually packaged prophy paste or fine-grit pumice and polishing cup running at no more than 3000 rpm.

After periodontal parameters assessment (see below), the procedure continued with subgingival irrigation to remove debris and polishing paste as well as inspection of the crown and subgingival areas. The Investigator used air or water syringe to inspect the visible subgingival areas for remaining calculus requiring removal.

Any local perioperative antibiotics, antiplaque substances such as barrier sealants or other local treatment were applied other teeth than those selected for the study. The treatment plan was recorded in dental chart of Investigator's choice and stored at each patient paper Case Report Form.

*5. 7. Periodontal parameter assessment at V2 and V5*

The examination was performed at visit V2 (Day 0) and visit V5 (90t 3 days).

At visit V2 (Day 0) and V5 (90 ± 3 days). the Investigator evaluated the following parameters for each tooth:

- Periodontal probing depth (PPD) - measured as the distance from the gingival margin to the bottom of the pocket using a graduated periodontal probe of Investigator's choice. The sulcus/pocket was measured circumferentially around the whole tooth and valued of 3 to 6 places, depending on the tooth localization (Figure 11). Measurements was done in millimeters (mm) and rounded to the nearest lower value. The mean PPD value of these measurements per tooth was calculated.
- Clinical attachment loss (CAL) - measured as the distance from the cemento-enamel-junction to the bottom of the pocket using a periodontal probe of the Investigator choice. CAL was measured in 3 to 6 sites per tooth depending on the tooth localization (Figure 11). Measurements was done in millimeters (mm) and rounded to the nearest lower value (Kortegaard et al. 2014). The mean CAL value of these measurements per tooth will be calculated.
- Bleeding Probing Index - assessed at each site of PPD and CAL measurement. Depending on the absence or presence of bleeding within 10 second following probing of the tooth, BPI scored either as 0 or 1 respectively. The mean BIP value was calculated per tooth.

The results of this examination were recorded in a dental chart of Investigator's choice and stored in each patient paper Case Report Form.

At visit V2 (Day 0) the Investigator selected minimum 3 teeth for the study based on the periodontal parameters assessment and alveolar bone loss measurements in CBCT-scan performed that day. Each of the selected teeth had to comply all the following requirements:

- Mandibular tooth.
- Periodontal disease of stage 2 or 3.
- Alveolar bone loss visible at CBCT and dental radiographs.
- Tooth which does not require additional local treatment.

Once 3 teeth were selected for the study, the Investigator recorded it in PERIODONTAL PARAMETERS FORM together with their results of their PPD, CAL and BoP.

At visit V5 (90t 3 days), the Investigator assessed PPD, CAL and BoP in only 3 selected teeth for the study and recorded it in PERIODONTAL PARAMETERS FORM.

*5.8. Dental imaging*

Dental imaging was performed at visit V2 (Day 0) and visit V5 (90t 3 days).

Each time a full mouth CBCT-scan was performed together with dental radiographs of selected teeth for the study. The results of these exams were recorded in IMAGING FORM by the Investigator.

*5.8.1. Cone-Beam Computed Tomography (CBCT)*

Full mouth cone-beam computed tomography (NewTom, 5G XL, Bologna, Italy) was performed at visit V2 (Day 0) and visit V5 (Day 90t 3 days).

The exam was performed only in dogs recruited in Polish site. It was performed under general anesthesia of Investigator's choice and aim at alveolar bone loss measurement.

Scans were analyzed with the use of NNT viewer, software provided by the manufacturer (version: 10.1; QR SRL, Verona, Italy). At both times, the volumetric assessment of dentition was set up for the same high-resolution mode (10 × 10 cm with 0.15 mm layers). Both readings were made in the same orientations of axes and locations measuring alveolar bone loss with the use of maximum magnification (Gawro, Strøm et Nemec 2022). To evaluate the effect of the treatment on the alveolar bone loss, the following three measurements (De Faria Vasconcelos et al. 2012) were performed on the selected

teeth for the study:

1. The height of the alveolar crest (AC), measured from the cementoenamel junction (CEJ) to the upper rim of AC (Figure 12A).
2. The depth of the defect, measured from the CEJ to the bottom of the defect (Figure 12B).
3. The width of the defect, measured from the highest point of the AC to the dental root adjacent to the defect (Figure 12C).

The results of these measurements were recorded in the IMAGING FORM.

*5.9. X-Ray radiography*

Dental radiography was performed at visit V2 (Day 0) and visit V5 (Day 90t 3 days).

To evaluate the effect of the treatment on the alveolar bone loss, the following three measurements (De Faria Vasconcelos et al. 2012) were performed on the selected teeth for the study:

1. The heigh of the alveolar crest (AC), measured from the cementoenamel junction (CEJ) to the upper rim of AC (Figure 13A).
2. The depth of the defect, measured from the CEJ to the bottom of the defect (Figure 13B).
3. The width of the defect, measured from the highest point of the AC to the dental root adjacent to the defect (Figure 13C).

These measurements were performed using digital ruler of the imaging software or external program (i.e., Image J) and recorded in the IMAGING FORM.

6. <u>Results</u>

6.1. - Primary Objective: Effects on plasma CTX-1 (bone resorption marker)

As compared to baseline value at Day-0, repeated oral administrations of MIV-701- besylate salt at doses of 25 and 50 mg/kg/day provoked comparable reduction of plasma CTX-1 at Day-30 and Day-60 without showing significant difference between the two groups (Figure 14).

Because of lack of safety concern in both groups of treatment after 60 days of treatment with MIV-701-besylate salt, all dogs were treated with 50 mg/kg/day for the remaining 30 days of the treatment period (dogs in the 25mg/kg/day group were shifted to 50 mg/kg/days whereas dogs in the 50 mg/kg/day continued treatment up to Day-90).

As shown in figure 15, the primary objectives of the study were clearly reached with a highly significant reduction of plasma CTX-1 at Day-90 at 50 mg/kg/day as compared to pre-treatment baseline value at Day-0. Most importantly, all but one patient experienced reduction in plasma CTX-1. The mean % change in CTX-1-reponders (n=9) was -67%±6%.

6.2. Secondary Objectives: Effect on alveolar bone loss parameters measured by CBCT-scan and X-ray radiography

As shown in Figure 16 in CTX-1 responders, width of the alveolar bone defect measured at the end of the treatment period (Day-90) with MIV-701 besylate salt was significantly reduced as compared to pretreatment period (Day-0) irrespective of the imagery technique used.

As shown in Figure 17 in CTX-1 responders, depth of the alveolar bone defect measured at the end of the treatment period (Day-90) with MIV-701 besylate salt was reduced as compared to pretreatment period (Day-0). Only favorable trends on the depth of the alveolar bone defect could be suspected in CBCT-images. However, significant improvements were obtained on depth of the alveolar bone loss when using X-ray images.

As shown in Figure 18 in CTX-1 responders, height of the alveolar crest measured at the end of the treatment period (Day-90) with MIV-701 besylate salt was reduced as compared to pretreatment period (Day-0). Significant improvements were obtained on height of the alveolar crest when using CBCT-scan images while only favorable trends could be suspected on X-ray radiography.

6.3. Secondary Objectives: Periodontal disease parameters

As shown in Figure 19 in CTX-1 responders, both Clinical Attachment Loss (CAL) and Periodontal Probing Depth (PPD) measured at the end of the treatment period (Day-90) with MIV-701 besylate salt was significantly improved as compared to pretreatment value (Day-0). In contrast, Gingival Bleeding Index (GBI) was not modified as compared to pre-treatment value.

*6.4. Safety*

Once-a-day treatments with MIV-701 besylate salt at 25 and 50 mg/kg/day were well tolerated throughout the 90-day treatment period. No death and no Serious Adverse Event (SAE) have been reported. Only 2 dogs experienced one episode of diarrhea of mild intensity for 2 days which spontaneously resolved under treatment. No safety concern could be depicted when analyzing evolution of blood cell count or evolution of clinical biochemistry parameters (Table 5).

*Table 5: Evolution of calcium, blood cell counts parameters and clinical biochemistry parameters during treatment with MIV-701 besylate salt for 90 days.*

| | Day 0 | Day 30 | Day 60 | Day 90 | SD |
|---|---|---|---|---|---|
| **Calcium (Mean)** | | | | | |
| Total calcium (mmol/L) | 2,5 | 2,4 | 2,3 | 2,4 | 0,07 |
| | | | | | |
| **Blood cell count (Mean)** | | | | | |
| Neutrophil (10^9/L) | 7,1 | 7,7 | 7,0 | 8,3 | 0,58 |
| Monocyte (10^9/L) | 0,8 | 0,8 | 0,7 | 0,9 | 0,05 |
| Lymphocyte (10^9/L) | 1,7 | 1,7 | 1,7 | 1,7 | 0,03 |
| Eosinophil (10^9/L) | 1,3 | 1,3 | 1,2 | 1,2 | 0,06 |
| Basophil (10^9/L) | 0,04 | 0,03 | 0,03 | 0,02 | 0,00 |
| Platelet (10^3 K/μL) | 423,1 | 381,3 | 384,8 | 423,0 | 23,14 |
| RBC (10^12/L) | 6,3 | 6,6 | 6,2 | 6,2 | 0,20 |
| HCT (%) | 40,5 | 41,8 | 40,0 | 40,1 | 0,81 |
| HGB (g/DL) | 13,7 | 14,2 | 13,6 | 13,6 | 0,3,0 |
| MCV (fL) | 64,2 | 63,5 | 64,6 | 65,7 | 0,93 |
| MCH (pg) | 21,6 | 21,5 | 21,8 | 22,2 | 0,28 |
| MCHC (g/DL) | 33,7 | 33,9 | 33,8 | 33,8 | 0,09 |
| Reticulocyte (K/μL) | 29,6 | 32,0 | 22,9 | 29.5 | 3,90 |
| **Biochemistry (Mean)** | | | | | |
| Glucose (mmol/L) | 5,6 | 5,3 | 5,7 | 5,7 | 0,21 |
| Creatinine (μmol/L) | 57,3 | 57,8 | 62,7 | 60,2 | 2,48 |
| Urea (mmol/L) | 4,5 | 4,0 | 5,4 | 5,4 | 0,66 |
| BUN/Creatinine ratio | 18,6 | 17,8 | 21,7 | 22,8 | 2,40 |
| Total protein (g/dL) | 7,2 | 7,0 | 6,8 | 7,1 | 0,19 |
| Albumin (g/L) | 27,2 | 26,3 | 26,9 | 28,3 | 0,84 |
| Globulin (g/L) | 44,7 | 43,6 | 40,6 | 43,0 | 1,73 |
| Albumin/Globulin ratio | 0,62 | 0,61 | 0,65 | 0,65 | 0,02 |
| Alanine transferase (U/L) | 38,5 | 37,9 | 41,7 | 36,8 | 2,10 |
| Alkalin phosphatase (U/L) | 34, 4 | 31,5 | 31,6 | 45,4 | 6,59 |

## REFERENCES

Albuquerque C, Morinha F, Requicha J, Martins T, Dias I, Guedes-Pinto H, Bastos E, Viegas C (2012). Canine periodontitis: the dog as an important model for periodontal studies. Vet J, 191, 299-305.

Baim S, Miller PD. Perspective: assessing the clinical utility of serum CTX in postmenopausal osteoporosis and its use in predicting risk of osteonecrosis of the jaw. J Bone Miner Res. 2009;24:561-574. doi: 10.1359/jbmr.090203). Bellows J. Periodontal equipment, materials, and techniques. Small Animal Dental Equipment, Materials, and Techniques: A Primer. Ames, IA: Blackwell, 2004, pp 115-173.

Butković, V., Šimpraga, M., Šehić, M., et al. (2001) Dental diseases of dogs: a retrospective study of radiological data. Acta Veterinaria Brno 70, 203-208

Chubb SA. Measurement of C-terminal telopeptide of type I collagen (CTX) in serum. Clin Biochem. 2012 Aug;45(12):928-35. doi: 10.1016/j.clinbiochem.2012.03.035. Epub 2012 Apr 6. PMID: 22504058.

Costa, A. G., Cusano, N. E., Silva, B. C., Cremers, S., and Bilezikian, J. P. (2011). Cathepsin K: its skeletal actions and role as a therapeutic target in osteoporosis. Nat. Rev. Rheumatol. 7, 447-456. doi: 10.1038/nrrheum.2011.77 Dai R, Wu Z, Chu HY, Lu J, Lyu A, Liu J, Zhang G. Cathepsin K: The Action in and Beyond Bone. Front Cell Dev Biol. 2020 Jun 4;8:433. doi: 10.3389/fcell.2020.00433. PMID: 32582709; PMCID: PMC7287012.

Debowes U: Problems with the gingiva. In: Small Animal dental, oral and maxillofacial disease, a color handbook (Niemiec BA ed.). London, Manson, 2010, pp159 - 181. Diehl KH, Hull R, Morton D, Pfister R, Rabemampianina Y, Smith D, Vidal JM, van de Vorstenbosch C; European Federation of Pharmaceutical Industries Association and European Centre for the Validation of Alternative Methods. A good practice guide to the administration of substances and removal of blood, including routes and volumes. J Appl Toxicol. 2001 Jan-Feb;21(1):15-23. doi: 10.1002/jat.727. PMID: 11180276.

De Faria Vasconcelos K, Evangelista KM, Rodrigues CD, Estrela C, de Sousa TO, Silva MA. Detection of periodontal bone loss using cone beam CT and intraoral radiography. Dentomaxillofac Radiol. 2012 Jan;41(1):64-9. Drake MT, Clarke BL, Oursler MJ, Khosla S. Cathepsin K Inhibitors for Osteoporosis: Biology, Potential Clinical Utility, and Lessons Learned. Endocr Rev. 2017 Aug 1;38(4):325-350

Duong LT. Therapeutic inhibition of cathepsin K-reducing bone resorption while maintaining bone formation. Bonekey Rep. 2012 May 2;1:67. Farley JR, Tarbaux N, Murphy LA, Masuda T, Baylink DJ. In vitro evidence that bone formation may be coupled to resorption by release of mitogen(s) from resorbing bone. Metabolism 1987;36:314-21.

Fiorellini JP, Ishikawa SO, Kim DM. Gingival Inflammation. Carranza's Clinical Periodontology. St. Louis: WB Saunders, 2006, pp 355-361.

Gawor JP, Strøm P, Nemec A. Treatment of Naturally Occurring Periodontitis in Dogs With a New Bio-Absorbable Regenerative Matrix. Front Vet Sci. 2022 Jun 21;9:916171.

Gowen, M., et al., Cathepsin K knockout mice develop osteopetrosis due to a deficit in matrix degradation but not demineralization. J Bone Miner Res, 1999. 14(10): p. 1654-63.

Hamp, S. E., Olsson, S. E., Farsø-Madsen, K., et al. (1984) A macroscopic and radiological investigation of dental diseases of the dog. Veterinary Radiology 25, 86-92

Hoffman, T. & Gaengler, P. (1996) Epidemiology of periodontal disease in poodles. Journal of Small Animal Practice 37, 309-316

Legeros, R. Z. & Shannon, I. L. (1979) The crystalline components of dental cal- culi: human vs. dog. Journal of Dental Research 58, 2371-2377

Li CY, Jepsen KJ, Majeska RJ, Zhang J, Ni R, Gelb BD, et al. Mice lacking cathepsin K maintain bone remodeling but develop bone fragility despite high bone mass. J Bone Miner Res 2006;21:865-75.

Lund, E. M., Armstrong, P. J., Kirk, C. A., et al. (1999) Health status and population characteristics of dogs and cats examined at private veterinary practices in the United States. Journal of the American Veterinary Medical Association 214, 1336-1341

Marshall, M. D., Wallis, C. V., Milella, L., et al. (2014) A longitudinal assessment of periodontal disease in 52 miniature schnauzers. BMC Veterinary Research 10, 166

Mazur (2006) Features of bone tissue metabolic processes in patients with generalized periodontitits in the period of disease exacerbation. Gerontologija 7(4): 174-179

Niemiec, B. A. (2008) Oral pathology. Topics in Companion Animal Medicine 23, 59-71

Niemiec BA. (2010) Pathologies of the oral mucosa. In; Small animal dental, oral, and maxillofacial disease, a color handbook (Niemiec BA (ed). London, Manson, 2010, 183-98.

Niemiec BA (2013a) Local and Regional Consequences of Periodontal Disease. In: Veterinary Periodontology. (Niemiec BA, ed). Ames, Wiley Blackwell, 69-80.

Niemiec BA (2013b) Systemic Manifestations of Periodontal Disease. In: Veterinary Periodontology. (Niemiec BA, ed). Ames, Wiley Blackwell, 81-90.

Niemiec B, Gawor J, Nemec A, Clarke D, McLeod K, Tutt C, Gioso M, Steagall PV, Chandler M, Morgenegg G, Jouppi R. World Small Animal Veterinary Association Global Dental Guidelines. J Small Anim Pract. 2020 Jul;61(7):E36-E161. doi: 10.1111/jsap.13132. Erratum in: J Small Anim Pract. 2020 Dec;61(12):786. PMID: 32715504.

Nikahval B, Nazifi S, Heidari F, Khafi M (2016) Evaluation of the changes of P1NP and CTX in synovial fluid and blood serum of dogs with experimental osteoarthritis. Comp Clin Pathol O'Neill, D. G., Church, D. B., Mcgreevy, P. D., et al. (2014) Prevalence of disorders recorded in dogs attending primary-care veterinary practices in England. PLoS One 9.

Pavlica, Z., Petelin, M., Juntes, P., et al. (2008) Periodontal disease burden and pathological changes in organs of dogs. Journal of Veterinary Dentistry 25, 97- 105

Pfeilschifter J, Mundy GR. Modulation of type beta transforming growth factor activity in bone cultures by osteotropic hormones. Proc Natl Acad Sci U S A 1987;84:2024-8.

Robinson, N. J., Dean, R. S., Cobb, M., et al. (2016) Factors influencing common diagnoses made during first-opinion small-animal consultations in the United Kingdom. Preventive Veterinary Medicine 131, 87-94

Shoukry M, Ben Ali L, Abdel Naby M, Soliman A. Repair of experimental plaque-induced periodontal disease in dogs. J Vet Dent. 2007 Sep;24(3):152-65.

Soukup J (2010). Periodontitis. In: Textbook of veterinary internal medicine, 7th edition. Editors: S Ettinger and E Feldman. Saunders Elsevier (Missouri), 179-184.

Stoch SA, Zajic S, Stone J, Miller DL, Van Dyck K et al. Effect of the cathepsin K inhibitor odanacatib on bone resorption biomarkers in healthy postmenopausal women: two double-blind, randomized, placebo-controlled phase I studies. Clin Pharmacol Ther. 2009 Aug;86(2): 175-82.

Vrbanac Z, Brkljaca Bottegaro N, Skrlin B, Bojanic K, Kusec V, Stanin D, Belic M. The Effect of a Moderate Exercise Program on Serum Markers of Bone Metabolism in Dogs. Animals (Basel). 2020 Aug 23;10(9):1481. doi: 10.3390/ani10091481. PMID: 32842472; PMCID: PMC7552239.

Wallis C, Holcombe LJ. A review of the frequency and impact of periodontal disease in dogs. J Small Anim Pract. 2020 Sep;61(9):529-540.

Wiggs, R.B. and Lobprise, H.B. (1997) Periodontology. In: Veterinary

Wiggs, R.B. and Lobprise, H.B. (1997) Periodontology. In: Veterinary Dentistry, Principles and Practice, Lippincott Raven Publishers, Philadelphia, PA, 186-231.

Wolf HF, Rateitschak EM, Rateitschak KH et al. Color atlas of dental medicine: periodontology, 3rd ed. Stuttgart: Georg Thieme Verlag, 2005.)

## Claims

1. A compound selected from N-[(S)-1-((3aS,6S,6aS)-6-fluoro-3-oxo-hexahydro-furo[3,2-b]pyrrole-4-carbonyl)-3-methyl-butyl]-4-[2-(4-methyl-piperazin-1-yl)-thiazol-4-yl]-benzamide, N-[(S)-1-((3aS,6S,6aS)-6-Fluoro-3,3-dihydroxy-hexahydro-furo[3,2-b]pyrrole-4-carbonyl)-3-methyl-butyl]-4-[2-(4-methyl-piperazin-1-yl)-thiazol-4-yl]-benzamide, mixtures thereof, and pharmaceutically acceptable salts thereof, for use in the treatment of a disorder mediated by cathepsin K in an animal.

2. Compound for use according to claim 1, wherein the disorder is a disorder associated with bone loss and/or a disorder associated with bone resorption.

3. Compound for use according to any of the preceding claims, wherein the disorder is a disorder associated with alveolar bone loss and/or a disorder associated with alveolar bone resorption.

4. Compound for use according to any of the preceding claims, wherein the disorder is a periodontal disease, such as gingivitis and/or periodontitis.

5. Compound for use according to any of the preceding claims, wherein the disorder is a disorder associated with alveolar bone loss and periodontal disease.

6. Compound for use according to any of claims 1 to 3, wherein the disorder is a tooth resorption, such as dentin resorption and/or root resorption.

7. Compound for use according to any of claims 1 or 2, wherein the disorder is arthritis, such as osteoarthritis or rheumatoid arthritis.

8. Compound for use according to any of claims 1 or 2, wherein the disorder is osteoporosis.

9. Compound for use according to any of the preceding claims, wherein the pharmaceutically acceptable salt is the besylate salt.

10. Compound for use according to any of the preceding claims, wherein the animal is mammal, preferably selected from a canine, a feline, a bovine, an ovine, an equine or a rodent, most preferably selected from a canine or a feline.

**11.** Compound for use according to any of the preceding claims, wherein the animal is a cat or a dog.

**12.** Compound for use according to any of the preceding claims, wherein the compound or pharmaceutically acceptable salt is administered orally or systemically.

**13.** Compound for use according to any of the preceding claims, wherein the compound or pharmaceutically acceptable salt is administered in a unit dosage form, preferably as capsule or tablets.

**14.** Compound for use according to any of the preceding claims, wherein the compound or pharmaceutically acceptable salt is administered in a daily amount corresponding to 1-300 mg/kg.

**15.** Compound for use according to any of the preceding claims, wherein the CTX-1 (Carboxy-terminal crosslinked telopeptide of type 1 collagen) plasma concentration in the animal is decreased by at least 10% compared to the CTX-1 plasma concentration before treatment.

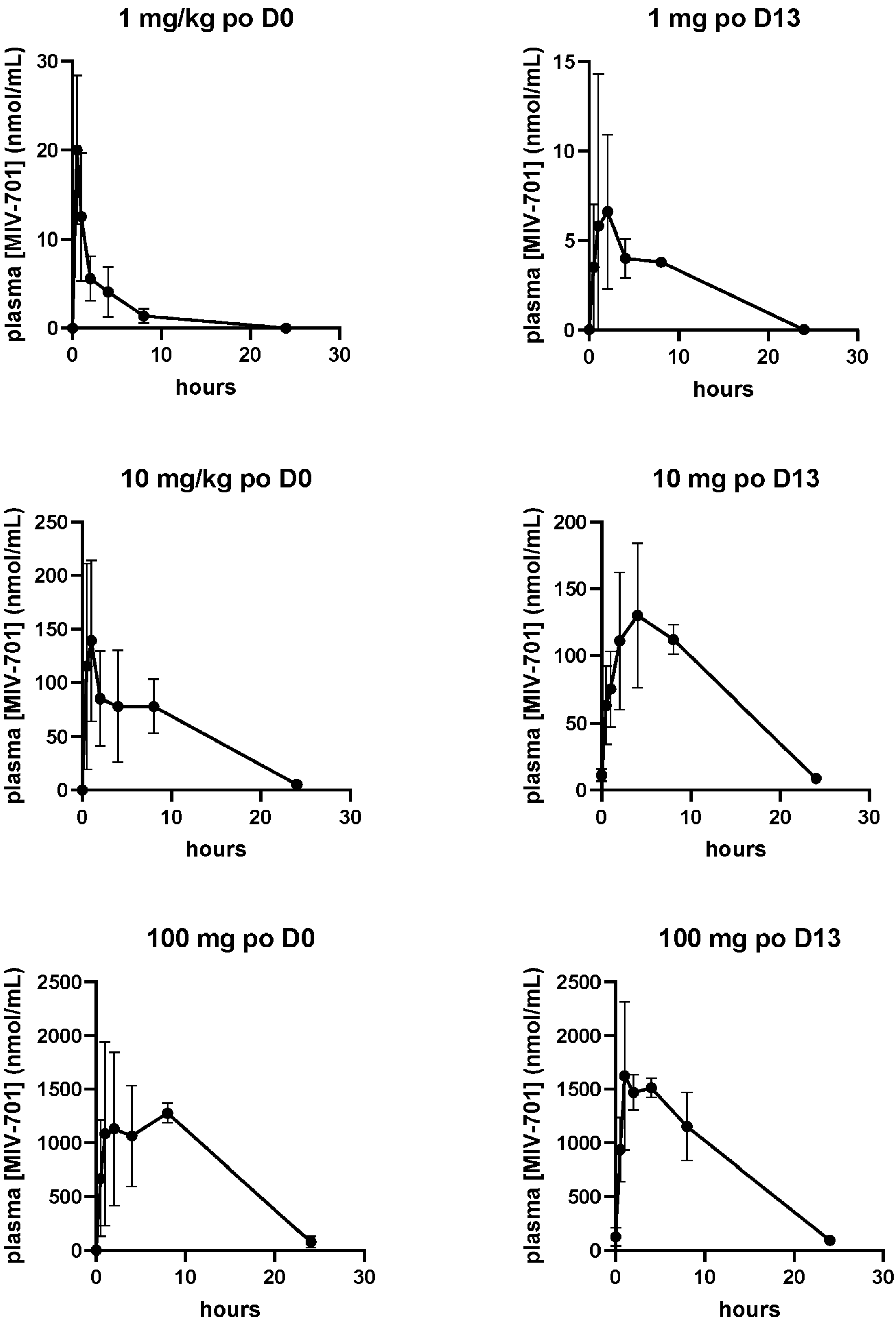
1 mg/kg po D0
plasma [MIV-701] (nmol/mL)
30
20
10
0
0
10
20
30
hours
1 mg po D13
plasma [MIV-701] (nmol/mL)
15
10
5
0
0
10
20
30
hours
10 mg/kg po D0
plasma [MIV-701] (nmol/mL)
250
200
150
100
50
0
0
10
20
30
hours
10 mg po D13
plasma [MIV-701] (nmol/mL)
200
150
100
50
0
0
10
20
30
hours
100 mg po D0
plasma [MIV-701] (nmol/mL)
2500
2000
1500
1000
500
0
0
10
20
30
hours
100 mg po D13
plasma [MIV-701] (nmol/mL)
2500
2000
1500
1000
500
0
0
10
20
30
hours

**Figure 1**

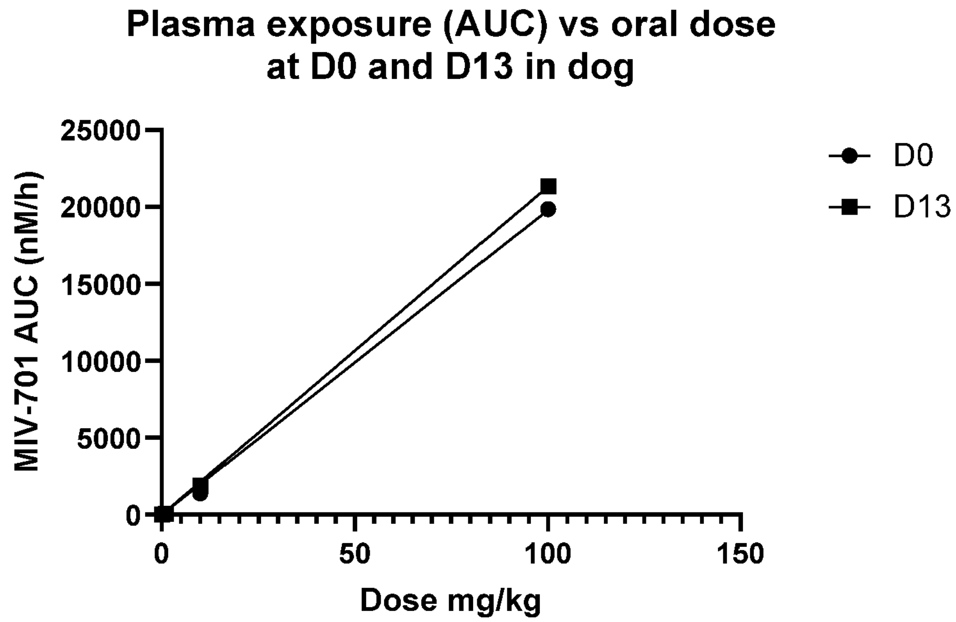


Figure 2

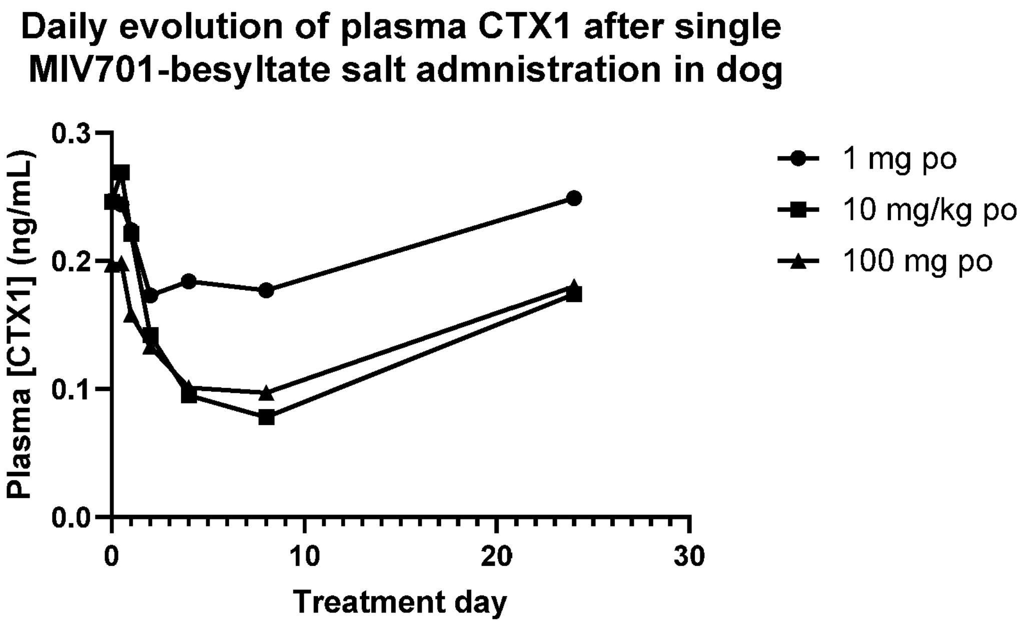


Figure 3

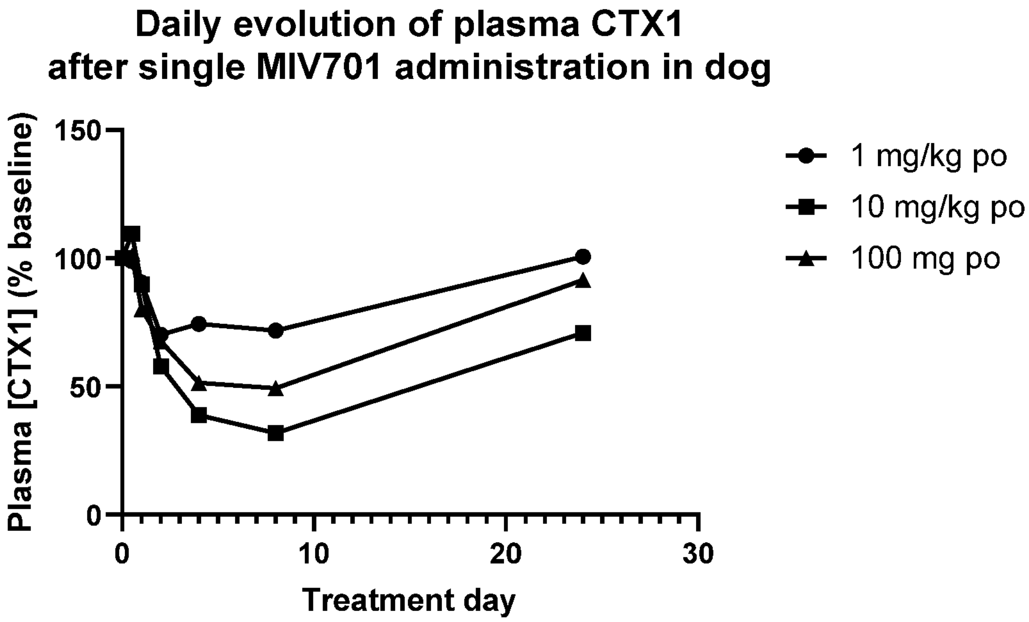


Figure 4

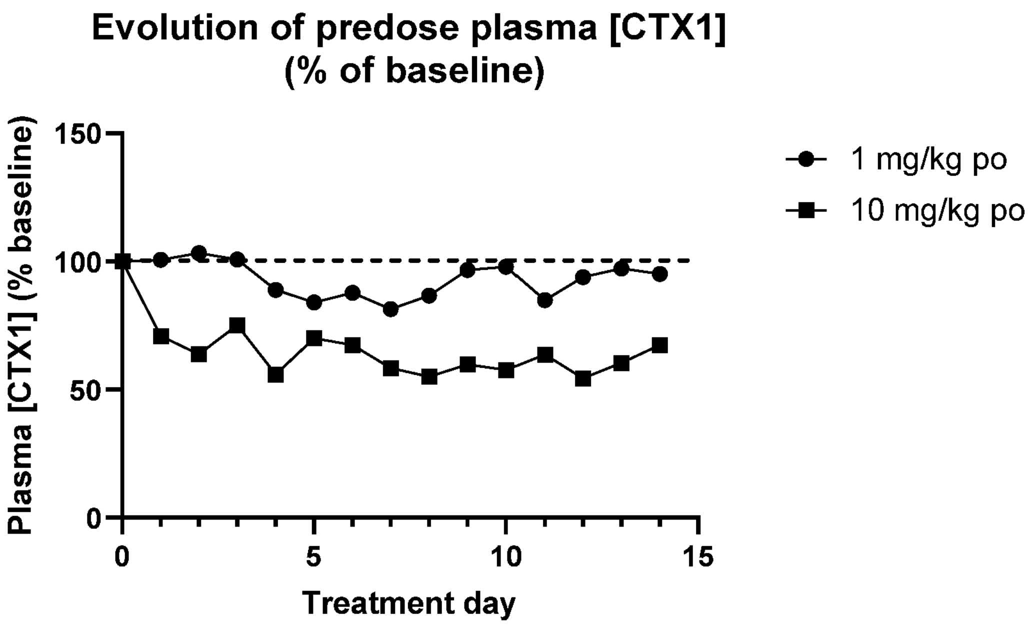


Figure 5

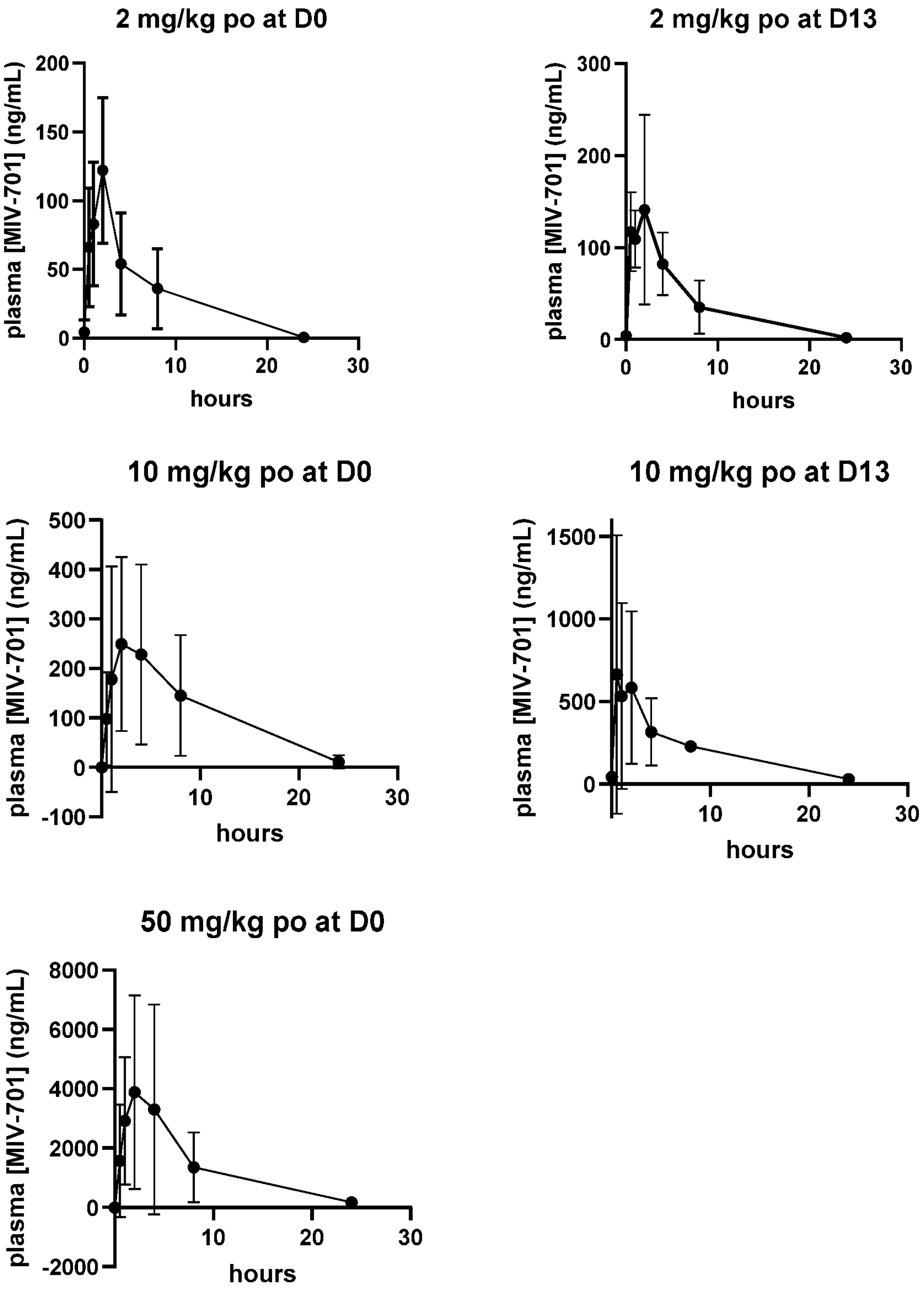
2 mg/kg po at D0
2 mg/kg po at D13
10 mg/kg po at D0
10 mg/kg po at D13
50 mg/kg po at D0
plasma [MIV-701] (ng/mL)
hours

**Figure 6**

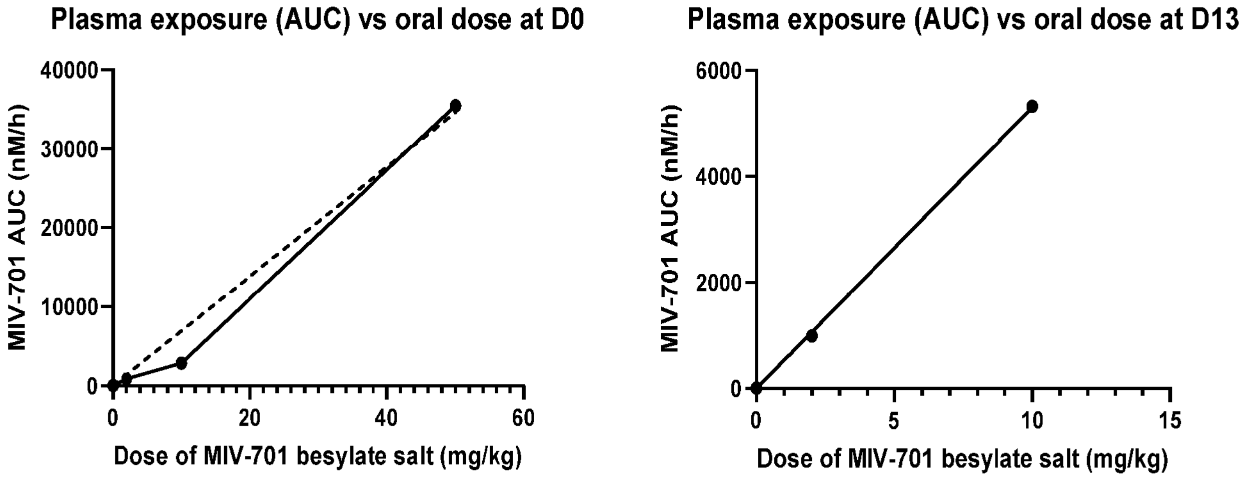


Figure 7

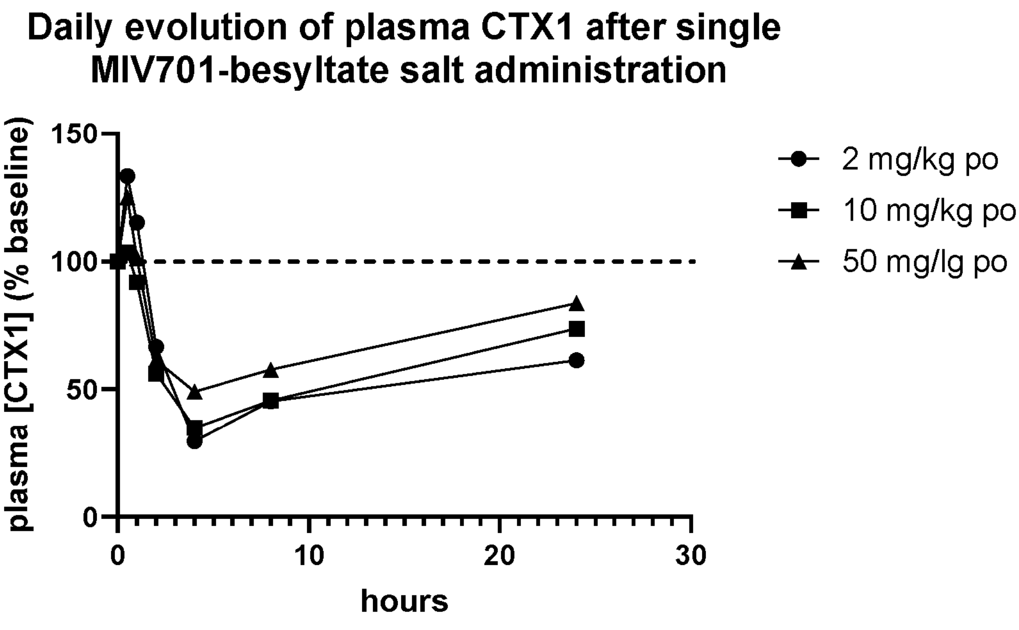


Figure 8

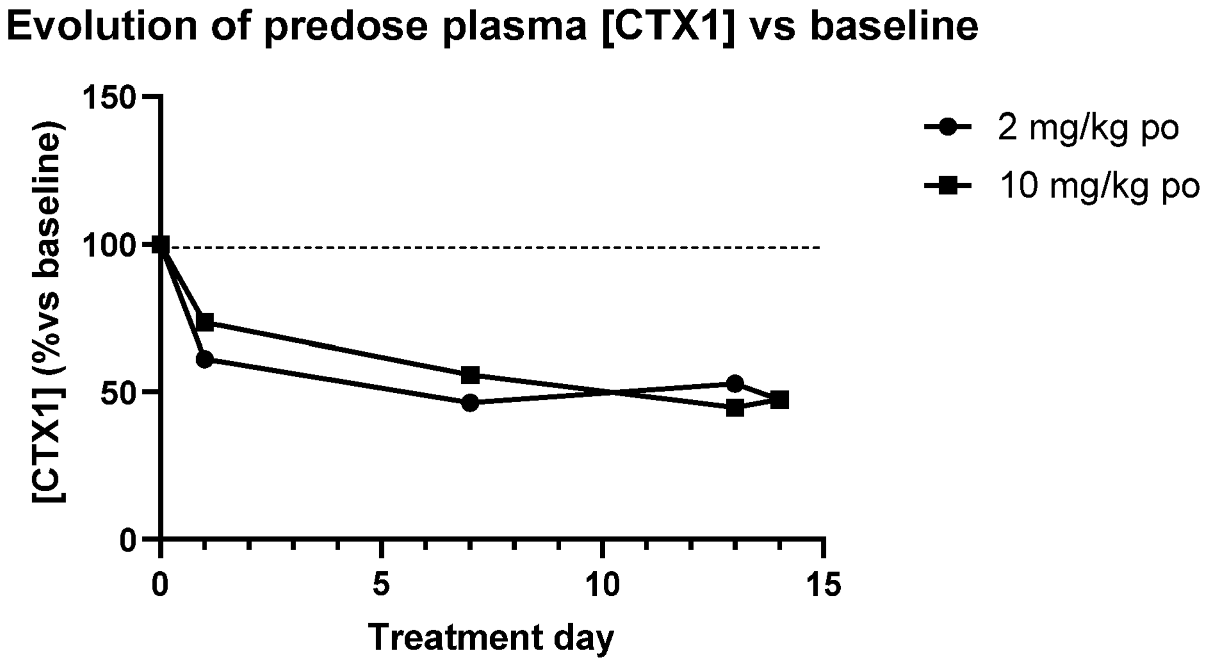
Evolution of predose plasma [CTX1] vs baseline
[CTX1] (%vs baseline)
150
100
50
0
0
5
10
15
Treatment day
2 mg/kg po
10 mg/kg po

**Figure 9**

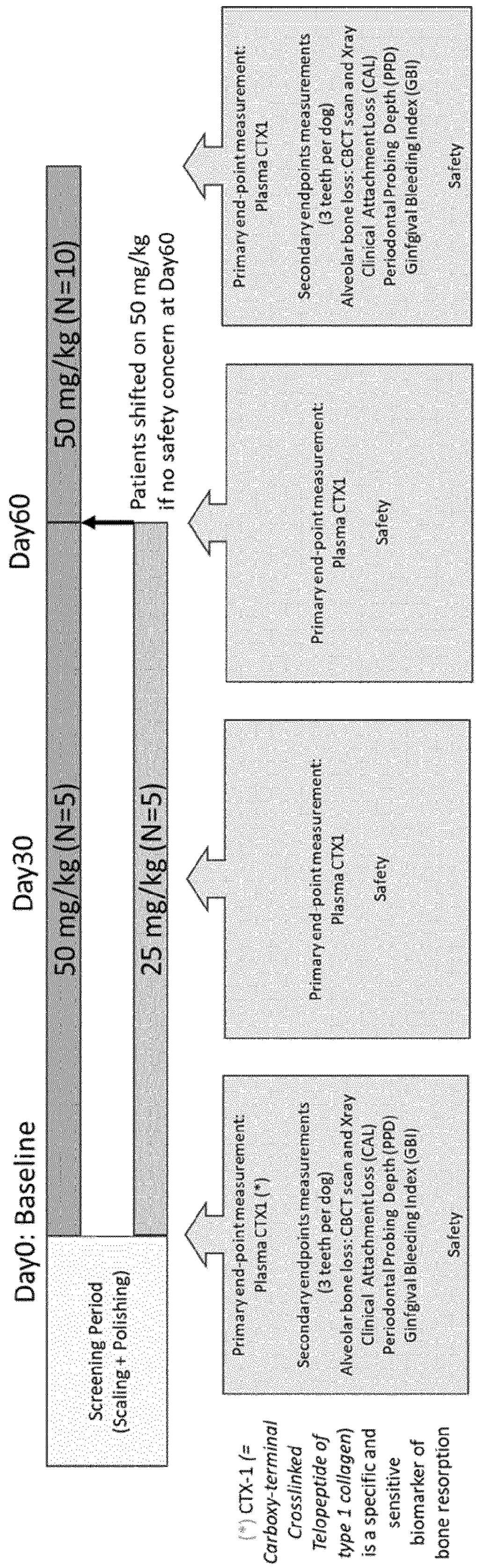
Day0: Baseline
Day30
Day60
Screening Period
(Scaling + Polishing)
50 mg/kg (N=5)
25 mg/kg (N=5)
50 mg/kg (N=10)
Patients shifted on 50 mg/kg
if no safety concern at Day60
Primary end-point measurement:
Plasma CTX1 (*)
Secondary endpoints measurements
(3 teeth per dog)
Alveolar bone loss: CBCT scan and Xray
Clinical Attachment Loss (CAL)
Periodontal Probing Depth (PPD)
Ginfgival Bleeding Index (GBI)
Safety
Primary end-point measurement:
Plasma CTX1
Safety
Primary end-point measurement:
Plasma CTX1
Safety
Primary end-point measurement:
Plasma CTX1
Secondary endpoints measurements
(3 teeth per dog)
Alveolar bone loss: CBCT scan and Xray
Clinical Attachment Loss (CAL)
Periodontal Probing Depth (PPD)
Ginfgival Bleeding Index (GBI)
Safety
(*) CTX-1 (= Carboxy-terminal Crosslinked Telopeptide of type 1 collagen) is a specific and sensitive biomarker of bone resorption

**Figure 10**

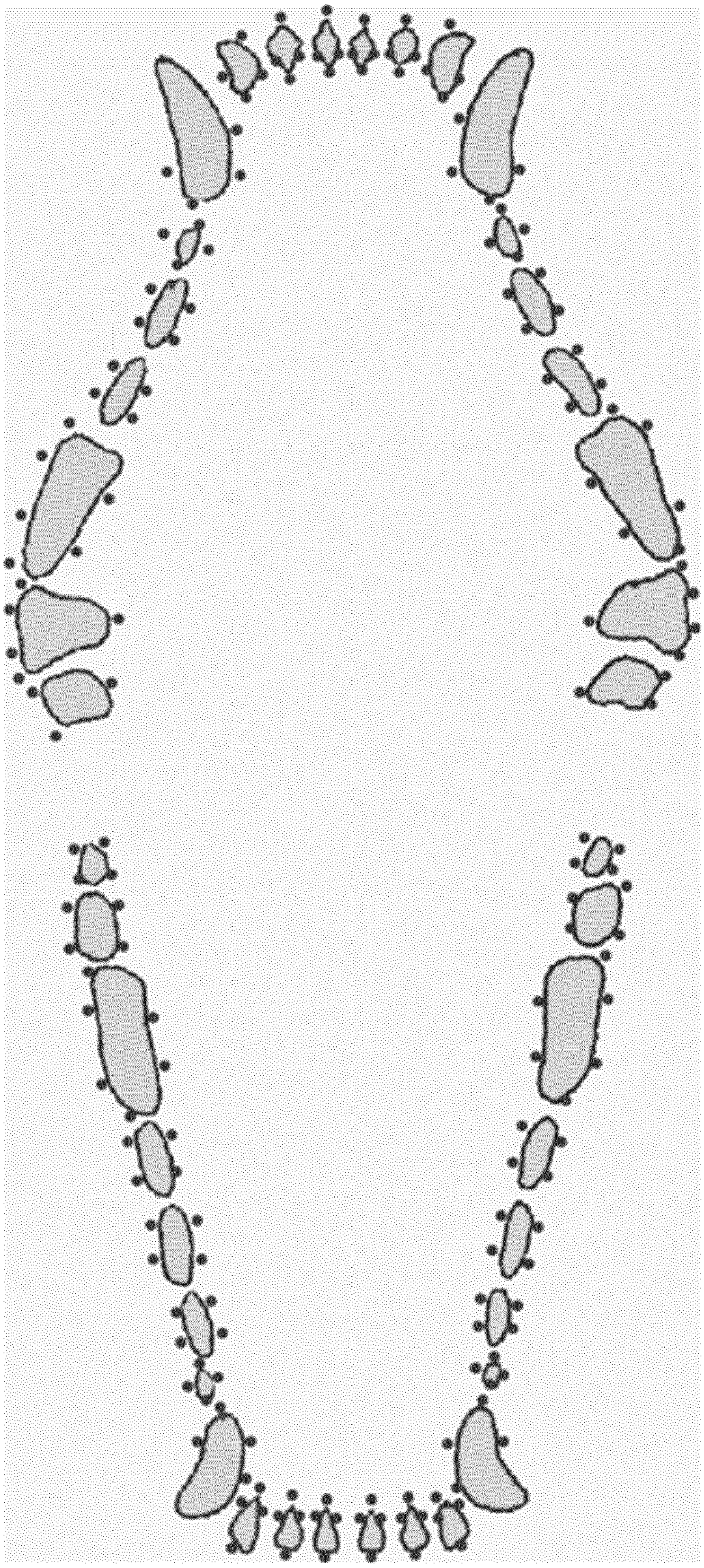

Figure 11

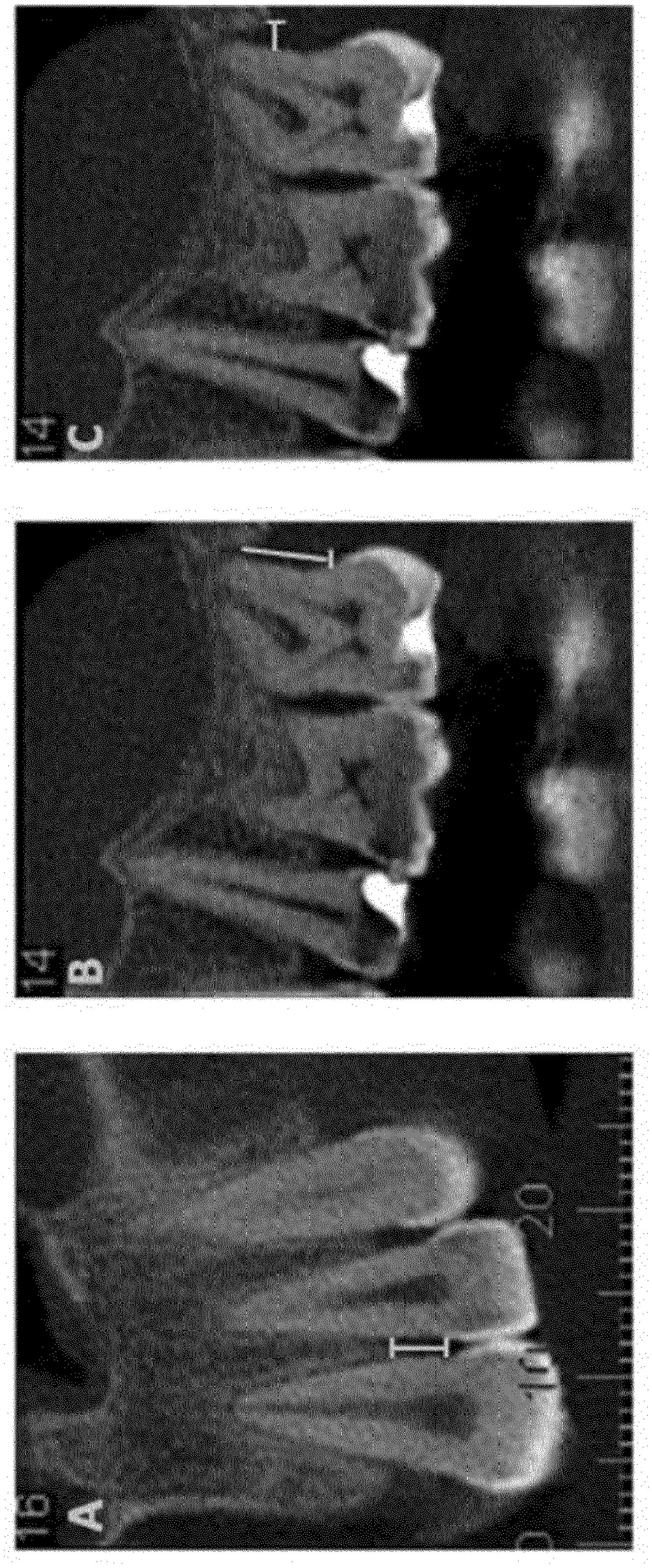


**Figure 12**

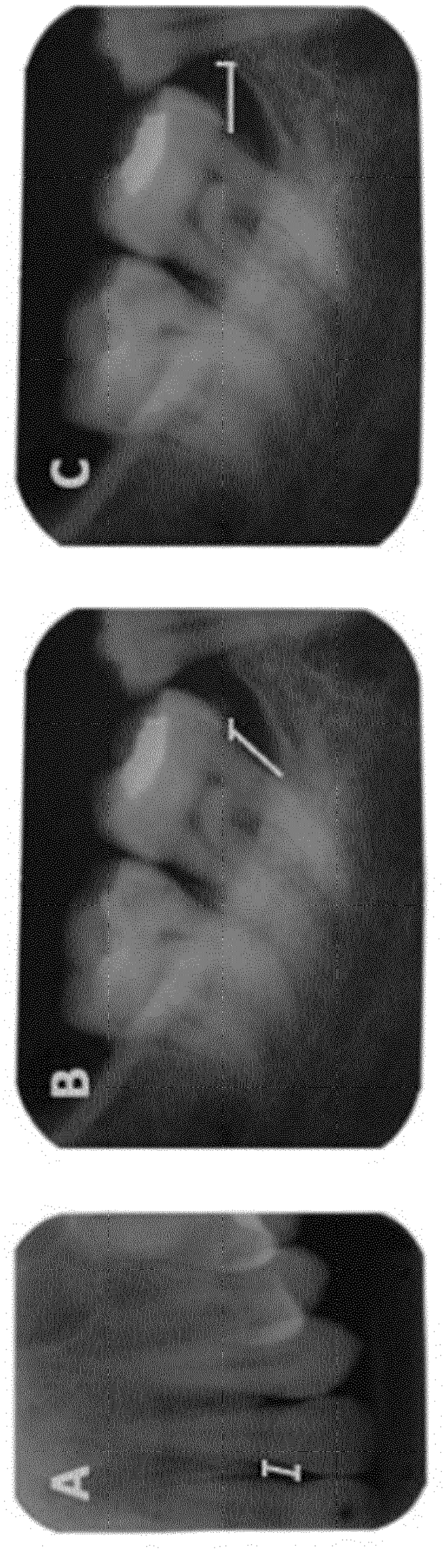


**Figure 13**

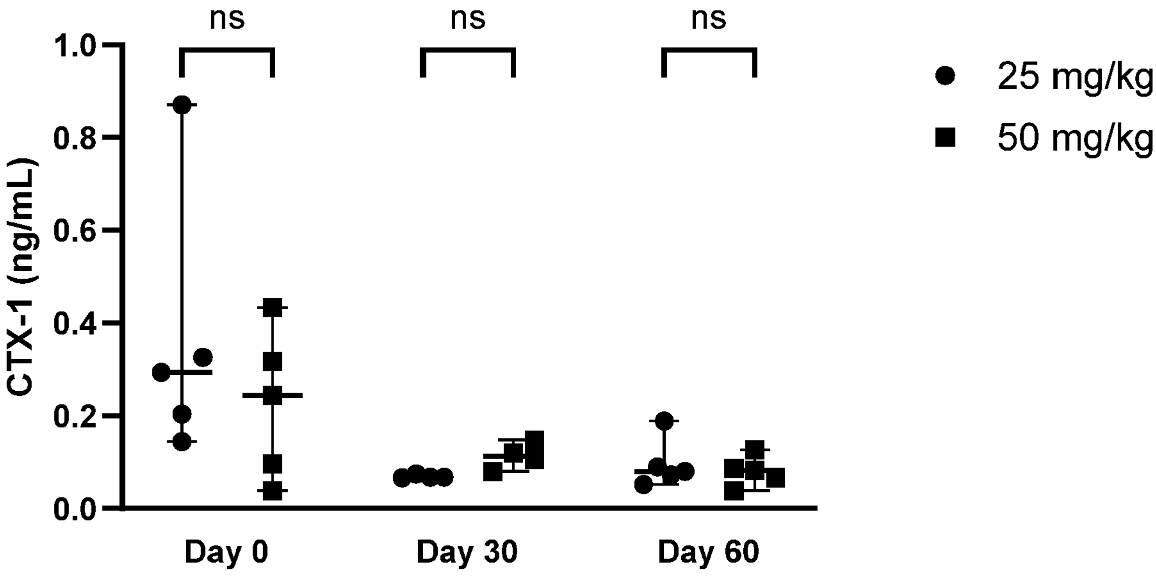


**Figure 14**

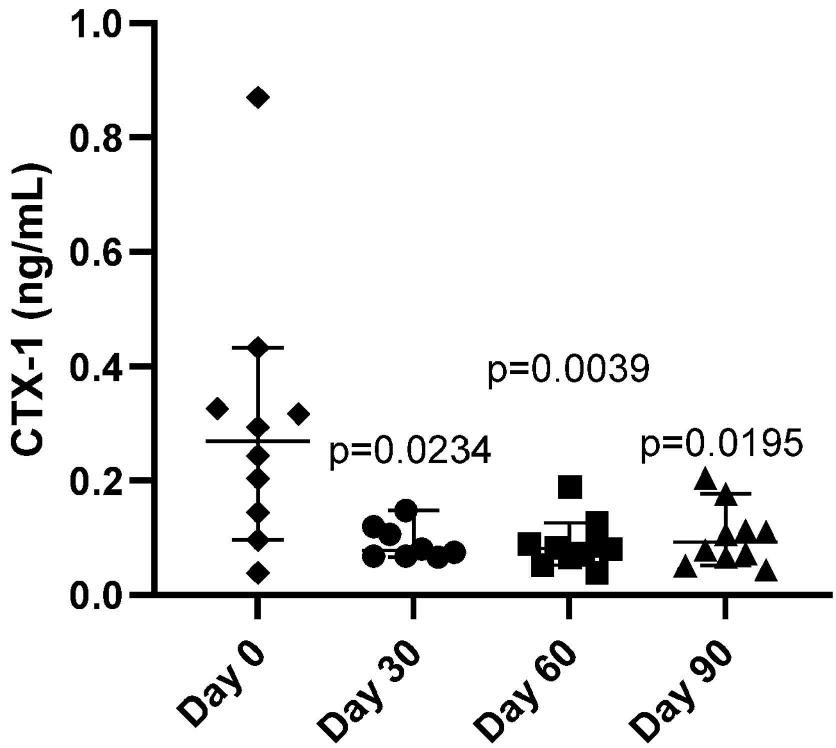


**Figure 15**

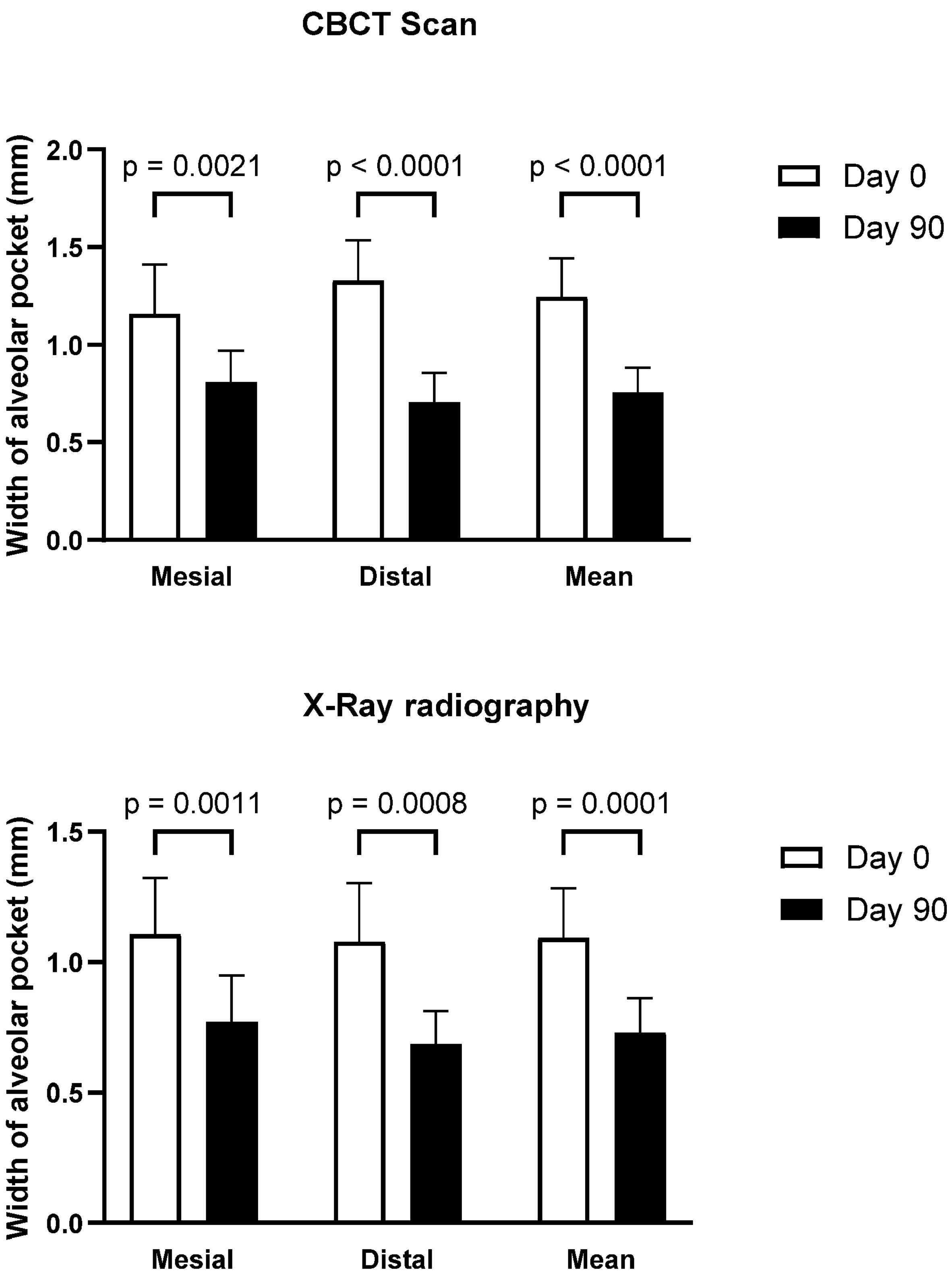
CBCT Scan
Width of alveolar pocket (mm)
2.0
1.5
1.0
0.5
0.0
p = 0.0021
p < 0.0001
p < 0.0001
Mesial
Distal
Mean
Day 0
Day 90
X-Ray radiography
Width of alveolar pocket (mm)
1.5
1.0
0.5
0.0
p = 0.0011
p = 0.0008
p = 0.0001
Mesial
Distal
Mean
Day 0
Day 90

**Figure 16**

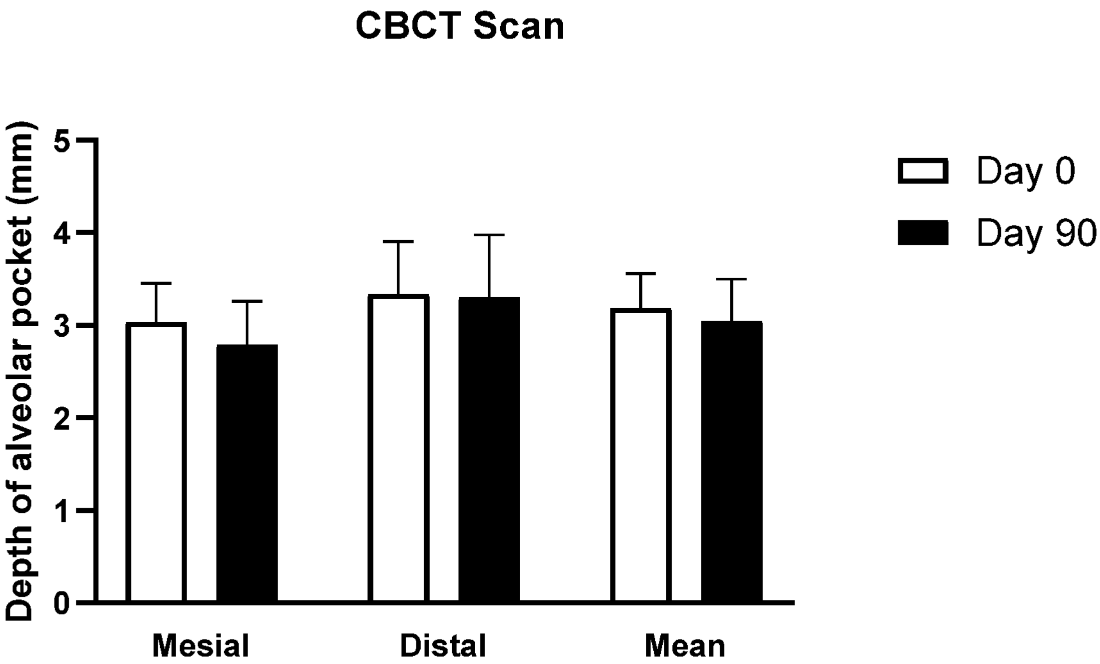
CBCT Scan
Depth of alveolar pocket (mm)
0
1
2
3
4
5
Mesial
Distal
Mean
Day 0
Day 90

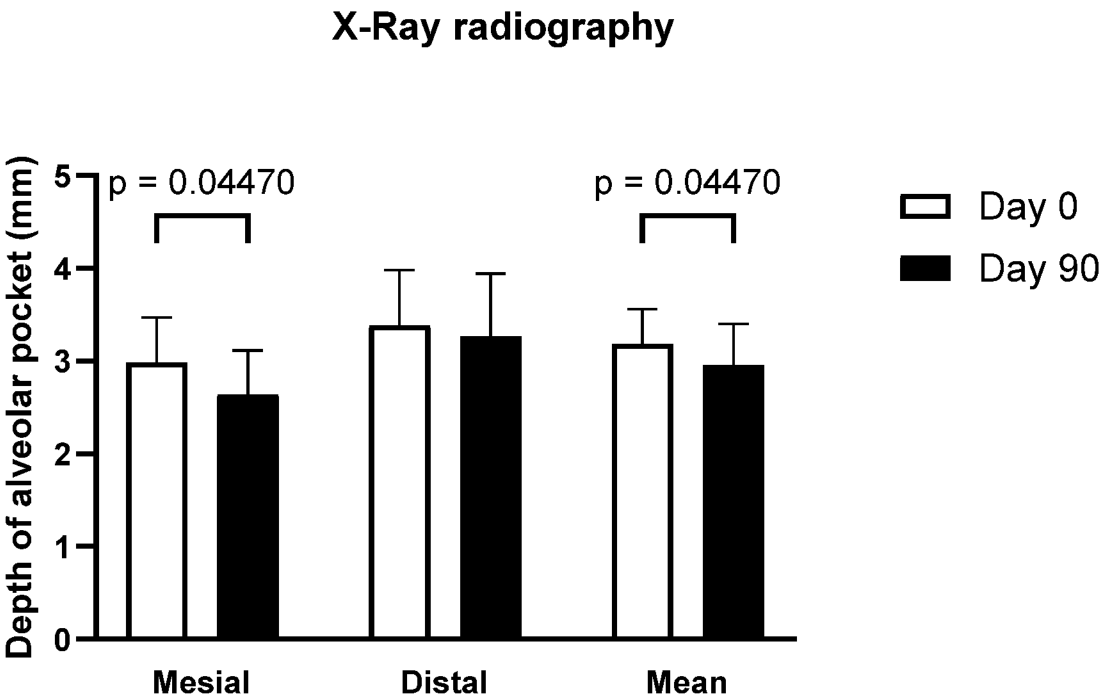
X-Ray radiography
Depth of alveolar pocket (mm)
0
1
2
3
4
5
p = 0.04470
p = 0.04470
Mesial
Distal
Mean
Day 0
Day 90

Figure 17

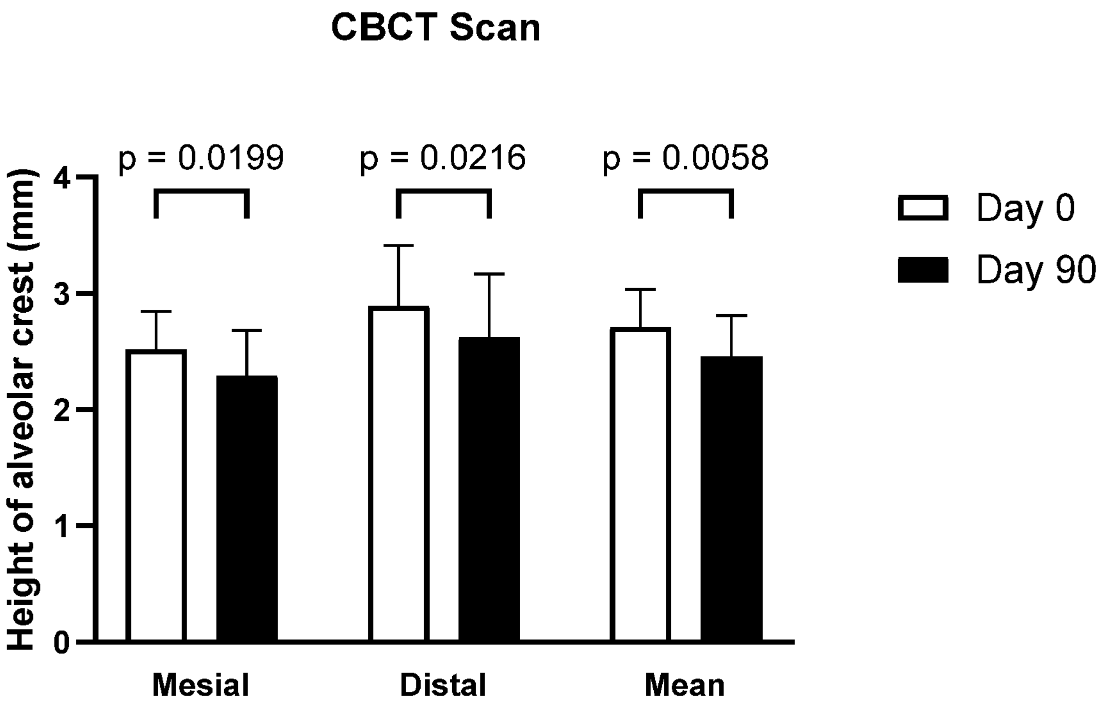
CBCT Scan
p = 0.0199
p = 0.0216
p = 0.0058
Height of alveolar crest (mm)
4
3
2
1
0
Mesial
Distal
Mean
Day 0
Day 90

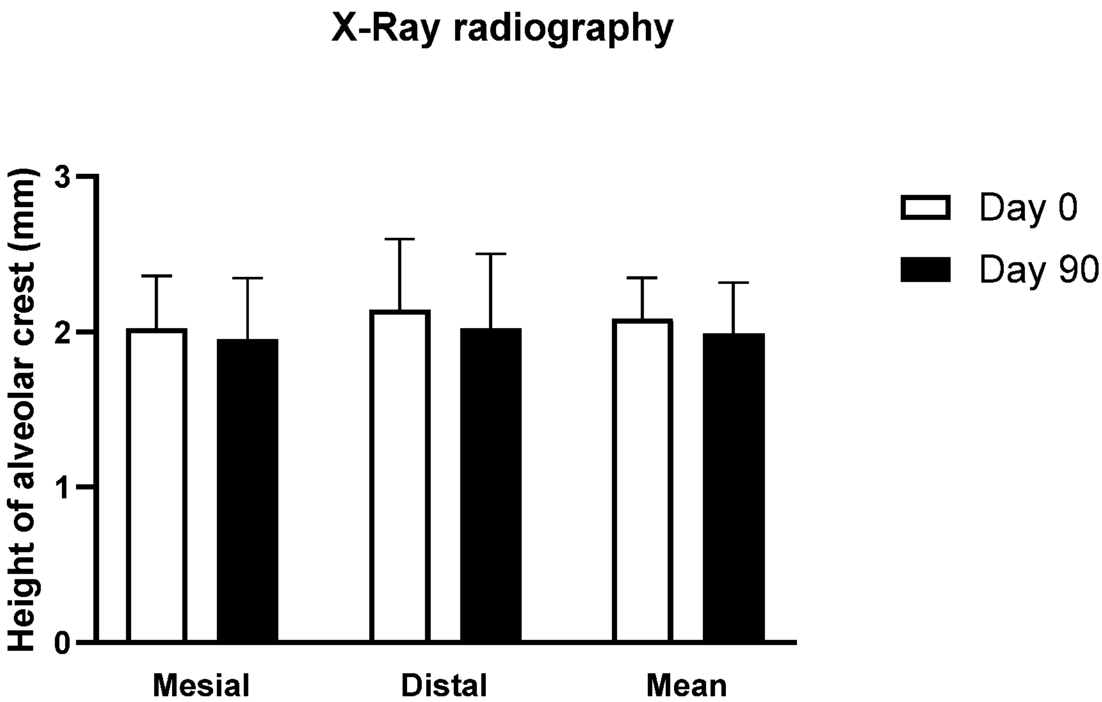
X-Ray radiography
Height of alveolar crest (mm)
3
2
1
0
Mesial
Distal
Mean
Day 0
Day 90

Figure 18

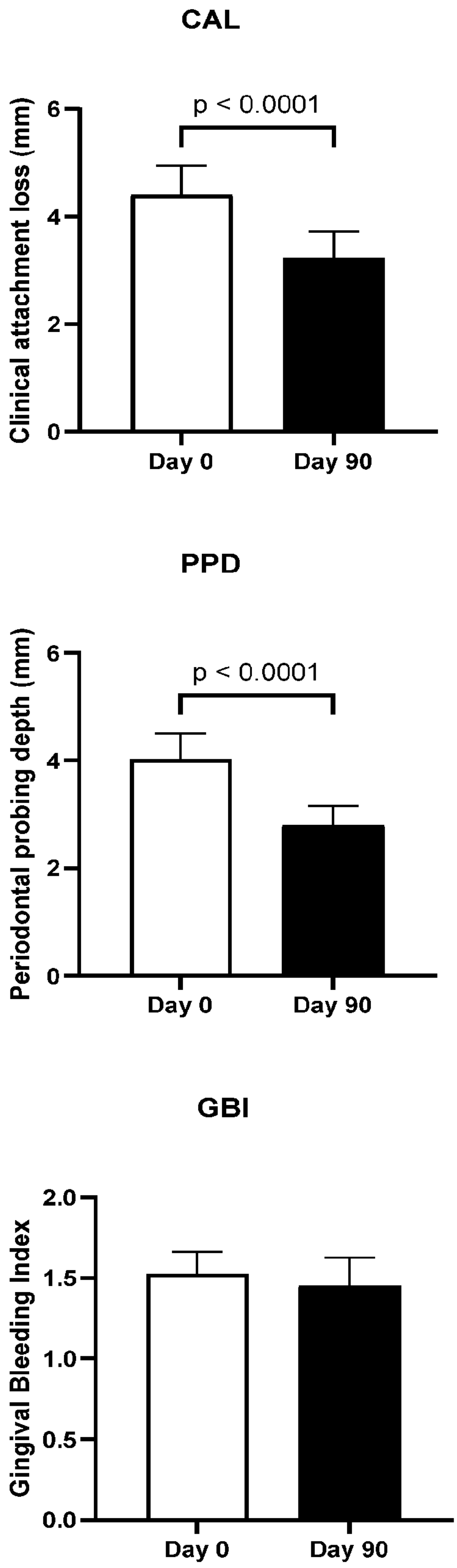
CAL
p < 0.0001
Clinical attachment loss (mm)
0
2
4
6
Day 0
Day 90
PPD
p < 0.0001
Periodontal probing depth (mm)
0
2
4
6
Day 0
Day 90
GBI
Gingival Bleeding Index
0.0
0.5
1.0
1.5
2.0
Day 0
Day 90

**Figure 19**

Europäisches Patentamt
European Patent Office
Office européen des brevets

# EUROPEAN SEARCH REPORT

Application Number
EP 24 30 5339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2008/034781 A1 (MEDIVIR AB [SE]; GRANT CRAIG [GB]; WATT STEPHEN [GB])<br>27 March 2008 (2008-03-27)<br>* Claims 1, 9-10, page 7, par.3 * | 1-11, 13-15<br>10,11, 13,14 | INV.<br>A61K31/496<br>A61P1/02<br>A61P19/00<br>A61P19/02<br>A61P19/10 |
| X<br>Y | WO 2005/066180 A1 (MEDIVIR AB [SE]; NILSSON MAGNUS [SE] ET AL.)<br>21 July 2005 (2005-07-21)<br>* Claims 1, 29-30, Compound 8.19 * | 1-4,7,15<br>10,11, 13,14 | |
| X<br>Y | US 9 943 522 B2 (GOLZ STEFAN [DE]; DELBECK MARTINA [DE] ET AL.)<br>17 April 2018 (2018-04-17)<br>* Claims 1, 4 * | 1,12,15<br>10,11, 13,14 | |
| Y | DATABASE BIOSIS [Online]<br>BIOSCIENCES INFORMATION SERVICE, PHILADELPHIA, PA, US;<br>September 2008 (2008-09),<br>BOETTIGER D ET AL: "Cathepsin K Inhibitor, MIV-701, Suppresses Bone Resorption Markers in Healthy PMW: Results of a Phase I Clinical Trial",<br>XP002812049,<br>Database accession no. PREV200900090853<br>* abstract *<br>& JOURNAL OF BONE AND MINERAL RESEARCH, vol. 23, no. Suppl. S,<br>September 2008 (2008-09), page S124,<br>30TH ANNUAL MEETING OF THE AMERICAN-SOCIETY-FOR-BONE-AND-MINERAL-RESEARCH; MONTREAL, CANADA; SEPTEMBER 12 -16, 2008<br>ISSN: 0884-0431<br>* page 5, right column, par.2 * | 10,11, 13,14 | TECHNICAL FIELDS SEARCHED (IPC)<br>A61K<br>A61P |

-/--

The present search report has been drawn up for all claims

2

| Place of search | Date of completion of the search | Examiner |
|---|---|---|
| Munich | 3 September 2024 | Healy, Cathal |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 24 30 5339

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | Lindberg Jens: "MEDIVIR AB INTERIM REPORT January to June 2022", Internet , 30 June 2022 (2022-06-30), pages 1-13, XP093180827, Retrieved from the Internet: URL:https://www.medivir.com/media/2001/q2-eng.pdf [retrieved on 2024-07-02] * page 5, right column, par.2 * ----- | 10,11, 13,14 | |

TECHNICAL FIELDS SEARCHED (IPC)

The present search report has been drawn up for all claims

2

| Place of search | Date of completion of the search | Examiner |
|---|---|---|
| Munich | 3 September 2024 | Healy, Cathal |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

page 2 of 2

## ANNEX TO THE EUROPEAN SEARCH REPORT ON EUROPEAN PATENT APPLICATION NO. EP 24 30 5339

This annex lists the patent family members relating to the patent documents cited in the above-mentioned European search report. The members are as contained in the European Patent Office EDP file on
The European Patent Office is in no way liable for these particulars which are merely given for the purpose of information.

03-09-2024

| Patent document cited in search report | Publication date | Patent family member(s) | Publication date |
|---|---|---|---|
| WO 2008034781 A1 | 27-03-2008 | NONE | |
| WO 2005066180 A1 | 21-07-2005 | AR 047373 A1 | 18-01-2006 |
| | | AT E461200 T1 | 15-04-2010 |
| | | AU 2005203972 A1 | 21-07-2005 |
| | | BR PI0506719 A | 02-05-2007 |
| | | CA 2552739 A1 | 21-07-2005 |
| | | EP 1701960 A1 | 20-09-2006 |
| | | ES 2341869 T3 | 29-06-2010 |
| | | HK 1096956 A1 | 15-06-2007 |
| | | JP 4787765 B2 | 05-10-2011 |
| | | JP 2007517852 A | 05-07-2007 |
| | | TW I343384 B | 11-06-2011 |
| | | US 2008234260 A1 | 25-09-2008 |
| | | US 2011112089 A1 | 12-05-2011 |
| | | WO 2005066180 A1 | 21-07-2005 |
| US 9943522 B2 | 17-04-2018 | AU 2012257779 A1 | 28-11-2013 |
| | | BR 112013029672 A2 | 17-01-2017 |
| | | CA 2835913 A1 | 22-11-2012 |
| | | CN 103687593 A | 26-03-2014 |
| | | EP 2709602 A1 | 26-03-2014 |
| | | ES 2648819 T3 | 08-01-2018 |
| | | JP 6000340 B2 | 28-09-2016 |
| | | JP 2014518868 A | 07-08-2014 |
| | | KR 20140034821 A | 20-03-2014 |
| | | RU 2013155509 A | 27-06-2015 |
| | | SG 194842 A1 | 30-12-2013 |
| | | US 2014155383 A1 | 05-06-2014 |
| | | WO 2012156311 A1 | 22-11-2012 |
| | | ZA 201308391 B | 25-02-2015 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 2005066180 A
- WO 2008034781 A


### Non-patent literature cited in the description


- *CHEMICAL ABSTRACTS*, 860343-27-7
- *VIth SCI-RSC Symposium on Proteinase Inhibitor Design, London UK*, 21 April 2008
- *ACS Spring Meeting Anaheim*, 21 March 2011
- **BIRGE, S.M. et al.** Pharmaceutical salts. *J. Pharm. Sci.*, 1977, vol. 66, 1-19
- **ALBUQUERQUE C** ; **MORINHA F** ; **REQUICHA J** ; **MARTINS T** ; **DIAS I** ; **GUEDES-PINTO H** ; **BASTOS E** ; **VIEGAS C**. Canine periodontitis: the dog as an important model for periodontal studies.. *Vet J*, 2012, vol. 191, 299-305
- **BAIM S** ; **MILLER PD**. Perspective: assessing the clinical utility of serum CTX in postmenopausal osteoporosis and its use in predicting risk of osteonecrosis of the jaw.. *J Bone Miner Res.*, 2009, vol. 24, 561-574
- **BELLOWS J.** Periodontal equipment, materials, and techniques.. *Small Animal Dental Equipment, Materials, and Techniques: A Primer. Ames, IA: Blackwell*, 2004, 115-173
- **CHUBB SA.** Measurement of C-terminal telopeptide of type I collagen (CTX) in serum.. *Clin Biochem.*, 06 April 2012, vol. 45 (12), 928-35
- **COSTA, A. G** ; **CUSANO, N. E.** ; **SILVA, B. C** ; **CREMERS, S.** ; **BILEZIKIAN, J. P**. Cathepsin K: its skeletal actions and role as a therapeutic target in osteoporosis.. *Nat. Rev. Rheumatol.*, 2011, vol. 7, 447-456
- **DAI R** ; **WU Z** ; **CHU HY** ; **LU J** ; **LYU A** ; **LIU J** ; **ZHANG G.** ; **CATHEPSIN K**. The Action in and Beyond Bone.. *Front Cell Dev Biol*, 04 June 2020, vol. 8, 433
- Problems with the gingiva.. **DEBOWES U**. Small Animal dental, oral and maxillofacial disease, a color handbook. 2010, 159-181
- **DIEHL KH** ; **HULL R** ; **MORTON D** ; **PFISTER R** ; **RABEMAMPIANINA Y** ; **SMITH D** ; **VIDAL JM** ; **VAN DE VORSTENBOSCH C**. European Federation of Pharmaceutical Industries Association and European Centre for the Validation of Alternative Methods. A good practice guide to the administration of substances and removal of blood, including routes and volumes.. *J Appl Toxicol.*, January 2001, vol. 21 (1), 15-23
- **DE FARIA VASCONCELOS K** ; **EVANGELISTA KM** ; **RODRIGUES CD** ; **ESTRELA C** ; **DE SOUSA TO** ; **SILVA MA**. Detection of periodontal bone loss using cone beam CT and intraoral radiography.. *Dentomaxillofac Radiol.*, January 2012, vol. 41 (1), 64-9
- **DRAKE MT** ; **CLARKE BL** ; **OURSLER MJ** ; **KHOSLA S**. Cathepsin K Inhibitors for Osteoporosis: Biology, Potential Clinical Utility, and Lessons Learned.. *Endocr Rev.*, 01 August 2017, vol. 38 (4), 325-350
- Therapeutic inhibition of cathepsin K-reducing bone resorption while maintaining bone formation.. *Bonekey Rep.*, 02 May 2012, vol. 1, 67
- **FARLEY JR** ; **TARBAUX N** ; **MURPHY LA** ; **MASUDA T** ; **BAYLINK DJ**. In vitro evidence that bone formation may be coupled to resorption by release of mitogen(s) from resorbing bone.. *Metabolism*, 1987, vol. 36, 314-21
- **FIORELLINI JP** ; **ISHIKAWA SO** ; **KIM DM**. Gingival Inflammation.. *Carranza's Clinical Periodontology.*, 2006, 355-361
- **GAWOR JP** ; **STRØM P** ; **NEMEC A.** Treatment of Naturally Occurring Periodontitis in Dogs With a New Bio-Absorbable Regenerative Matrix.. *Front Vet Sci.*, 21 June 2022, vol. 9, 916171
- **GOWEN, M. et al.** Cathepsin K knockout mice develop osteopetrosis due to a deficit in matrix degradation but not demineralization.. *J Bone Miner Res*, 1999, vol. 14 (10), 1654-63
- **HAMP, S. E** ; **OLSSON, S. E** ; **FARSØ-MADSEN, K. et al.** A macroscopic and radiological investigation of dental diseases of the dog.. *Veterinary Radiology*, 1984, vol. 25, 86-92
- **HOFFMAN, T.** ; **GAENGLER, P**. Epidemiology of periodontal disease in poodles.. *Journal of Small Animal Practice*, 1996, vol. 37, 309-316
- **LEGEROS, R. Z** ; **SHANNON, I. L**. The crystalline components of dental cal- culi: human vs. dog.. *Journal of Dental Research*, 1979, vol. 58, 2371-2377

- **LI CY** ; **JEPSEN KJ** ; **MAJESKA RJ** ; **ZHANG J** ; **NI R** ; **GELB BD et al.** Mice lacking cathepsin K maintain bone remodeling but develop bone fragility despite high bone mass.. *J Bone Miner Res*, 2006, vol. 21, 865-75
- **LUND, E. M** ; **ARMSTRONG, P. J.** ; **KIRK, C. A et al.** Health status and population characteristics of dogs and cats examined at private veterinary practices in the United States. *Journal of the American Veterinary Medical Association*, 1999, vol. 214, 1336-1341
- **MARSHALL, M. D** ; **WALLIS, C. V.** ; **MILELLA, L. et al.** A longitudinal assessment of periodontal disease in 52 miniature schnauzers.. *BMC Veterinary Research*, 2014, vol. 10, 166
- **MAZUR**. Features of bone tissue metabolic processes in patients with generalized periodontitits in the period of disease exacerbation.. *Gerontologija*, 2006, vol. 7 (4), 174-179
- **NIEMIEC, B. A.** Oral pathology.. *Topics in Companion Animal Medicine*, 2008, vol. 23, 59-71
- Pathologies of the oral mucosa.. **NIEMIEC BA**. Small animal dental, oral, and maxillofacial disease, a color handbook. 2010, vol. 2010, 183-98
- Local and Regional Consequences of Periodontal Disease.. **NIEMIEC BA**. Veterinary Periodontology. Ames, Wiley Blackwell, 2013, 69-80
- Systemic Manifestations of Periodontal Disease.. **NIEMIEC BA**. Veterinary Periodontology.. Ames, Wiley Blackwell, 2013, 81-90
- **NIEMIEC B** ; **GAWOR J** ; **NEMEC A** ; **CLARKE D** ; **MCLEOD K** ; **TUTT C** ; **GIOSO M** ; **STEAGALL PV** ; **CHANDLER M** ; **MORGENEGG G**. World Small Animal Veterinary Association Global Dental Guidelines.. *J Small Anim Pract.*, July 2020, vol. 61 (7), E36-E161
- **ERRATUM**. *J Small Anim Pract.*, December 2020, vol. 61 (12), 786
- **NIKAHVAL B** ; **NAZIFI S** ; **HEIDARI F** ; **KHAFI M**. Evaluation of the changes of P1NP and CTX in synovial fluid and blood serum of dogs with experimental osteoarthritis.. *Comp Clin Pathol*, 2016
- **O'NEILL, D. G** ; **CHURCH, D. B.** ; **MCGREEVY, P. D. et al.** Prevalence of disorders recorded in dogs attending primary-care veterinary practices in England.. *PLoS One*, 2014, vol. 9
- **PAVLICA, Z** ; **PETELIN, M** ; **JUNTES, P. et al.** Periodontal disease burden and pathological changes in organs of dogs.. *Journal of Veterinary Dentistry*, 2008, vol. 25, 97-105
- **PFEILSCHIFTER J** ; **MUNDY GR**. Modulation of type beta transforming growth factor activity in bone cultures by osteotropic hormones.. *Proc Natl Acad Sci U S A*, 1987, vol. 84, 2024-8
- **ROBINSON, N. J.** ; **DEAN, R. S** ; **COBB, M. et al.** Factors influencing common diagnoses made during first-opinion small-animal consultations in the United Kingdom.. *Preventive Veterinary Medicine*, 2016, vol. 131, 87-94
- **SHOUKRY M** ; **BEN ALI L** ; **ABDEL NABY M** ; **SOLIMAN A.** Repair of experimental plaque-induced periodontal disease in dogs.. *J Vet Dent.*, September 2007, vol. 24 (3), 152-65
- Periodontitis.. **SOUKUP J**. Textbook of veterinary internal medicine. Saunders Elsevier, 2010, 179-184
- **STOCH SA** ; **ZAJIC S** ; **STONE J** ; **MILLER DL** ; **VAN DYCK K et al.** Effect of the cathepsin K inhibitor odanacatib on bone resorption biomarkers in healthy postmenopausal women: two double-blind, randomized, placebo-controlled phase I studies. *Clin Pharmacol Ther.*, August 2009, vol. 86 (2), 175-82
- **VRBANAC Z** ; **BRKLJACA BOTTEGARO N** ; **SKRLIN B** ; **BOJANIC K** ; **KUSEC V** ; **STANIN D** ; **BELIC M**. The Effect of a Moderate Exercise Program on Serum Markers of Bone Metabolism in Dogs.. *Animals (Basel).*, 23 August 2020, vol. 10 (9), 1481
- **WALLIS C** ; **HOLCOMBE LJ.** A review of the frequency and impact of periodontal disease in dogs.. *J Small Anim Pract.*, September 2020, vol. 61 (9), 529-540
- **WIGGS, R.B** ; **LOBPRISE, H.B**. Periodontology.. *Veterinary*, 1997
- Periodontology.. **WIGGS, R.B** ; **LOBPRISE, H.B.** Veterinary Dentistry, Principles and Practice. Lippincott Raven Publishers, 1997, 186-231
- **WOLF HF** ; **RATEITSCHAK EM** ; **RATEITSCHAK KH et al.** Color atlas of dental medicine: periodontology. Georg Thieme Verlag, 2005